(19) Europäisches Patentamt
European Patent Office
Office européen des brevets

(11) EP 0 498 723 B1

(12) **FASCICULE DE BREVET EUROPEEN**

(45) Date de publication et mention de la délivrance du brevet: **19.09.2001 Bulletin 2001/38**

(21) Numéro de dépôt: **92400296.7**

(22) Date de dépôt: **05.02.1992**

(51) Int Cl.$^7$: **C07D 215/12**, A61K 31/395, C07D 215/233, C07D 215/04, C07D 215/44, C07D 237/28, C07D 237/32, C07D 239/74, C07D 241/44

(54) **Dérivés bicycliques azotés, leur procédé de préparation, leur application à titre de médicament et les compositions pharmaceutiques les renfermant**

Stickstoff enthaltende bicyclische Verbindungen, Verfahren zu ihrer Herstellung, ihre Verwendung als Arzneimittel und diese enthaltende Zusammensetzungen

Nitrogen containing bicycle compounds, method for their preparation, their use as pharmaceutical and compositions containing them

(84) Etats contractants désignés:
**AT BE CH DE DK ES FR GB GR IT LI LU MC NL PT SE**

(30) Priorité: **07.02.1991 FR 9101374**
**20.08.1991 FR 9110435**

(43) Date de publication de la demande:
**12.08.1992 Bulletin 1992/33**

(73) Titulaire: **Aventis Pharma S.A.
92160 Antony (FR)**

(72) Inventeurs:
- **Clemence, François
F-75009 Paris (FR)**
- **Fortin, Michel
F-75018 Paris (FR)**
- **Haesslein, Jean-Luc
F-77181 Courtry (FR)**

(56) Documents cités:

| | |
|---|---|
| **EP-A- 0 191 603** | **EP-A- 0 326 328** |
| **EP-A- 0 342 775** | **EP-A- 0 412 848** |
| **EP-A- 3 263 330** | **WO-A-84/00489** |
| **DE-A- 2 339 240** | **FR-A- 2 377 400** |
| **GB-A- 1 419 788** | **GB-A- 2 143 814** |
| **US-A- 3 936 461** | **US-A- 4 025 629** |
| **US-A- 4 956 371** | |

- **CHIMIE THERAPEUTIQUE, no. 2, mars-avril 1973, pages 154-168; A. ALLAIS et al.: "Recherche de composés analgésiques non narcotiques. Etude de nouvelles (alcoxycarbonyl-2' phénylamino)-4 quinoléines et de molécules apparentées"**
- **CHEMICAL ABSTRACTS, vol. 90, no. 23, 1979, page 669, abrégé no. 186992m, Columbus, Ohio, US; & ES-A-453 829 (LABORATORIO FORTUNY S.A.) 01-01-1979**
- **CHEMICAL ABSTRACTS, vol. 55, no. 10, mai 1961, abrégé no. 9333h, Columbus, Ohio, US; H. MITSUBISHI et al.: "Constituents of Umbelliferae plants. II. Isolation of the active principles of lugusticum root", & CHEM. PHARM. BULL. (TOKYO) 8, 243-5(1960)**
- **CHEMICAL ABSTRACTS, vol. 85, 1976, page 466, abrégé no. 45850y, Columbus, Ohio, US; S.I. YAKIMOVICH et al.: "Tautomerism in a series of nitrogen derivatives of ethyl alpha-ethoxalylcarboxylates", & ZH. ORG. KHIM. 1976, 12(5), 949-56**
- **EUR. J. MED. CHEM., 1977, vol.12, pages 324-331**
- **J. HET. CHEM., 1978, vol. 15. pages 1033-1037**
- **CHEMICAL ABSTRACTS, Vol. 111, no. , 1989, Columbus, Ohio, US, page 758, colonne , abstract no. 78027u, : "Preparation of quinoxaline derivatives as aldose reductase inhibitors and a process for their preparation"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 498 723 B1

## Description

La présente invention concerne de nouveaux dérivés bicycliques azotés, leur procédé de préparation, les nouveaux intermédiaires obtenus, leur application à titre de médicaments et les compositions pharmaceutiques les renfermant. EP-A-0412848 qui est un document intermédiaire opposable au titre de l'art 54(3) (4) décrit des dérivés de quinoléine comme antagonistes de l'angotensine II.

US-A-3936461 décrit des dérivés de quinoléine comme antiangineux (vaso-dilatateurs coronariens).
GB-A2143814 décrit des dérivés de quinazolines comme vasodilatateurs (et/ou anti-aggrégants plaquettaires).
US-A-4956371 décrit des dérivés d'isoquinoléine comme inhibiteurs de réponses cellulaires au PAF.
EP-A-0191603 décrit des dérivés de quinoléine comme antihypertenseurs.
US-A-4025629 décrit des dérivés de quinoléine comme antihypertenseurs.
Chimie thérapeutique No-2,1972, p.154-168 analgésiques et anti-inflammatoires.
EP-A-0343775 décrit des dérivés de quinoléines comme inhibiteurs de la sécrétion gastrique.
WO-A-8400489 décrit des dérivés d'(iso) quinoléine comme agent pour lutter contre l'arrythmie cardiaque.
CAS 186992n (vol 90, 1979) décrit un dérivé de quinazolinone. CAS. 9333h (vol 55, 1961) décrit un dérivé de phtalazinone (phenodiazinone).
FR-A-2377400 décrit des dérivés de quinoléine comme antalgiques.
CAS 45850y (vol 85, 1976) décrit un tautomérisme, sans mention d'un bicycle.
GB-A-1419788 décrit des dérivés de quinoléine comme herbicides.
EP-A-0326328 décrit des dérivés de quinoléine comme fongicides.
DE-A-2339240 décrit des dérivés quinoléine comme agent d'extraction de dérivés métalliques.
EP-A-0326330 décrit des dérivés de quinoléine comme fongicides.
EUR.J.Med. Chem 1997, vol 12, p.324-331 décrit des dérivés de quinazoline comme fongicides.
J. Het Chem 1978, vol 15, p1033-1037 décrit des dérivés de quinazolinones.
CAS 78027u (vol 111, 1989) décrit des dérivés de quinoxaline comme inhibiteurs de l'aldose réductase.

La présente invention a pour objet les produits formule (I) :

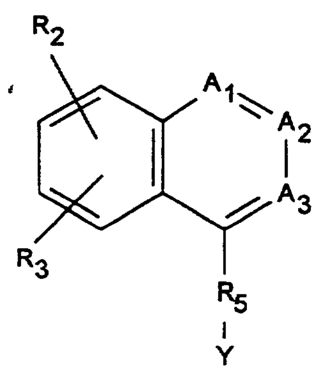

(I)

dans laquelle :

$R_2$ et $R_3$ représentent un atome d'hydrogène ou un radical alkylthio renfermant au plus 4 atomes de carbone,
$A_1$, $A_2$ et $A_3$ sont tels que l'un ou deux d'entre eux représentent un atome d'azote, et les autres, identiques ou différents, représente le radical

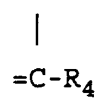

tel que $R_4$ est choisi parmi l'atome d'hydrogène, le radical n-butyle et alkylthio renfermant au plus 4 atomes de carbone,
$R_5$ représente le radical $-CH_2-$, -NH-, -O- et $-OCH_2-$ et Y représente phényle substitué par un radical tétrazole ou biphényle éventuellement substitué par un ou plusieurs radicaux choisis parmi les radicaux cyano, carboxy libre,

salifié et estérifié et tétrazolyle, à l'exception des produits dans lesquels :

a) $A_1$ représente un atome d'azote, $A_2$ et $A_3$, identiques ou différents, représentent le radical

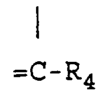

dans lequel $R_4$ représente l'atome d'hydrogène ou le radical n-butyle, $R_5$ représente $-O-CH_2$,

b) $R_2$ et $R_3$ représentent hydrogène,

$A_1$ représente un atome d'azote,
$A_2$ et $A_3$ sont tels que ou bien l'un représente un atome d'azote et l'autre représente

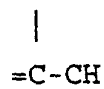

ou bien les deux représentent

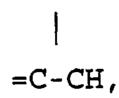

$R_5$ représente O, NH ou $CH_2$
et Y représente biphényle éventuellement substitué par CN, étant entendu que les 5 produits suivants :

- Chlorhydrate d'acide 4-[[(2-butyl 4-quinoléinyl) oxy] méthyl] benzoïque ;
- 4'-[[(3-butyl 5-méthylthio 4-quinoléinyl) oxy] méthyl] (1,1'-biphényl)-2-carboxylate de méthyle ;
- Sel de diéthylamine de l'acide 4'-[[(3-butyl 1,4-dihydro 4-(méthylthio) 4-quinoléinyl) oxy] méthyl] (1,1'-biphényl) 2-carboxylique ;
- Acide 4'-[[(3-butyl 4-quinoléinyl) oxy] méthyl] (1,1'-biphényl)-2-carboxylique ;
- Acide 4'-[(2-phényl 4-quinazolinyl) méthyl] (1,1'-biphényl)-2-carboxylique,

appartiennent à la présente invention,
lesdits produits de formule (I) et ces 5 produits étant sous toutes les formes isomères possibles racémiques, énantiomères et diastéréoisomères, ainsi que les sels d'addition avec les acides minéraux et organiques ou avec les bases minérales et organiques desdits produits de formule (I) et de ces 5 produits.
Les 5 produits indiqués en dessus en b) seront nommés ci-après "les 5 produits définis ci dessus".

Dans les produits de formule (I) et dans ce qui suit :

- le terme atome d'halogène désigne de préférence l'atome de chlore, mais peut aussi représenter un atome de fluor, de brome ou d'iode,
- le terme radical acyle désigne de préférence un radical ayant au plus 7 atomes de carbone tel que le radical acétyle, propionyle, butyryle ou benzoyle, mais peut également représenter un radical valéryle, hexanoyle, acryloyle, crotonoyle ou carbamoyle : on peut également citer le radical formyle ;
- le terme carboxy estérifié désigne de préférence un groupe alkyloxy inférieur carbonyle tel que méthoxycarbonyle, éthoxycarbonyle, tert- butoxycarbonyle ou un groupe benzyloxycarbonyle,
- le terme radical alkyle linéaire ou ramifié désigne de préférence les radicaux méthyle, éthyle, propyle, isopropyle, n-butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement butèn-1-yl, ou pentényle,

- le terme radical alkynyle linéaire ou ramifié désigne de préférence un radical éthynyle, propargyle, butynyle ou pentynyle,
- le terme radical alkyloxy linéaire ou ramifié désigne de préférence les radicaux méthoxy ou éthoxy, mais peut aussi représenter un radical propoxy, isopropoxy, butoxy linéaire, secondaire ou tertiaire,
- le terme acyloxy désigne les radicaux dans lesquels les radicaux acyle ont la signification indiquée ci-dessus et par exemple les radicaux acétoxy ou propionyloxy,
- le terme radical alkylthio linéaire ou ramifié désigne les radicaux dans lesquels le radical alkyle peut représenter, par exemple, les valeurs indiquées ci-dessus pour le radical alkyle; le radical alkylthio représente de préférence les radicaux méthylthio ou éthylthio, mais peut aussi représenter un radical propylthio, isopropylthio, n- butylthio, sec-butylthio, tert-butylthio, isopentylthio ou isohexylthio,

Les radicaux amino que peuvent représenter l'un ou plusieurs des éventuels substituants des radicaux définis dans les produits de la présente invention sont le carbamoyle,ou les radicaux monoalkyl- ou dialkylamino dans lesquels les radicaux alkyles linéaires ou ramifiés renferment de 1 à 6 atomes de carbone et en particulier des radicaux méthyle, éthyle, isopropyle, trifluorométhyle, pentafluoroéthyle, hydroxyméthyle, hydroxyéthyle, méthoxyméthyle, méthoxyéthyle, éthoxyéthyle; ou les cycles pyrrolyle, imidazolyle, pyridyle, pyrazinyle, pyrimidyle, indolyle, indolinyle, purinyle, quinolyle, pyrrolidinyle, pipéridyle, pipéridino, morpholino, pipérazinyle ; ces radicaux peuvent être éventuellement substitués par les substituants déjà mentionnés précédemment Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoique, maléique, fumarique, succinique, tartrique, citrique, oxalique, glyoxylique, aspartique, ascorbique, les acides alcanemonosulfoniques tels que par exemple l'acide méthanesulfonique, l'acide éthanesulfonique, l'acide propanesulfonique, les acides alcanedisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Le ou les radicaux carboxy des produits de formule (I) peuvent être salifiés par des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris(hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

De tels produits de formule (I) représentent ainsi en particulier des dérivés :

- de la quinoline dans lesquels $A_1$ représente un atome d'azote,
- de l'isoquinoline dans lesquels l'un de $A_2$ et $A_3$ représente un atome d'azote et des dérivés :

- de la cinnoline dans lesquels $A_1$ et $A_2$ représentent tous deux un atome d'azote,
- de la quinazoline dans lesquels $A_1$ et $A_3$ représentent tous deux un atome d'azote.

Parmi les produits, objet de l'invention, peut être cité tout particulièrement :

- l'acide 4'- [(2-butyl-4-quinoleinyl) méthyl] (1,1'-biphényl)-2-carboxylique.

L'invention a pour objet un procédé de préparation de produits de formule (I) et des 5 produits tels que définis ci-dessus, caractérisé en ce que :

a) soit l'on soumet le composé de formule (III) :

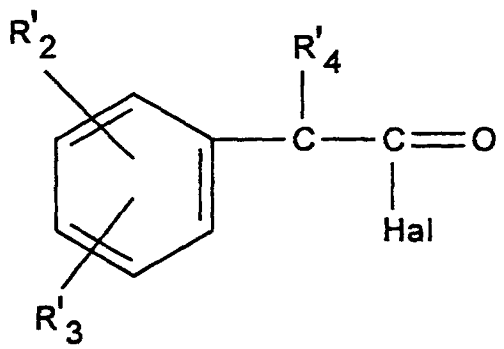

(III)

dans laquelle $R'_2$, $R'_3$ et $R'_4$ ont les significations indiquées à la revendication 1 respectivement pour $R_2$, $R_3$ et $R_4$ dans lesquelles les éventuelles fonctions réactives sont éventuellement protégées par des groupements protecteurs, $R'_4$ pouvant également représenter un radical phényle,
et Hal représente un atome d'halogène, à une réaction de substitution pour obtenir le produit de formule (IV) :

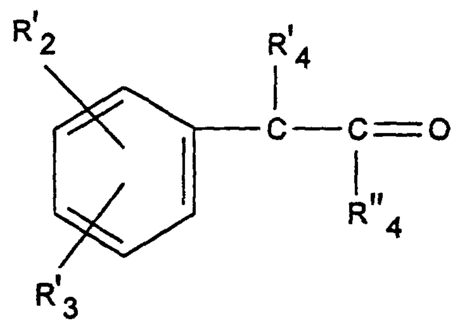

(IV)

dans laquelle $R'_2$, $R'_3$ et $R'_4$ ont les significations indiquées ci-dessus et $R''_4$, identique ou différent de $R'_4$, a la signification indiquée ci-dessus pour $R_4$ dans laquelle les éventuelles fonctions réactives sont éventuellement protégées par des groupements protecteurs, que l'on fait réagir :
avec un composé de formule (V) :

$$R_4''' - C \equiv N \qquad (V)$$

dans laquelle $R_4'''$, identique ou différent de $R'_4$ ou $R''_4$ a la signification indiquée à la revendication 1 pour $R_4$ dans laquelle les éventuelles fonctions réactives sont éventuellement protégées par des groupements protecteurs, pour obtenir aprés cyclisation un produit de formule (Ii) :

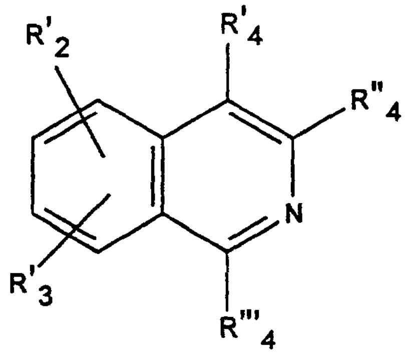

(Ii)

dans laquelle R'$_2$, R'$_3$, R'$_4$, R"$_4$ et R"'$_4$ ont les significations indiquées ci-dessus,
b) soit l'on fait réagir un produit de formule (VI) :

(VI)

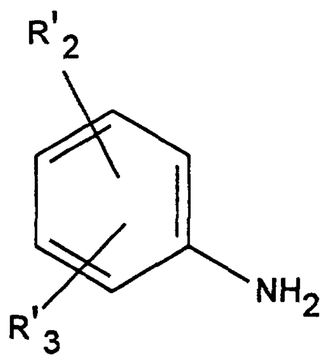

dans laquelle R'$_2$ et R'$_3$ ont les significations indiquées ci-dessus avec un produit de formule (VII) :

$$R'_4\text{-CH}(CO_2\text{alk})\text{-CO-}R''_4 \quad \text{(VII)}$$

dans laquelle R'$_4$ et R"$_4$, identiques ou différents, ont les significations indiquées ci-dessus et alk représente un radical alkyle renfermant au plus 6 atomes de carbone, pour obtenir des produits de formule (VIII) :

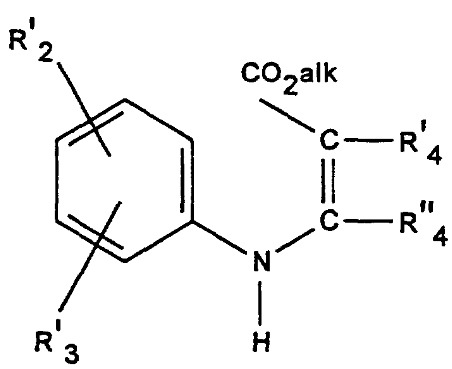

(VIII)

dans laquelle R'$_2$, R'$_3$, R'$_4$, R'$_4$ et alk ont les significations indiquées ci-dessus, que l'on cyclise pour obtenir des

produits de formule (IIb) :

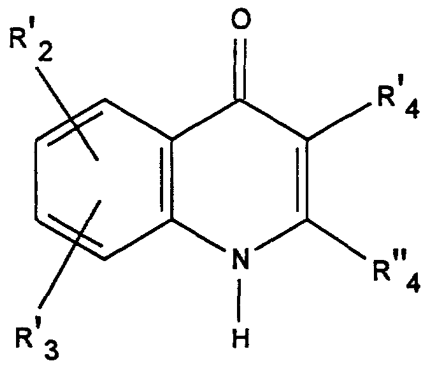

(IIb)

dans laquelle $R'_2$, $R'_3$, $R'_4$ et $R''_4$ ont les significations indiquées ci-dessus,
c) soit l'on fait réagir un produit de formule (IX):

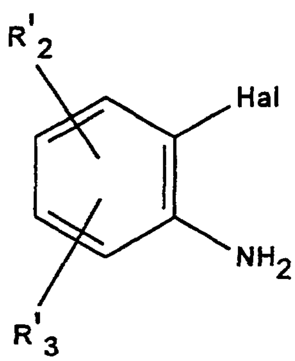

(IX)

dans laquelle $R'_2$ et $R'_3$ ont les significations indiquées ci-dessus avec un produit de formule (X) :

$$R'''_4\text{-}C{\equiv}CH \qquad (X)$$

dans laquelle $R'''_4$ a la signification indiquée ci-dessus pour obtenir des produits de formule (XI) :

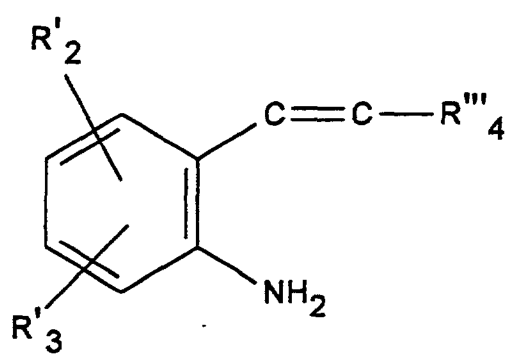

(XI)

dans laquelle $R'_2$, $R'_3$ et $R'''_4$ ont les significations indiquées ci-dessus, que l'on soumet à une réaction de cyclisation en présence d'un donneur d'azote tel que le nitrite de sodium, pour obtenir un produit de formule (IIC) :

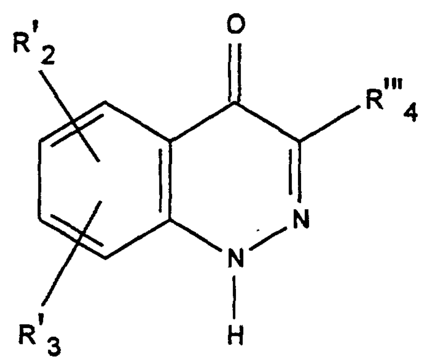

(IIc)

dans laquelle R'$_2$, R'$_3$ et R'' '$_4$ ont les significations indiquées ci-dessus,
d) soit l'on soumet un produit de formule (XIV) :

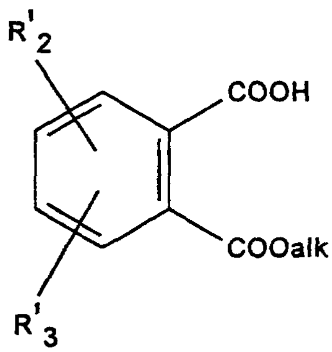

(XIV)

dans laquelle R'$_2$ et R'$_3$ et alk ont les significations indiquées ci-dessus, aprés, si désiré, une réaction d'halogénation de la fonction carboxy libre, à une réaction d'addition sur cette fonction carboxy d'un composé de formule R'$_4$-H, R'$_4$ ayant la signification indiquée ci-dessus, pour obtenir aprés cyclisation en présence d'hydrazine ou d'un dérivé des produits de formule (IIe) :

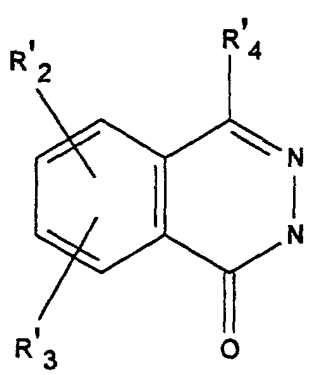

(IIe)

dans laquelle R'$_2$, R'$_3$ et R'$_4$ ont les significations indiquées ci-dessus,
e) soit l'on fait réagir un produit de formule (XV) :

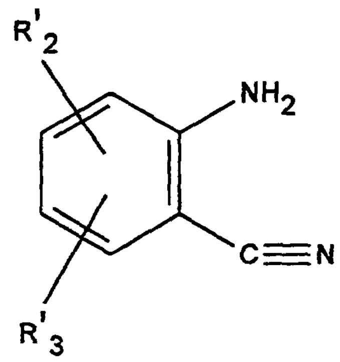

(XV)

dans laquelle $R'_2$ et $R'_3$ ont les significations indiquées ci-dessus avec un produit de formule (XVI) :

$R'_4$-CO-Cl (XVI)

dans laquelle $R'_4$ a la signification indiquée ci-dessus pour donner le produit de formule (XVII) :

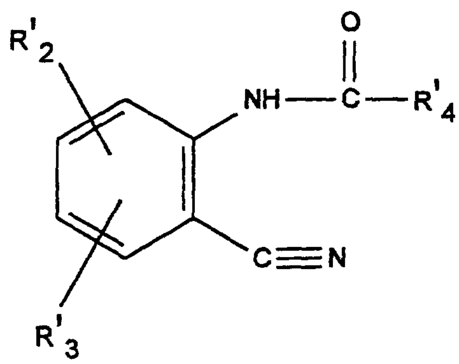

(XVII)

dans laquelle $R'_2$, $R'_3$ et $R'_4$ ont les significations indiquées précédemment, pour obtenir, aprés cyclisation, des produits de formule (IIf) :

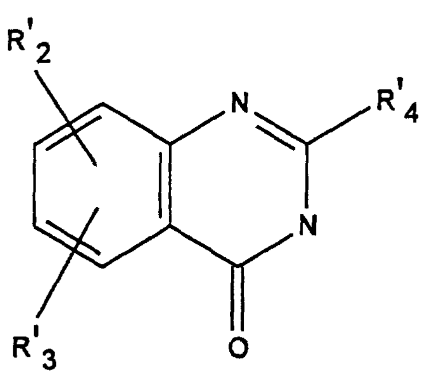

(IIf)

dans laquelle $R'_2$, $R'_3$ et $R'_4$ ont les significations indiquées ci-dessus, produits de formule (Ii) qui peuvent représenter des produits de formule (I) ou les 5 produits définis ci-dessus et produits de formules (IIb), (IIc), (IIe) et (IIf) telles que définies ci-dessus qui peuvent représenter des produits de formule (I)ou les 5 produits définis ci-dessus dans laquelle l'un au moins de $A_1$, $A_2$, $A_3$ et $A_4$ représente un radical méthine portant un radical hydroxyle, que l'on soumet, si désiré et si nécessaire, à l'une ou plusieurs des réactions suivantes, dans un ordre quelconque :

- une réaction de réduction complète du radical hydroxyle ou oxo en radical méthine suivie d'une aromatisation,
- sur les produits de formule (Ii) dans lesquels l'un de $R'_4$, $R''_4$ ou $R'''_4$ représente un radical hydroxyle ou sur les produits de formules (IIb), (IIc), (IIe) et (IIf) que l'on soumet

soit d'abord à une réaction de substitution du radical hydroxyle par un atome d'halogène suivie de l'action d'un produit de formule $R_{p4}$ - M - Hal dans lequel $R_{p4}$ a la signification indiquée à la revendication 1 pour $R_4$ dans lesquelles les éventuelles fonctions réactives sont éventuellement protégées par des groupements protecteurs, M représente un atome de métal choisi parmi le magnésium, le cuivre et le zinc et Hal représente un atome d'halogène,
soit à l'action d'un produit de formule $R_{p4}$ - Hal dans lequel Hal représente un atome d'halogène, pour obtenir les produits de formule (I) correspondants,

- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification d'une fonction acide,
- une réaction de saponification de fonction ester en fonction acide,
- une réaction de transformation de la fonction cyano en fonction acide,
- une réaction de dédoublement des formes racémiques, lesdits produits de formule (I) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :

- la réaction de substitution sur le dérivé halogéné de formule (III) pour obtenir le produit de formule (IV) peut être réalisée selon les méthodes usuelles connues de l'homme de métier telles que par exemple par réaction d'un organométallique tel que par exemple un organozincique de formule Zn-Br-$R''_4$ sur le chlorure d'acide de formule (III) ou encore, par exemple, en particulier lorsque $R''_4$ représente un radical butyle, par réaction d'un dérivé de l'étain tel que le composé de formule $Sn(R''_4)_4$ de préférence en présence de palladium dans un solvant tel que par exemple l'éther ou le tétrahydrofuranne,
- la réaction d'addition du composé de formule (V) sur le composé de formule (IV) ainsi obtenu peut être réalisée selon les méthodes usuelles connues de l'homme de métier telles que par exemple dans du trichlorure de phosphoryle en présence d'un acide de Lewis et la réaction de cyclisation donnant le produit de formule (Ii) se produit in situ,
- la réaction d'addition du composé de formule (VII) sur le composé de formule (VI) peut être réalisée selon les méthodes usuelles connues de l'homme de métier telles que, par exemple, en présence d'un agent dessicant tel qu'un tamis moléculaire ou encore d'un acide tel que, par exemple, l'acide paratoluène sulfonique,
- la réaction de cyclisation du composé de formule (VIII) en composé de formule (IIb) peut être réalisée par exemple dans un solvant tel que le DOWTHERM diphényléther ou encore en absence de solvant en portant le composé à son point de fusion
- la réaction d'addition du composé de formule (X) sur le composé de formule (IX) pour obtenir le composé de formule (XI) peut être réalisée, par exemple, en présence de sel cuivreux de préférence en présence d'un catalyseur tel que par exemple un catalyseur au palladium dans un solvant basique tel que par exemple la triéthylamine ou la diéthylisopropylamine,
- la réaction de cyclisation du composé de formule (XI) en composé de formule (IIc) peut être réalisée, par exemple, en présence d'un donneur d'azote tel que le nitrite de sodium dans un milieu acide tel que par exemple dans de l'acide chlorhydrique,
- la réaction d'halogénation du composé de formule (XIV) peut être réalisée selon les méthodes usuelles connues de l'homme de métier telles que par exemple en présence de chlorure d'oxalyle ou encore de chlorure de thionyle et la réaction de cyclisation du composé ainsi obtenu pour donner le composé de formule (IIe) se produit en présence d'hydrazine ou d'un dérivé de l'hydrazine à chaud dans un alcool tel que par exemple du méthanol ou de l'éthanol,
- la réaction d'addition du chlorure d'acide de formule (XVI) sur le dérivé aminé de formule (XV) pour obtenir le composé de formule (XVII) peut être réalisée, par exemple, au reflux d'un solvant tel que, par exemple, la pyridine,
- la réaction de cyclisation du produit de formule (XVII) ainsi obtenu pour obtenir le produit de formule (IIf) se produit, par exemple, dans de l'eau oxygénée en présence de soude aqueuse, par exemple au reflux d'un solvant tel que le dioxanne.

Les produits de formules (Ii) peuvent constituer des produits. de formule (I), les produits de formules (IIb),

(IIc), (IIe) et (IIf) ainsi obtenus représentent des produits de formule (I) dans laquelle $A_1$', $A_2$'et $A_3$' peuvent représenter les radicaux

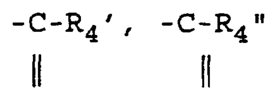

ou

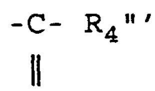

tels que définis ci-dessus soit les significations indiquées respectivement pour $A_1$, $A_2$ et $A_3$ dans lesquelles les éventuelles fonctions réactives sont éventuellement protégées par des groupements protecteurs et dans laquelle l'un au moins de $A_1$, $A_2$ et $A_3$ représente un radical méthine portant un radical hydroxyle.

Les produits de formule (Ii) et les produits de formules (IIb), (IIc),(IIe) et (IIf) ainsi obtenus, en particulier pour donner des produits de formule (I) ou les 5 produits tels que définis ci-dessus, peuvent être soumis, si désiré et si nécessaire, à l'une ou plusieurs des réactions indiquées ci-dessus, ces réactions pouvent être, dans des conditions préférentielles, réalisées de la façon indiquée ci-aprés.

Les radicaux hydroxyle existant ou issus de la forme tautomère oxo des composés de formules (IIb), (IIc), (IIe) et (IIf) ou éventuellement des produits de formule (Ii) obtenus ainsi qu'il est indiqué ci-dessus peuvent être, si nécessaire et si désiré, soumis à une réaction de réduction complète en radical méthine.

Cette réaction de réduction complète en radical méthine telle que définie ci-dessus peut être réalisée après transformation de la fonction hydroxy par exemple en halogène ou mésylate à l'aide d'un agent réducteur tel que par exemple l'hydrure de lithium et d'aluminium ou encore par réduction catalytique sur palladium en présence d'hydrogène.

La réaction de substitution des produits de formules (Ii), (IIb), (IIc),(IIe) et (IIf) par le radical $R_{4p}$ est soumise au préalable à une réaction de substitution du radical hydroxyle réalisée, par exemple, par la préparation du dérivé halogéné tel que par exemple le dérivé chloré préparé par traitement par un agent chlorant tel que par exemple le pentachlorure de phosphore ou l'oxychlorure de phosphore éventuellement dans un solvant tel que par exemple le dioxanne ou le tétrahydrofuranne ou encore réalisé par exemple par addition du radical hydroxyle par la préparation par exemple du trifluoroacétate sulfonate.

La réaction de substitution par le radical $R_{4p}$ telle que définie ci-dessus peut être réalisée par exemple par réaction avec un organométallique tel que par exemple un organozincique de formule $R_{4p}$ - Zn - Br si désiré en présence d'une quantité catalytique d'un complexe de métal de transition tel que par exemple le palladium ou la nickel au reflux d'un solvant tel que par exemple le tétrahydrofuranne.

La réaction de transformation de la fonction oxo en fonction thioxo peut être réalisée selon les méthodes usuelles connues de l'homme de métier telles que par exemple à l'aide du réactif de Lawesson ou encore de pentasulfure de phosphore au reflux dans un solvant tel que par exemple le toluène ou un alcool tel que par exemple l'éthanol.

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :

- les groupements hydroxyle peuvent être protégés par exemple par les radicaux alkyle, trialkylsilyle, dihydropyranne, méthoxyméthyle ou tétrahydropyrannyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les fonctions acides des produits décrits ci-dessus peuvent être, si désiré, amidifiées par une amine primaire ou secondaire par exemple dans du chorure de méthylène en présence de chlorhydrate de 1-éthyl 3-(diméthylaminopropyl) carbodiimide à la température ambiante,
- les fonctions acides peuvent être protégées par exemple sous forme d'esters formés avec les esters facilement clivables tels que les esters benzyliques ou tert-butyliques ou des esters connus dans la chimie des peptides.

L'élimination de ces groupements protecteurs est effectuée dans les conditions usuelles connues de l'homme de métier notamment l'hydrolyse acide effectuée avec un acide tel que l'acide chlorhydrique, benzènesulfonique ou para-toluène sulfonique, formique ou trifluoroacétique.

Le groupement phtalimido est éliminé par l'hydrazine. On trouvera une liste de différents groupements protecteurs utilisables par exemple dans le brevet BF 2 499 995.

Les produits décrits ci-dessus peuvent, si désiré, faire l'objet de réactions de salification par un acide minéral ou organique ou par une base minérale ou organique, en particulier sur les éventuelles fonctions carboxy, ces réactions pouvant être réalisées selon les méthodes usuelles connues de l'homme de métier.

Les produits décrits ci-dessus peuvent, si désiré, faire l'objet, sur les éventuelles fonctions carboxy, de réactions d'estérification qui peuvent être réalisées selon les méthodes usuelles connues de l'homme de métier.

Les éventuelles fonctions ester des produits décrits ci-dessus peuvent être, si désiré, saponifiées en fonction acide, ces réactions de saponification pouvant être réalisées dans les conditions usuelles connues de l'homme de métier notamment par hydrolyse acide ou alcaline par exemple par de la soude ou de la potasse en milieu alcoolique tel que, par exemple, dans du méthanol ou encore par de l'acide chlorhydrique ou sulfurique.

Les éventuelles fonctions alkyloxy telles que notamment méthoxy des produits décrits ci-dessus peuvent être, si désiré, transformées en fonction hydroxyle soit alcool dans les conditions usuelles connues de l'homme de métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.

Les éventuelles fonctions cyano des produits décrits ci-dessus peuvent être, si désiré, transformées en fonction acide dans les conditions usuelles connues de l'homme de métier par exemple par hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.

Les éventuelles fonctions carboxy estérifiées des produits décrits ci-dessus peuvent, si désiré, être réduites en fonction alcool par les méthodes connues de l'homme de métier et notamment par de l'hydrure de lithium et d'aluminium dans un solvant tel que par exemple le tétrahydrofuranne ou encore le dioxane ou l'éther éthylique.

Les éventuelles fonctions carboxy des produits décrits ci-dessus peuvent, si désiré, être réduites en fonction alcool par les méthodes connues de l'homme de métier et peuvent ainsi par exemple être d'abord estérifiées puis transformées en fonction alcool par exemple ainsi qu'il est indiqué ci-dessus.

Les éventuelles formes optiquement actives des produits de formule (I) peuvent être préparées par dédoublement des racémiques selon les méthodes usuelles.

Les composés de formule (I) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Certains produits de la présente invention possèdent également des propriétés antagonistes pour le récepteur à l'endothéline et sont ainsi notamment antagoniste de l'effet vasoconstricteur de l'endothéline.

Les composés de formule (I) possèdent également la propriété d'améliorer les fonctions cognitives.

Ces propriétés justifient leur application en thérapeutique et l'invention a également pour objet à titre de médicaments, les produits tels que définis par les formules (I) ci-dessus, lesdits produits de formules (I) étant sous toutes les formes isoméres possibles racémiques ou optiquement actives, ainsi que les sels d'addition avec les acides minéraux ou organiques pharmaceutiquement acceptables desdits produits de formule (I).

L'invention a tout particulièrement pour objet l'utilisation des produits tels que définis ci-dessus répondant à la formule (Ia) :

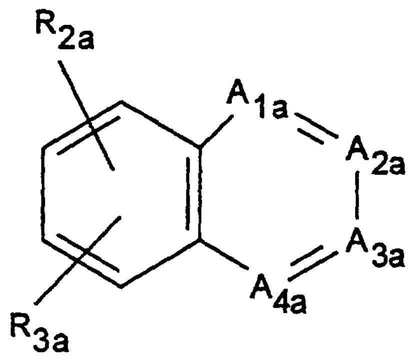

(Ia)

dans laquelle :

$R_{2a}$ et $R_{3a}$, identiques ou différents, sont choisis dans le groupe formé par :

- l'atome d'hydrogène,
- les atomes d'halogène,
- le radical hydroxyle,
- le radical mercapto, cyano, nitro, formyle, benzoyle, acyle ayant au plus 6 atomes de carbone,
- les radicaux carboxy libre, salifié ou estérifié par un radical alkyle linéaire ou ramifié renfermant au plus 4 atomes de carbone,
- les radicaux alkyle, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole et isoxazole,
- les radicaux amino, mono- ou dialkylamino, carbamoyle, pyrrolyle, morpholino, pipérazinyle, pyrrolylméthyle, morpholinométhyle, pipérazinyleméthyle, pyrrolylcarbonyle, morpholinocarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,

$A_{1a}$, $A_{2a}$, $A_{3a}$ et $A_{4a}$, identiques ou différents, représentent un atome d'azote et le radical $=C-R_{4a}$ tel que :
soit $R_{4a}$ représente le radical $R_{1a}$ tel que $R_{1a}$ représente :

- l'atome d'hydrogène, le radical hydroxyle, cyano, carboxy libre, salifié ou estérifié par un radical alkyle renfermant au plus 4 atomes de carbone,
- le radical alkyle, alkényle, alkyloxy, acyle ou alkylthio, ces radicaux étant linéaires ou ramifiés et renfermant au plus 7 atomes de carbone, le radical phényle, benzyle, phénoxy, phénylthio, tous ces radicaux aliphatiques ou cycliques étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, tétrazole et isoxazole,

soit $R_{4a}$ représente le radical - $R_{5a}$ - Ya dans lequel :

- $R_{5a}$ représente un radical $-CH_2-$, -NH-, -O-, $-OCH_2-$ ou $-SCH_2-$ et - Ya représente un radical phényle substitué par un radical tétrazole ou isoxazole ou biphényle éventuellement substitué par un ou plusieurs radicaux choisis parmi les radicaux hydroxyle, halogène, alkyle et alkyloxy renfermant au plus 4 atomes de carbone, trifluorométhyle, cyano, nitro, carboxy libre, salifié ou estérifié, tétrazole et isoxazole,
  étant entendu que :
  a) les produits de formule (Ia) sont tels que $A_{1a}$, $A_{2a}$, $A_{3a}$ et $A_{4a}$ sont tels que l'un au moins et deux au plus représentent un atome d'azote et l'un au moins représente le radical méthine substitué par le radical - $R_{5a}$ -

Ya, à l'exception des produits dans lesquels :

$A_{1a}$ représente un atome d'azote,
$A_{2a}$ représente le radical $=C-R_{4c}$ dans lequel $R_{4c}$ représente un atome d'hydrogène, un radical alkyle éventuellement substitué par un ou plusieurs atomes de fluor, les radicaux hydroxyle ou alcoxy (1-4C) ou un radical phényle,
$A_{3a}$ représente le radical $=C-R_{4a}$ dans lequel $R_{4a}$ représente un atome d'hydrogène, un radical alkyle, carboxy, libre estérifié ou salifié, cyano, nitro, phényle ou phénylalkyle,
$A_{4a}$ représente le radical $=C-R_{4a}$ dans lequel $R_{4a}$ représente le radical $-R_{5a}-Y_a$ dans lequel $R_{5a}$ représente le radical $-O-CH_2-$, Y représente le radical phényle éventuellement substitué par tétrazole ou phényle, lui-même éventuellement substitué par un substituant choisi parmi les radicaux tétrazolyle, carboxy éventuellement salifié ou estérifié, alkyle, alkoxy, halogène, trifluorométhyle, cyano et nitro,
$R_{2a}$ et $R_{3a}$ sont choisis parmi l'atome d'hydrogène ; les atomes d'halogène ; le radical hydroxyle ; trifluorométhyl ; cyano ; nitro ; alkyle renfermant au plus 4 atomes de carbone éventuellement substitué par hydroxyle ou alcoxy renfermant au plus 4 atomes de carbone ; alcoxy renfermant au plus 4 atomes de carbone éventuellement substitué par un atome de fluor ; alkylthio renfermant au plus 6 atomes de carbone ; carbamoyle ; amino éventuellement substitué par un ou deux radicaux alkyle pour renfermer au plus 6 atomes de carbone ; carboxy ; alcoxycarbonyl renfermant au plus 4 atomes de carbone ; acyl renfermant au plus 4 atomes de carbone.

B) Les 4 produits suivants :

Chlorhydrate d'acide 4-[[2-butyl 4-quinoléinyl) oxy] méthyl] benzoïque.
4'-[[2-butyl 4-quinoléinyl) oxy] méthyl] (1,1'-biphényl)-2-carboxylate de méthyle.
4'-[[(3-butyl 5-méthylthio 4-quinoléinyl) oxy] méthyl] (1,1'-biphényl)-2-carboxylate de méthyle.
Sel de diéthylamine de l'acide 4'-[[(3-butyl 1,4-dihydro 4-(méthylthio) 4-quinoléinyl) oxy] méthyl] (1,1'-biphényl) 2-carboxylique.
Acide 4'-[[(3-butyl 4-quinoléinyl) oxy] méthyl] (1,1'-biphényl)-2-carboxylique, appartiennent à la présente invention, lesdits produits de formule (Ia) et les produits indiqués en
b) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule ($I_a$)et des produits indiqués en b) pour la préparation de médicaments destinés, soit au traitement de l'hypertension artérielles, des insuffisances cardiaques, des insuffisances rénales et dans la préparation des resténoses post-angioplastie, soit au traitement de certains désordres gastro-intestinaux ou gynécologiques. De tels produits de formule (Ia) peuvent être préparés par le procédé tel que défini ci-dessus et/ou par analogie.

Les médicaments, objet de l'invention, peuvent être utilisés dans le traitement des affections cardiovasculaires présentant une altération de la vasomotricité : infarctus du myocarde, insuffisance cardiaque, insuffisance rénale, angine de poitrine, spasme vasculaire cérébral, maladie de Raynaud, hypertension artérielle et toutes les affections consécutives à une ischémie. Ces médicaments, objet de l'invention, pourraient également être utilisés pour le traitement du glaucome, de l'atherosclérose, de l'asthme et de différents types de spasmes viscéraux, ainsi qu'à titre de substance protectrice neuromale ou encore dans la prévention des resténoses post-angioplastie.
Ils peuvent également être utilisés dans le traitement de certains désordres gastro-intestinaux, gynécologiques et en particulier pour un effet relaxant au niveau de l'utérus.
Ces médicaments objets de l'invention peuvent également être utilisés dans le traitement de troubles de la mémoire, de la démence sénile et de la maladie d'Alzheimer.
L'invention s'étend aux compositions pharmaceutiques contenant à titre de principe actif l'un au moins des médicaments tels que définis ci-dessus.
Ces compositions pharmaceutiques peuvent être administrées par voie buccale, rectale, par voie parentérale ou par voie locale en application topique sur la peau et les muqueuses.
Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les composés de départ de formules (III), (V), (VI), (VII), (IX), (X), (XII), (XIII), (XIV), (XV) et (XVI) peuvent être disponibles dans le commerce ou peuvent être préparées selon les méthodes usuelles connues de l'homme de métier.

Les composés de formule (III) peuvent constituer des dérivés du chlorure de phénylacétyle.

Parmi les exemples de préparation de tels composés de formule (III) décrits dans la littérature, on peut citer notamment les références suivantes :

- Org. Synth. 1972, 36 ;
- Can. J. Chem. 1957, 35, 651.

Les composés de formule (V) peuvent constituer des dérivés de type nitrile.

Parmi les exemples de préparation de tels composés de formule (V) décrits dans la littérature, on peut citer notamment les références suivantes :

- Synthesis 1987, 514.

Les composés de formule (VI) peuvent constituer des dérivés de l'aniline.

Comme composés de formule (VI) qui peuvent être trouvés dans le commerce, on peut citer par exemple certains composés de formule (VI) telle que l'un de $R_2$ ou $R_3$ représente un radical carbométhoxy, comme par exemple, l'anthranilate de méthyle, le 3-amino benzoate de méthyle, que le l'on peut trouver, par exemple, sous forme de produits commercialisé par exemple par Aldrich.

Les composés de formule (VII) peuvent constituer des esters dérivés de l'acide formylacétique.

Parmi les exemples de préparation de tels composés de formule (VII) décrits dans la littérature, on peut citer notamment les références suivantes :

- J. Het. Chem. 1983, 20, 623,
- Liebigs Ann. Chem. 1966, 697, 62.

Les composés de formule (IX) peuvent constituer notamment des dérivés de l'ortho halo aniline.

Parmi les exemples de préparation de tels composés de formule (IX) décrits dans la littérature, on peut citer notamment les références suivantes :

- Ann. Chim. 1962, 52, 727.

Les composés de formule (X) peuvent constituer notamment des dérivés de l'acétylène.

Parmi les exemples de préparation de tels composés de formule (X) décrits dans la littérature, on peut citer notamment les références suivantes :

- J. Am. Chem. Soc. 1937, 59, 1490.

Les composés de formule (XIV) peuvent constituer des dérivés de l'acide phtalique.

Parmi les exemples de préparation de tels composés de formule (XIV) décrits dans la littérature, on peut citer notamment les références suivantes :

- J. Org. Chem. 1963, 28, 582,
- J. Chem. Soc. 1952, 553.

Les composés de formule (XV) peuvent constituer des dérivés de la cyanoaniline.

Ces composés peuvent être trouvés dans le commerce ; on peut citer par exemple le 2-aminobenzonitrile, commercialisé par exemple par Aldrich, ou encore la 5-chloro 2-cyanoaniline, commercialisée par exemple par Bayer.

Les composés de formule (XVI) peuvent constituer par exemple des dérivés de chlorure d'acyle.

Parmi les exemples de préparation de tels composés de formule (XVI) décrits dans la littérature, on peut citer notamment les références suivantes :

- Org. Synth. Coll. Vol. III, p. 190.

D'autre part, certains produits intermédiaires peuvent être trouvés dans le commerce tel que par exemple des dérivés chlorés tel que le composé 4 chloro 2 phénylquinazoline qui peut être fourni par exemple par ALDRICH: un exemple de préparation de ce composé peut être trouvé dans la référence suivante :

- J. Org. Chem., 37, 1681 (1972)

Le composé 4 chloro 2-phénylquinazoline permet de préparer des produits de formule (I) dérivés de la 2-phénylquinazoline dans lesquels par l'intermédiaire par exemple d'un organométallique tel que par exemple le composé $R_4$ - Zn - Br le radical $R_4$ tel que défini ci-dessus peut être introduit par substitution sur l'atome de chlore ou d'un radical trifluorométhane sulfonate par exemple dans les conditions usuelles connues de l'homme de métier.
Les produits de formule (I), ainsi qu'il a été indiqué ci-dessus, sont tels que $A_1$, $A_2$ et $A_3$ représente un atome d'azote ou le radical méthine substitué par $R_4$.
Lorsque le radical - $R_5$ - Y représente le radical biphénylméthyle, un procédé de préparation d'un tel radical peut, par exemple, consister à soumettre le iodobenzoate de méthyle à l'action du iodotoluène, la réaction se réalisant par exemple en présence de cuivre en poudre à une température d'environ 100°C à 300°C, pour obtenir un produit de formule (c) :

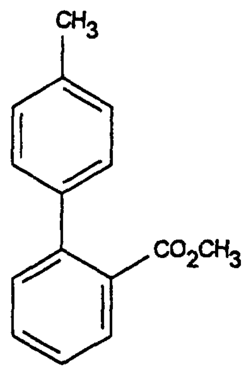

(c)

dont le radical carboxy estérifié peut, si désiré, être libéré du radical alkyle par les méthodes classiques connues de l'homme de métier ou indiquées ci-dessus, par exemple d'hydrolyse acide ou alcaline, que l'on peut soumettre à une réaction de bromation sur le radical méthyle par les méthodes classiques connues de l'homme de métier par exemple par action du n-bromosuccinimide dans le tétrachlorure de carbone.
Des exemples de préparation de tels composés de formule - $R_5$ - Y telle que définie ci-dessus sont décrits dans la littérature et des exemples en sont donnés notamment dans le brevet US 4,880,804.
Parmi les produits de formule (I) particulièrement préférés se trouvent également les produits répondant aux formules indiquées ci-dessous dans lesquelles $R_2$, $R_3$ et - $R_5$- Y ont les significations indiquées ci-dessus et n-bu représente le radical n-butyle :

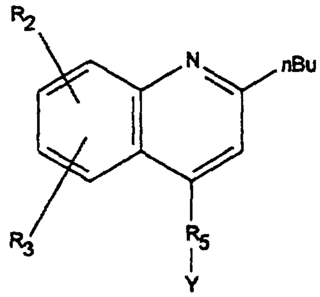

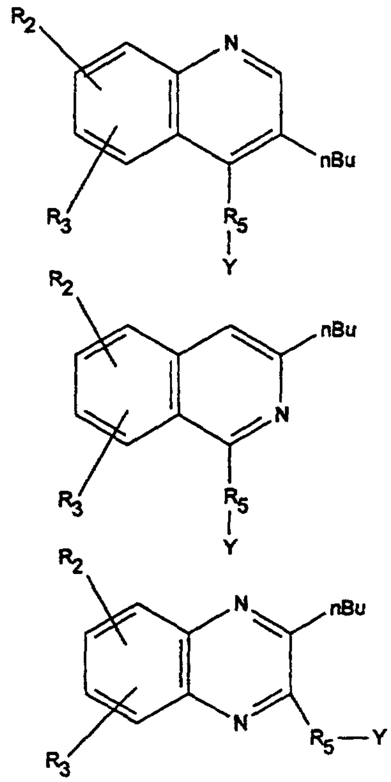

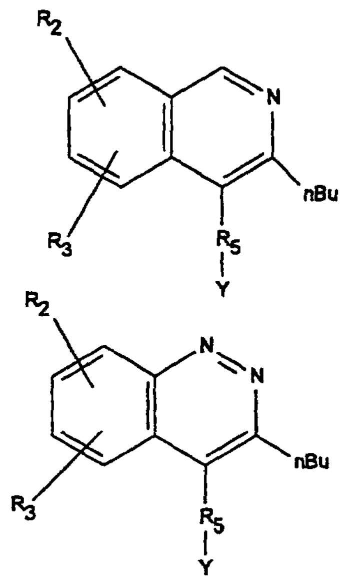

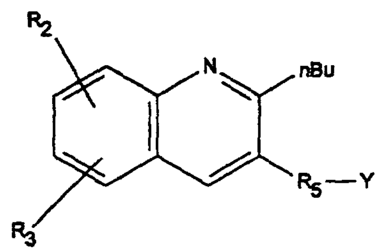

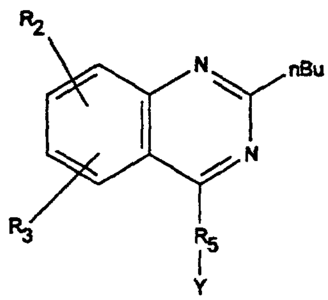

Parmi les produits dont les schémas sont indiqués ci-dessus, Y représente notamment un radical biphényle substitué par un radical carboxy libre, salifié ou estérifié, cyano, tétrazolyle éventuellement salifié que défini ci-dessus.
Les exemples suivants illustrent l'invention sans toutefois la limiter.

**<u>Exemple 1</u> : 4'-[[3-butyl 4-quinoléinyl] méthyl] (1,1'-biphényl) 2-carboxylate de méthyle.**

On introduit 475 mg de zinc électrolytique, 275 mg de tétrakis(triphénylphosphine) palladium, 2,08 g de 4-(bromométhyl) (1,1-biphényl) 2-carboxylate de méthyle (préparé selon EP 0025331)) dans 13 $cm^3$ de tétrahydrofuranne. On introduit 503 mg de 3-butyl 4-chloro quinoléine obtenu au stade F de la préparation décrite ci-après. On agite 15 heures dans un bain à ultrasons en laissant la température monter à 65°C. On ajoute 50 $cm^3$ d'acide chlorhydrique 0,1N et extrait à l'acétate d'éthyle, lave avec une solution saturée de chlorure de sodium, sèche et évapore à sec. Après chromatographie sur silice (éluant : chloroforme-hexane-acétate d'éthyle 100-100-10), on obtient 624 mg de produit attendu.
IR $CHCl_3$

| | |
|---|---|
| COOMe | : 1720 à 1436 $cm^{-1}$ |
| aromatique et hétérocycle | : 1615, 1600, 1574, 1508 $cm^{-1}$ |

**Préparation de l'exemple 1 : 3-butyl 4-chloro quinoléine.**

Le produit utilisé au départ de l'exemple 1 a été préparé de la façon suivante :

Stade A : 2-butyl 3-oxo butanedioate de diéthyle.

A une solution d'éthylate de sodium préparée par agitation pendant 1 heure à 40°C de 2,3 g de sodium et 150 $cm^3$ d'éthanol, on ajoute 100 $cm^3$ d'éther puis 13,55 $cm^3$ de diéthyloxalate. On chauffe 15 minutes au reflux, refroidit légèrement et ajoute 50 $cm^3$ de caproate d'éthyle, agite 3 heures au reflux et 16 heures à 30-35°C. On ajoute 50 $cm^3$ d'eau, sépare la phase aqueuse par décantation, la lave avec de l'éther par deux fois et acidifie avec de l'acide chlorhydrique 2N. On extrait 3 fois à l'éther, lave les phases organiques à l'eau puis avec une solution saturée de chlorure de sodium, sèche et évapore à sec. Le produit obtenu, 9,5 g est utilisé tel quel pour le stade suivant.

Stade B : 2-butyl 3-(phénylamino) 2-butènedioate de diéthyle.

On agite 3 jours à 75°C un mélange de 1 g d'aniline avec 2,65 g du produit obtenu au stade A ci-dessus et 150 mg de silipointe $^{R}$ NK 10, refroidit et chromatographie le milieu réactionnel sur silice (éluant : hexane-acétate d'éthyle 9-1). On obtient 1,55 g de produit attendu.
Spectre IR ($CHCl_3$)

| | |
|---|---|
| =C-NH | : 3260 $cm^{-1}$ |
| C=O | : 1733, 1656 $cm^{-1}$ |
| C=C + aromatique | : 1610, 1596, 1584, 1500 $cm^{-1}$ |

Stade C : 3-butyl 1,4-dihydro 4-oxo 2-quinoléinecarboxylate d'éthyle et 3-butyl 4-hydroxy 2-quinoléine carboxylate d'éthyle.

On chauffe pendant 30 minutes à l'aide d'un bain à 250°C un mélange de 2,5 g du produit obtenu au stade B ci-dessus avec 30 $cm^3$ de diphényléther. On laisse revenir à température ambiante, essore, lave avec du pentane et recueille 1,82 g de produit recherché.
Spectre IR ($CHCl_3$)

| | |
|---|---|
| =C-NH | : 3425, 3383 $cm^{-1}$ |
| C=O | : 1746, 1706 $cm^{-1}$ |
| autre C=O, C=C, aromatique | : 1624, 1605, 1585, 1572, 1532 $cm^{-1}$ |

Stade D : Acide 3-butyl 1,4-dihydro 4-oxo 2-quinoléinecarboxylique.

On chauffe 1 heure à 60°C 1,8 g de l'ester obtenu au stade C ci-dessus avec 25 $cm^3$ de soude en solution N. On refroidit et acidifie avec de l'acide chlorhydrique N, filtre, lave à l'eau et sèche à 60°C sous pression réduite. On obtient 1,58 g du produit recherché.
Spectre RMN (60 mHz, DMSO, ppm).

| | |
|---|---|
| 0,89 (t) | :$CH_3$-$CH_2$-$CH_2$-$CH_2$ |
| 1,39 (m) | : C-$\underline{C}$-$\underline{C}$-C |
| 2,78 | : C-C-C-$\underline{C}$- |
| 7,30 (t)-7,63 (t) | : $H_6$ et $H_7$ |
| 7,80 (d)-8,08 (d) | : $H_5$ et $H_8$ |
| 11,64 | : proton mobile |

Stade E : 3-butyl 4(1H)-quinolone.

On chauffe 30 minutes à 250°C un mélange de 1,55 g du produit obtenu au stade D ci-dessus et 10 $cm^3$ de diphényléther. On refroidit, filtre, lave avec du pentane et sèche sous pression réduite. On obtient 1,17 g de produit que l'on dissout dans 80 $cm^3$ d'éthanol et traite 15 minutes au reflux en présence de charbon actif, on filtre sur hyflosupercel, évapore l'éthanol jusqu'à sec et reprend avec du pentane, filtre, lave avec du pentane et sèche à 50°C sous pression réduite. On obtient 0,971 g de produit recherché.
Spectre IR ($CHCl_3$)

(mélange 2 formes : énol et céto)

| | |
|---|---|
| =C-NH | : 3440 $cm^{-1}$ |
| autre C=O, C=C, aromatique | : 1632, 1590, 1570, 1558, 1524, 1506 $cm^{-1}$ |

Stade F : 3-butyl 4-chloro quinoléine.

On chauffe 2 heures à 120°C un mélange de 515 mg du produit obtenu au stade E ci-dessus avec 0,6 $cm^3$ d'oxychlorure de phosphore. On refroidit, ajoute 10 $cm^3$ d'eau et alcalinise à pH 9 avec de l'ammoniaque concentré. On extrait 2 fois avec du chlorure de méthylène, lave avec une solution saturée de chlorure de sodium, sèche et évapore à sec. Le résidu est dissous dans 30 $cm^3$ d'éthanol et traite 15 minutes au reflux en présence de charbon actif, on filtre sur hyflosupercel, on obtient 511 mg du produit recherché.

| RMN $CDCl_3$ (250 MHz) | |
|---|---|
| $\underline{CH_3}$-$CH_2$-$CH_2$-$CH_2$-C- | 0,98 (t) |
| $CH_3$-$\underline{CH_2}$-$CH_2$-$CH_2$-C- | 1,45 (m) |
| $CH_3$-$CH_2$-$\underline{CH_2}$-$CH_2$-C- | 1,68 (m) |
| $CH_3$-$CH_2$-$CH_2$-$\underline{CH_2}$-C- | 2,96 (m) |
| | 7,63 (dt) |
| H aromatiques | 7,72 (dt) |
| | 8,10 (dd) |
| | 8,25 (dd) |
| H noyau pyridine | 8,74 (s) |

**Exemple 2 : Acide 4'-[(3-butyl 4-quinoléinyl) méthyl] (1,1'-biphényl)-2-carboxylique.**

On introduit 550 mg du produit obtenu à l'exemple 1 dans 16 $cm^3$ de soude et 3 $cm^3$ de méthanol. On agite pendant 3 heures et demie à la température de 80°C. On ajoute 1 $cm^3$ de lessive de soude et 1 $cm^3$ de méthanol et chauffe de nouveau pendant 7 heures. On évapore le méthanol, reprend à l'eau et acidifie avec de l'acide chlorhydrique concentré, filtre, reprend et lave le précipité avec de l'eau, sèche sous pression réduite à 60°C pendant 24 heures. Le précipité est dissous en chauffant dans 50 $cm^3$ de chlorure de méthylène. On filtre, évapore et chromatographie sur silice (éluant : chlorure de méthylène seul puis chlorure de méthylène-méthanol 9-1). L'élution est filtrée, évaporée et reprise à l'éther. Le produit cristallisé est séché sous pression réduite à 60°C en présence de pentoxyde de phosphore. On obtient 422 mg de produit attendu.
Spectre IR (nujol)

| | |
|---|---|
| C=O | : 1695 $cm^{-1}$ |
| aromatiques + hétérocyclique | : 1610, 1595, 1575, 1510 $cm^{-1}$ |

| Analyse : $C_{27}H_{25}NO_2$ = 395,51 | | | |
|---|---|---|---|
| Calculé : | C% 81,09 | H% 6,37 | N% 3,54 |
| Trouvé : | 82,00 | 6,30 | 3,40 |

**Exemple 3 : 4'-[[2-butyl 4-quinoléinyl] méthyl] (1,1'-biphényl) 2-carboxylate de méthyle.**

On opère comme à l'exemple 1 à partir de 500 mg de 2-butyl 4-chloro quinoléine obtenu comme indiqué ci-après au stade D de la préparation de l'exemple 3 et introduit dans une solution préparée au préalable de 475 mg de zinc électrolytique, 275 mg de tétrakistriphénylphosphine palladium et 2,075 g de bromure de [[2'-(méthoxycarbonyl) (1,1'-biphényl) 4-yl] méthyl zinc préparé ainsi qu'il est indiqué à l'exemple 1 dans 13 $cm^3$ de tétrahydrofuranne. Après chromatographie sur silice (éluant : chloroforme-hexane-acétate d'éthyle 100-100-10), on obtient 621 mg de produit attendu.
Spectre IR ($CHCl_3$)

| | |
|---|---|
| -COOMe | : 1720, 1435 $cm^{-1}$ |

aromatiques + hétérocyclique : 1602, 1560, 1508, 1480 $cm^{-1}$

Le produit utilisé au départ de l'exemple 3 a été préparé comme suit :

**Préparation de l'exemple 3 : 2-butyl 4-chloro quinoléine**

Stade A :

A une suspension de 27,3 g d'hydrure de sodium à 50 % dans l'huile préalablement lavé à l'heptane) et 250 $cm^3$ d'éther, on ajoute 70,8 g de carbonate d'éthyle en solution dans 50 $cm^3$ d'éther. On agite 10 minutes, ajoute en 30 minutes 30 g d'hexanone et chauffe 2 heures au reflux. On ajoute 35 $cm^3$ d'éther contenant 12 $cm^3$ d'éthanol et agite 16 heures à température ambiante. On refroidit à 0°C et ajoute une solution de 36 $cm^3$ d'acide acétique dans 300 $cm^3$ d'eau puis 12 $cm^3$ d'une solution saturée de bicarbonate de sodium, le pH est alors de 7. On extrait avec de l'éther, lave à l'eau, sèche, évapore à sec. On obtient après distillation à 70°C sous pression réduite de 3 mbar, 32,5 g de produit recherché.

| Spectre RMN | |
|---|---|
| $\underline{CH_3}$-$CH_2$ | 0,91 ppm |
| les $CH_2$ centraux | 1,34 - 1,59 ppm |
| $CH_2$-C<br>O | 2,55 ppm (t) |
| $CO_2E^t$ | 1,28 ppm (t)<br>4,20 ppm (q) |
| C-$CH_2$-C<br>O O | 3,44 ppm (s) |

Stade B : 3-(phénylamino) 2-heptanoate d'éthyle.

On opère comme au stade B de la préparation de l'exemple 1 à partir de 45 g de produit obtenu comme au stade A, ci-dessus. On obtient, après chromatographie sur silice (éluant hexane-acétate d'éthyle (95-5)) 28,7 g du produit attendu.
Spectre IR ($CHCl_3$)

NH : 3260 $cm^{-1}$
C=O : 1648 $cm^{-1}$
C=C + aromatique : 1612, 1594, 1588 $cm^{-1}$

Stade C : 2-butyl 4(1H)-quinolone

On opère comme au stade C de la préparation de l'exemple 1 à partir de 28,7 g du composé obtenu au stade B ci-dessus. On obtient 16,95 g du produit recherché. F = 140°C.
Spectre IR ($CHCl_3$)

=C-NH : 3428 $cm^{-1}$ + absorption générale forte
C=O + C=C + C=N + aromatiques : 1636, 1596, 1547, 1502 $cm^{-1}$

Stade D : 4-chloro 2-butyl quinoléine.

On opère comme à l'exemple F de la préparation de l'exemple 1, à partir de 4 g du composé obtenu au stade C ci-dessus, on obtient 3,89 g de produit recherché.

| RMN $CDCl_3$ | |
|---|---|
| $\underline{CH_3}$-$CH_2$-$CH_2$-$CH_2$ | 0,97 (t) |
| $CH_3$-$\underline{CH_2}$-$CH_2$-$CH_2$ | 1,45 (m) |

(suite)

| | |
|---|---|
| $CH_3$-$CH_2$-<u>$CH_2$</u>-$CH_2$ | 1,72 (m) |
| $CH_3$-$CH_2$-$CH_2$-<u>$CH_2$</u> | 2,95 (m) |
| | 7,41 (dt) |
| H aromatiques | 7,74 (dt) |
| | 8,06 (dd) |
| | 8,19 (dd) |
| H pyridine | 7,41 (s) |

**Exemple 4 : Acide 4'-[(2-butyl 4-quinoléinyl) méthyl] (1,1'-biphényl)-2-carboxylique.**

On introduit 614 mg du produit obtenu à l'exemple 3 dans 10 $cm^3$ de soude 1N et 10 $cm^3$ d'éthanol. On chauffe à 70°C pendant 2 heures. On évapore l'éthanol, reprend à l'eau et acidifie avec de l'acide chlorhydrique 1N. On filtre le précipité, lave à l'eau et sèche sous pression réduite à 60°C. On reprend le précipité dans l'éthanol au reflux, traite avec du charbon actif et après filtration, on évapore et chromatographie sur silice (éluant : chlorure de méthylène-éthanol 9-1). Après cristallisation dans l'éther, on obtient 363 mg du produit attendu.

**Préparation du chlorhydrate.**

On reprend le produit obtenu ci-dessus dans 20 $cm^3$ de soude 0,1N et 10 $cm^3$ de soude N. On acidifie à pH = 1 la solution limpide avec de l'acide chlorhydrique concentré. Le précipité blanc est filtré, lavé à l'eau et séché sous pression réduite à 60°C. On recristallise dans l'éther et filtre de nouveau. On obtient 180 mg de chlorhydrate atendu. F # 227°C.

| Analyse : $C_{27}H_{25}NO_2$ = 395,51 | | | |
|---|---|---|---|
| Calculé | C% 81,99 | H% 6,37 | N% 3,54 |
| Trouvé | 81,8 | 6,50 | 3,40 |

| Analyse : $C_{27}R_{26}NClO_2$ = 431,97 | | | | |
|---|---|---|---|---|
| Calculé | C% 75,07 | H% 6,06 | N% 3,24 | Cl% 8,21 |
| Trouvé | 75,4 | 6,1 | 3,1 | 8,1 |

Spectre IR (Nujol)

| | |
|---|---|
| Absorption région OH/NH | : # 2640 $cm^{-1}$ |
| C=O complexe | : 1725 $cm^{-1}$ (ép), 1720 $cm^{-1}$ (max) 1708 $cm^{-1}$ (max) |
| aromatiques + hétérocyclique | : 1640, 1600, 1518, 1495 $cm^{-1}$ |

**Exemple 5 : Acide 4-[(3-butyl 4-quinoléinyl) amino] benzoïque.**

On introduit 0,520 g du produit obtenu au stade F de la préparation de l'exemple 1 et 0,390 g d'acide 4-amino benzoique dans 6 $cm^3$ d'acide chlorhydrique 2N. On chauffe au reflux pendant 63 heures, filtre le précipité, lave à l'eau, reprend par 10 $cm^3$ de soude 2N dans 40 $cm^3$ d'eau puis lave à l'acétte d'éthyle. La phase aqueuse est reprise à l'acide acétique, filtre le précipité, lave à l'eau, essore et reprend avec de l'éther. Après séchage, on obtient 220 mg du produit attendu.
F = 276°C.

**Préparation du chlorhydrate.**

On agite 156 mg du produit obtenu ci-dessus dans 10 $cm^3$ d'un mélange 50-50 de chlorure de méthylène-éthanol puis porte au reflux et filtre. Après évaporation, l'extrait sec est repris à l'éthanol à 96% et on ajoute de l'acide chlorhydrique concentré jusqu'à pH # 1. On évapore de nouveau et recristallise dans l'éther. Après filtration puis séchage sous pression réduite à 65°C, on obtient 162 mg du chlorhydrate attendu.

| Analyse : $C_{20}H_{20}N_2O_2$, HCl = 356,85 | | | | |
|---|---|---|---|---|
| Calculé | C% 67,32 | H% 5,93 | N% 7,85 | C1% 9,93 |
| Trouvé | 67,0 | 6,0 | 7,6 | 10,0 |

Spectre IR (Nujol)

OH/NH : 3260 $cm^{-1}$
-C=O : 1736, 1668 $cm^{-1}$
aromatiques, hétérocyclique : et $NH_2$ : 1605, 1570, 1518, 1500 $cm^{-1}$

**Exemple 6 : 4-[[(2-butyl 4-quinoléinyl) oxy] méthyl] benzonitrile.**

On introduit 0,6 g du produit obtenu au stade C de la préparation de l'exemple 3 dans 30 $cm^3$ d'acétone anhydre. On ajoute 0,828 g de carbonate de potassium, agite 10 minutes et ajoute 1,16 g de 4-(bromométhyl) benzonitrile. Le milieu réactionnel est porté au reflux 3 heures. L'acétone est évaporée et le résidu est repris dans 100 $cm^3$ d'eau. La phase aqueuse est extraite avec 3 fois 50 $cm^3$ d'acétate d'éthyle. La phase organique est lavée avec 100 $cm^3$ d'une solution saturée de chlorure d'ammonium, séchée et évaporée. Après chromatographie sur silice (éluant : chlorure de méthylène-méthanol 98-2), on obtient 0,78 g du produit attendu. F = 74°C.
Spectre IR ($CHCl_3$)

C≡N : 2236 $cm^{-1}$
C=C : 1621, 1598 $cm^{-1}$
aromatique : 1568, 1507 $cm^{-1}$

**Exemple 7 : Chlorhydrate d'acide 4-[[(2-butyl 4-quinoléinyl) oxy] méthyl] benzoïque.**

On introduit 0,75 g du produit obtenu à l'exemple 6 dans 20 $cm^3$ d'éthanol. Après agitation, on ajoute 2,1 $cm^3$ de soude 5N. On chauffe au reflux pendant une nuit. Le milieu réactionnel est refroidi à 0°C puis acidifié avec de l'acide chlorhydrique concentré. Les cristaux obtenus sont filtrés puis repris par 20 $cm^3$ d'éthanol et 15 $cm^3$ de soude concentrée. On chauffe 2 heures au reflux. La solution est refroidie à 0°C, acidifiée avec de l'acide chlorhydrique concentré puis agitée pendant 1 heure à 0°C. Les cristaux obtenus sont lavés à l'eau et séchés à 90°C. On obtient 0,45 g de produit recherché. F = 143°C. Après recristallisation dans 20 $cm^3$ d'éthanol, on obtient 0,295 g de produit attendu. F = 145°C.

| Analyse : $C_{21}H_{21}NO_3$, HCl = 371,81 | | | | |
|---|---|---|---|---|
| Calculé | C% 67,82 | H% 5,9 | N% 3,76 | Cl% 9,53 |
| Trouvé | 67,5 | 6,1 | 3,6 | 9,2 |

Spectre IR (Nujol)

C=O : 1700 $cm^{-1}$
C=C, C=N, aromatique : 1640, 1612, 1599, 1576, 1536, 1490 $cm^{-1}$

**Exemple 8 : 4'-[[(2-butyl 4-quinoléinyl) oxy] méthyl] (1,1'-biphényl)-2-carboxylate de méthyle.**

On opère comme à l'exemple 6 à partir de 0,45 g du produit obtenu au stade C de la préparation de l'exemple 3 dans 25 $cm^3$ d'acétone anhydre en utilisant 0,6 g de carbonate de potassium et 0,7 g de 4-(bromométhyl) (1,1'-biphényl) 2-carboxylate de méthyle (préparée selon EP 0 253 310). Après chromatographie sur silice (éluant : chlorure de méthylène-méthanol 98-2), on obtient 0,8 g du produit attendu.
F = 105°C.
Spectre IR ($CHCl_3$)

COOMe : 1720, 1436 $cm^{-1}$
aromatique, hétéroatome : 1620, 1598, 1568, 1507, 1485 $cm^{-1}$

**Exemple 9 : Acide 4'-[[(2-butyl 4-quinoléinyl) oxy] méthyl] (1,1'-biphényl) -2-carboxylique.**

On introduit 0,75 g du produit obtenu à l'exemple 8 dans 120 $cm^3$ de soude 1N. On chauffe à 90°C pendant 5 heures puis ajoute 20 $cm^3$ d'éthanol. Le milieu est agité 1 heure à 90°C puis 1 nuit à température ambiante. L'éthanol est évaporé, on refroidit à 0°C et acidifie à l'acide chlorhydrique concentré. Après agitation pendant 1 heure à 0°C, les cristaux sont essorés, lavés à l'eau et séchés à 50°C. On recueille 0,70 g de produit que l'on recristallise dans 150 $cm^3$ d'éthanol. On obtient 550 mg du produit attendu. F = 145°C.

| Analyse : $C_{27}H_{25}NO_3$ = 411,51 | | | |
|---|---|---|---|
| Calculé | C% 78,8 | H% 6,12 | N% 3,4 |
| Trouvé | 78,6 | 6,12 | 3,3 |

Spectre IR (Nujol)

Absorption région OH/NH=0 : 1710 $cm^{-1}$
hétérocycle et aromatique : 1599, 1570, 1499 $cm^{-1}$

**Exemple 10 : 4-[(2-butyl 4-quinoléinyl) oxy] benzoate d'éthyle.**

On introduit 0,950 g du produit obtenu au stade D de la préparation de l'exemple 3 et 2,2 g de 4-hydroxy benzoate d'éthyle et chauffe à 170°C pendant une demi-heure sous agitation. Après refroidissement, le milieu réactionnel est chromatographié sur silice (éluant : hexane-acétate d'éthyle 9-1). On obtient 0,250 g de produit attendu. Spectre IR ($CHCl_3$)

C=O : 1714 $cm^{-1}$
hétérocycle et aromatique : 1621, 1609, 1598, 1564, 1498 $cm^{-1}$

**Exemple 11 : Chlorhydrate de l'acide 4-[(2-butyl 4-quinoléinyl) oxy] benzoïque.**

a) Acide 4-[(2-butyl 4-quinoléinyl) oxy] benzoïque.

On introduit 250 mg du produit obtenu à l'exemple 10 dans 6 $cm^3$ de soude 2N et 5 $cm^3$ d'éthanol. On chauffe à 80°C pendant 1 heure, évapore l'éthanol et reprend à l'eau.

b) Préparation du chlorhydrate.

On ajoute à la solution obtenue ci-dessus de l'acide chlorhydrique 2N jusqu'à pH = 1, filtre, lave à l'eau, sèche et recristallise le produit dans du pentane. On sèche à 60°C sous pression réduite en présence de pentoxyde de phosphore. On obtient 204 mg du produit attendu.

| Analyse : $C_{20}H_{19}NO_3$, HCl = 357,84 | | | | |
|---|---|---|---|---|
| Calculé | C% 67,13 | H% 5,63 | N% 3,91 | Cl% 9,91 |
| Trouvé | 67,1 | 5,5 | 3,8 | 9,9 |

Spectre IR (Nujol)

Absorption région OH/NH C=O : 1704 $cm^{-1}$
système conjugué + aromatique : 1644, 1608, 1594, 1538, 1500, 1490 $cm^{-1}$

**Exemple 12 : Chlorhydrate de l'acide 4-[(2-butyl 4-quinoléinyl) amino] benzoïque.**

On introduit 0,840 g du produit obtenu au stade D de la préparation de l'exemple 3 et 0,640 g d'acide amino benzoïque dans 15 $cm^3$ d'acide chlorhydrique 1N. On chauffe au reflux pendant 3 heures, filtre le précipité obtenu, lave à l'eau et sèche à 60°C sous pression réduite. On obtient 0,830 g du produit attendu.

| Analyse : $C_{20}H_{20}N_2O_3$, HCl = 356,85 | | | | |
|---|---|---|---|---|
| Calculé | C% 67,32 | H% 5,93 | N% 7,85 | Cl% 9,93 |
| Trouvé | 67,1 | 5,9 | 7,7 | 9,6 |

Spectre IR (Nujol)

Absorption région OH/NH C=O : 1710 $cm^{-1}$
aromatique + hétérocycle :1640, 1613, 1591, 1554, 1517, 1495 $cm^{-1}$

**Exemple 13 : 4'-[(3-butyl 4-cinnolinyl) méthyl] (1,1'-biphényl)-2-carboxylate de méthyle:**

On opère comme à l'exemple 1 à partir de 1,1 g de produit obtenu au stade C de la préparation décrite ci-après, 1,83 g de 4-(bromométhyl) (1,1' -biphényl) -2-carboxylate de méthyle (préparé selon EP 0 253 310), 0,4 g de zinc éthanolique, 700 mg de tétrakis(triphénylphosphine) palladium dans 100 $cm^3$ de tétrahydrofuranne. Le milieu réactionnel est placé sous ultrasons pendant 15 heures, puis repris par de l'eau et extrait à l'acétate d'éthyle. La phase organique est lavéd à l'eau, séchée et évaporée. Après chromatographie sur silice (éluant : chlorure de méthylène-acétonitrile 8-2), on obtient 760 mg du produit attendu.
Spectre IR ($CHCl_3$)

C=O : 1720 $cm^{-1}$
système conjugué + aromatique : 1614, 1599, 1567, 1535, 1515 $cm^{-1}$

**Préparation du produit utilisé au départ de l'exemple 13 : 3-butyl 4-chloro cinnoline**

Stade A : 2-(1-hexynyl) benzeneamine

A une solution de 4,4 g de 2-iodo aniline dans 100 $cm^3$ de triéthylamine, on ajoute 32 mg d'iodure de cuivre et 140 mg de chlorure de bistriphényl phosphine palladium et 2,3 $cm^3$ de 1-hexyne. On agite 15 heures à température ambiante. On évapore à sec et reprend à l'éther, filtre l'insoluble, le lave à l'éther et les fractions éthérées sont évaporées à sec. On chromatographie le résidu sur silice (éluant : hexane-acétate d'éthyle (9-1)). On obtient 3,27 g de produit recherché.
Spectre IR

$C_6H_4$-$NH_2$ : 3486 $cm^{-1}$
$NH_2$ def+ aromatique : 1613, 1570, 1493 $cm^{-1}$

Stade B : 3-butyl 4-hydroxy cinnoline

A une suspension à 0°C de 3,2 g du produit obtenu au stade A, ci-dessus, dans 100 $cm^3$ d'acide chlorhydrique concentré, on ajoute à 0°C, une solution de 2 g de nitrite de sodium dans 60 $cm^3$ d'eau, on agite 1 heure 30 à 0°C, puis 1 heure à 100°C. On verse le milieu réactionnel dans 100 $cm^3$ d'eau glacée, essore, lave à l'eau glacée. Le produit humide est repris par 100 $cm^3$ d'eau et alcalinise avec de l'ammoniaque concentrée. On essore, lave à l'eau et sèche à 70°C sous pression réduite. On obtient 1,47 g du produit attendu.
F = 180°C.
Spectre IR : Nujol
Absorption région OH/NH

C=C + Aromatique 1636 $cm^{-1}$
1604 $cm^{-1}$ (épaulement)
1580 $cm^{-1}$
1578 $cm^{-1}$
1498 $cm^{-1}$

Stade C : 3-butyl 4-chloro cinnoline

On opère comme au stade F de la préparation 1, à partir de 1,2 g du produit obtenu au stade B ci-dessus,

en utilisant 10 $cm^3$ d'oxychlorure de phosphore. Après chromatographie sur silice (éluant : hexane-acétate d'éthyle (6-4)) on obtient 1,16 g du produit recherché. F < 50°C.
Spectre IR : $CHCl_3$
C=C + aromatique : 1616, 1558 $cm^{-1}$

**Exemple 14 : Acide 4'-[(3-butyl 4-cinnolinyl) méthyl] (1,1(-biphényl) 2-carboxylique.**

On opère comme à l'exemple 2 à partir de 700 mg de produit obtenu à l'exemple 13 dans 10 $cm^3$ de soude 2N et 20 $cm^3$ d'éthanol. Le milieu réactionnel est proté au reflux pendant 2 heures, versé sur de l'eau et acidifié par addition d'acide chlorhydrique concentré. Le précipité est lavé à l'eau, séché sous pression réduite et recristallisé dans 20 $cm^3$ d'acétonitrile. On obtient 0,27 g du produit attendu. F = 210°C.

| Analyse : $C_{26}H_{24}N_2O_3$, HCl = 396,489 | | | |
|---|---|---|---|
| Calculé | C% 78,76 | H% 6,1 | N% 7,06 |
| Trouvé | 78,6 | 6,0 | 7,1 |

Spectre IR (Nujol)

Absorption région OH/NH C=O : 1718 $cm^{-1}$
système conjugué + aromatique : 1622, 1598, 1576, 1558, 1522 $cm^{-1}$

**Exemple 15 : 4-[[(3-butyl 4-cinnolinyl) oxy] méthyl] benzonitrile.**

On opère comme à l'exemple 6 à partir du produit obtenu au stade B de la préparation de l'exemple 13 et de 4-(bromométhyl) benzonitrile.

**Exemple 16 : Acide 4-[[(3-butyl 4-cinnolinyl) oxy] méthyl] benzoique.**

On opère comme à l'exemple 7 à partir du produit obtenu à l'exemple 15.

**Exemple 17 : 4'-[[(3-butyl 5-méthylthio 4-quinoléinyl) oxy] méthyl] (1,1'-biphényi)-2-carboxylate de méthyle.**

On prépare une solution de 1,1 g du produit obtenu au stade D de la préparation décrite ci-après et de 1,63 g de 4-(bromométhyl) (1,1'-biphényl)-2-carboxylate de méthyle (préparé selon EP 0 253 310) dans 50 $cm^3$ d'acétone sous agitation et on ajoute 1,2 g de bicarbonate de potassium. Le milieu réactionnel est porté au reflux pendant 2 heures. La suspension est essorée, l'insoluble est lavé par de l'acétone, filtré et séché. On chromatographie sur silice (éluant : acétate d'éthyle-hexane 5-5) et obtient 1,8 g du produit attendu.
Spectre IR ($CHCl_3$)

C=OMe : 1720, 1435 $cm^{-1}$
aromatique + hétéroatome : 1600, 1593, 1557, 1505, 1483 $cm^{-1}$

**Préparation de l'exemple 17: 3-butyl 5-méthylthio 4-(1H)-quinolone.**

Stade A : 2-butyl 3-[[3-(méthylthio) phényl] amino] 2-butènedioate de diéthyle.

A une solution de 4,3 g de 2-butyl 3-oxo butanedioate de diéthyle avec 2 $cm^3$ de 3-(méthylthio) aniline, dans 100 $cm^3$ de toluène, on ajoute 50 mg d'acide paratoluène sulfonique. On agite 4 heures au reflux en éliminant l'eau formée. On évapore à sec et chromatographie le résidu sur silice (éluant : chlorure de méthylène à 30 % d'hexane), on obtient 5,1 g du produit recherché. F = 55°C.
Spectre IR ($CHCl_3$)

=C-NH complexe : 3240 $cm^{-1}$
C=O : 1732 - 1658 $cm^{-1}$
C=C + aromatique : 1598, 1583, 1488 $cm^{-1}$

**Préparation de 2-butyl 3-oxo butanedioate de diéthyle**

A une solution d'éthylate de sodium, préparée par agitation pendant 1 heure à 40°C de 2,3 g de sodium et 150 $cm^3$ d'éthanol, on ajoute 100 $cm^3$ d'éther puis 13,55 $cm^3$ de diéthyloxalate. On chauffe 15 minutes au reflux, refroidit légèrement et ajoute 50 $cm^3$ de caproate d'éthyle, on agite 3 heures au reflux et 16 heures à 30-35°C. On ajoute 50 $cm^3$ d'eau, sépare la phase aqueuse, par décantation, la lave avec de l'éther par deux fois et acidifie avec de l'acide chlorhydrique 2N. On extrait 3 fois à l'éther, lave les phases organiques à l'eau puis avec une solution saturée de chlorure de sodium, sèche et évapore à sec. Le produit obtenu 9,5 g est utilisé tel quel pour le stade suivant.

Stade B : 3-butyl 1,4-dihydro 5-(méthylthio) 4-oxo 2-quinoléine carboxylate d'éthyle

On chauffe 45 minutes à 250°C, 1 g du produit obtenu au stade A. On refroidit et chromatographie sur silice le produit brut réactionnel (éluant : chlorure de méthylène) on obtient 700 mg du produit recherché. F = 80°C.
Spectre IR ($CHCl_3$)

| | |
|---|---|
| NH complexe | : 3330 $cm^{-1}$ (F) |
| C=O | : 1743 $cm^{-1}$, 1707 (f) |
| C=O + C=C + aromatique | : 1610, 1596, 1559, 1530 $cm^{-1}$ |

Stade C : Chlorhydrate de l'acide 3-butyl 1,4-dihydro 5-(méthylthio) 4-oxo 2-quinoléinecarboxylique

On agite 2 heures au reflux 0,63 g du produit obtenu au stade B ci-dessus dans 10 $cm^3$ d'une solution N de soude. On verse dans l'eau glacée, acidifie avec de l'acide chlorhydrique concentré, essore, lave à l'eau, sèche et empâte dans 100 $cm^3$ d'acétate d'éthyle. On obtient 495 mg du produit recherché. F = 240°C.
Spectre IR (Nujol)

| | |
|---|---|
| Absorption OH/NH | : 3328 $cm^{-1}$ |
| C=O | : 1744 $cm^{-1}$ |
| C=O + C=C + aromatique | : 1610, 1592, 1560, 1540, 1516 $cm^{-1}$ |

Stade D : 3-butyl 5-(méthylthio) 4 (1H)-quinolone

On chauffe 5 minutes à 260°C, 390 mg du produit obtenu au stade C. On obtient 300 mg de produit recherché. F = 144°C.
Spectre IR ($CHCl_3$)

| | |
|---|---|
| =C-NH | : 3365 $cm^{-1}$ |
| C=O + C=C + aromatique | : 1627, 1609, 1585, 1560, 1520 $cm^{-1}$ |

**EXEMPLE 18 : Sel de diéthylamine de l'acide 4'-[[(3-butyl 1,4-dihydro 5-(méthylthio) 4-quinoléinyl) oxy] méthyl] (1,1'-biphényl) 2-carboxylique.**

On agite pendant 2 heures au reflux un mélange de 1,8 g du produit obtenu à l'exemple 17 avec 20 $cm^3$ d'une solution de soude 5N dans 20 $cm^3$ d'éthanol. On coule dans l'eau et acidifie avec de l'hydrogénophosphate de sodium, extrait avec de l'acétate d'éthyle, lave à l'eau, sèche et évapore à sec. On chromatographie le résidu sur silice (éluant : chlorure de méthylène-méthanol (9-1)). Le produit obtenu est dissous dans 50 $cm^3$ d'acétate d'éthyle, on ajoute 0,15 $cm^3$ de diéthylamine et agite 30 minutes. Après essorage et lavage par 2 fois 10 $cm^3$ d'acétate d'éthyle puis par 50 $cm^3$ d'éther isopropylique, on sèche à la température ambiante. On obtient 380 mg du produit attendu. F = 160°C.

| Analyse : $C_{32}H_{38}N_2O_3S$ = 530,735 | | | | |
|---|---|---|---|---|
| Calculé | C% 72,42 | H% 7,22 | N% 5,28 | S% 6,04 |
| Trouvé | 71,6 | 7,1 | 5,1 | 5,9 |

Spectre IR ($CHCl_3$)
Absorption type $NH_2$- et ou -$N^+$-H ~ 3100 --> 2200 $cm^{-1}$

C=O + aromatique : 1624, 1592, 1558, 1516 $cm^3$

**Exemple 19 : 3-butyl 4-[[2'-méthoxycarbonyl) (1,1'-biphényl) 4-yl] méthoxy] 2-quinoléinecarboxylate d'éthyle.**

On opère comme à l'exemple 8 à partir de 0,2 g du produit obtenu au stade C de la préparation de l'exemple 1 dans 5 $cm^3$ d'acétone anhydre et ajoute 0,2 g de carbonate de potassium. Après agitation, on ajoute 0,21 g de 4-(bromométhyl) (1,1'-biphényl) 2-carboxylate de méthyle (préparé selon EP 0 253 310). Après chromatographie sur silice (éluant : chlorure de méthylène-méthanol-99-1), on obtient 180 mg du produit attendu.
Spectre IR ($CHCl_3$)

=O : 3100 --> 2200 $cm^{-1}$
système conjugué+aromatique : 1618, 1599, 1584, 1562, 1492 $cm^3$

**Exemple 20 : Acide 3-butyl 4-[[2'-carboxy (1,1'-biphényl) 4-yl] méthoxy] 2-quinoléinecarboxylique.**

On opère comme à l'exemple 9 à partir de 0,3 g du produit obtenu à l'exemple 19 dans 5 $cm^3$ de soude concentrée. On ajoute 2 $cm^3$ d'eau et 5 $cm^3$ d'éthanol et chauffe pendant 3 heures au reflux. L'éthanol est évaporé, la solution refroidie à 0°C et acidifiée avec de l'acide chlorhydrique. Les cristaux sont essorés, lavés avec de l'eau et séchés. Après recristallisation dans 10 $cm^3$ de diméthylformamide à ~ 1% d'eau, on obtient 0,176 g du produit attendu.
F = 162°C.

| Analyse : $C_{28}H_{25}NO_5$ = 455,52 | | | |
|---|---|---|---|
| Calculé | C% 73,12 | H% 5,68 | N% 3,16 |
| Trouvé | 72,8 | 5,7 | 3,1 |

Spectre IR (Nujol)

=O : 1710, 1672 $cm^{-1}$
aromatique + hétéroatome : 1645, 1615, 1603, 1522, 1485 $cm^3$

**Exemple 21 : Acide 4'-[[(3-butyl 4-quinoléinyl) oxy] méthyl] (1,1'-biphényl)-2-carboxylique.**

Le produit est obtenu à partir du produit obtenu à l'exemple 20 par décarboxylation dans l'oxyde de biphényle en chauffant à 250°C avant 5 minutes.

**Exemple 22 : 4'-[(2-phényl 4-quinazolinyl) méthyl] (1,1'-biphényl)-2-carboxylate de méthyle.**

On prépare au préalable le bromure de [[2'-(méthoxycarbonyl) (1,1'-biphényl) 4-yl] méthyl] zinc à partir de 400 mg de zinc dans 1 $cm^3$ de tétrahydrofuranne et 1,53 g de 4'-(bromométhyl) (1,1'-biphényl)-2-carboxylate de méthyle (Réf. P. KNOCHEL J. Org. Chem. 1988, vol. 53, p. 5789-5791). On ajoute à la solution ainsi obtenue 240,69 mg de 4-chloro 2-phényl quinazoline et 115 mg de complexe de palladium tétrakis(triphénylphosphine) et laisse à 50°C pendant 6 heures. Le milieu réactionnel est versé sur un mélange eau/glace/acide acétique. La phase aqueuse est extraite à l'acétate d'éthyle. La phase organique est lavée à l'eau, à l'eau saturée en chlorure de sodium, séchée et évaporée. Après chromatographie sur silice (éluant : acétate d'éthyle-hexane 1-9 puis 2-8), on obtient 260 mg du produit attendu.
F = 121-122°C après recristallisation dans l'éther isopropylique.
Spectre IR ($CHCl_3$)

C=O : 1722 $cm^{-1}$
aromatique + système conjugué : 1618, 1600, 1534, 1520, 1459 $cm^3$

**Exemple 23 : Acide 4'-[(2-phényl 4-quinazolinyl) méthyl] (1,1'-biphényl) 2-carboxylique.**

On introduit 240 mg du produit obtenu à l'exemple 22 dans 2 $cm^3$ d'éthanol et ajoute 0,55 $cm^3$ de soude 2N. La solution est agitée 2 jours à la température ambiante puis portée au reflux pendant 2 heures. La solution est alors évaporée à sec, reprise par de l'eau et neutralisée avec de l'acide chlorhydrique concentré. Le précipité est filtré, séché et recristallisé dans un mélange eau-alcool isopropylique 10-90. On obtient 150 mg du produit attendu. F = 210°C.

| Analyse : $C_{28}H_{20}N_2O_2$ = 416,48 | | | |
|---|---|---|---|
| Calculé | C% 80,75 | H% 4,84 | N% 6,72 |
| Trouvé | 80,7 | 4,9 | 6,7 |

Spectre IR (Nujol)

C=O : 1698 $cm^{-1}$
aromatique : 1618, 1600, 1572, 1548, 1518, 1498 $cm^3$

**Exemple 24 : 4'-[(2-butyl 4-quinazolinyl) méthyl] (1,1'-biphényl)-2-carboxylate de méthyle.**

On opère comme à l'exemple 22 à partir de la 4-chloro 2-butyl quinazoline obtenue au stade C de la préparation indiquée ci-après et de l'organozincique préparé ainsi qu'il est indiqué à l'exemple 22.

Préparation de la 4-chloro 2-butyl quinazoline utilisée à l'exemple 24.

Stade A : N-(2(cyanophényl) valéramide.

On dissout 10 g d'orthocyanoaniline dans 50 $cm^3$ de pyridine anhydre puis ajoute 11,2 $cm^3$ de chlorure de valéroyle et porte au reflux pendant 2 heures. Le mélange réactionnel est versé sur 100 $cm^3$ d'eau glacée et on acidifie par HCl aqueux 2N jusqu'à pH = 7. On extrait à l'acétate d'éthyle, réunit les phases organiques et lave d'abord avec 100 $cm^3$ d'eau puis avec 100 $cm^3$ de solution saturée en chlorure de sodium.On déshydrate la phase organique sur sulfate de magnésium anhydre, filtre et évapore. Le solde obtenu est empâté dans de l'éther isopropylique. On filtre et évapore. On obtient 13,7 g du produit attendu. F = 76°C.
Spectre IR ($CHCl_3$)

-NH : 3416 $cm^{-1}$
-C≡N : 2222 $cm^{-1}$
aromatique + amide : 1605, 1583, 1520 $cm^3$

Stade B : 2-butyl 4-oxo quinazoline.

On dissout 13,7 g de produit obtenu au stade A dans 150 $cm^3$ de dioxanne. On ajoute 400 $cm^3$ de solution aqueuse de soude puis 18 $cm^3$ d'eau oxygénée à 30%. On porte au reflux pendant 2 heures, ajoute de l'acide acétique jusqu'à pH = 3 puis de l'ammoniaque à 28% jusqu'à pH = 8 dans un bain glacé. On filtre, lave à l'eau et sèche sous pression réduite à 50°C. On obtient 10,56 g du produit attendu. F = 156°C.
Spectre IR ($CHCl_3$)

= NH- : 3385 $cm^{-1}$
=O : 1674 $cm^{-1}$
aromatique : 1613, 1565 $cm^3$

Stade C : 2-butyl 4-chloro quinazoline.

On introduit sous agitation magnétique à 0°C 100 mg du produit obtenu au stade B dans 1 $cm^3$ d'oxychlorure de phosphore puis ajoute 85 microlitres de N,N-diisopropyléthylamine. On laisse revenir à la température ambiante, chauffe au reflux pendant 2 heures puis laisse revenir à la température ambiante. On évapore à sec, puis dissout dans un minimum de chlorure de méthylène, lave à l'eau et à l'eau saturée en chlorure de sodium. On extrait à l'acétate d'éthyle, sèche sur sulfate de magnésium, filtre et évapore à sec. Le résidu obtenu est purifié sur silice (éluant : chlorure de méthylène-acétate d'éthyle 95-5). On obtient 28 mg du produit attendu utilisé tel quel.

**Exemple 25 : Acide 4'-[(2-butyl 4-quinazolinyl) méthyl] (1,1'-biphényl)-2-carboxylique.**

On opère comme à l'exemple 23 à partir du produit obtenu à l'exemple 24.
En plus des produits décrits dans les exemples ci-dessus, qui illustrent l'invention sans toutefois la limiter, les produits de formule (I) tels que définis ci-dessous constituent des produits pouvant être obtenus dans le cadre de

la même invention comme exemples 26 à 29 :

**Exemple 26 : 4'-[[[3-butyl 2-(hydroxyméthyl) 4-quinoléinyl] oxy] méthyl] (1,1'-biphényl)-2-carboxylate de méthyle.**

**Exemple 27 : Acide 4'-[[[3-butyl 2-(hydroxyméthyl) 4-quinoléinyl] oxy] méthyl] (1,1'-biphényl) -2-carboxylique.**

**Exemple 28 : 4-[2-[(2-butyl 4-quinoléinyl) oxy] éthyl] benzoate de méthyle.**

**Exemple 29 : Acide 4-[2-[(2-butyl 4-quinoléinyl) oxy] éthyl] benzoique.**

Les exemples 26 à 29 ont été préparés ainsi qu'il est indiqué pour les exemples précédents.

Les acides carboxyliques 27 et 29 sont respectivement préparés à partir de leurs sels de méthyle 26 et 28 et sont caractérisés par leurs points de fusion soit respectivement : 174°C pour l'exemple 27 et 205°C pour l'exemple 29.

**Préparation pour l'exemple 30 du 3-butyl-1,4,5,6,7,8-hexahydro-4-oxo-1-quinoléine.**

On introduit 1 g du produit obtenu au stade a) dans 60 ml de méthanol. On ajoute environ 10 mg d'oxyde de platine et on met à hydrogéner sous pression d'environ 200 mBar.

Après environ 3 heures d'agitation à la température ambiante, on filtre la solution et évapore.

Après chromatographie (éluant chlorure de méthylène : méthanol/98 : 2). On obtient 0,6 g de produit attendu. F=220°C.
Spectre IR dans le chloroforme

| | |
|---|---|
| =C-N-H | 3430 $cm^{-1}$ |
| =O | 1632 $cm^{-1}$ |
| systèmes conjugués | : 1538 $cm^{-1}$, 1510 $cm^{-1}$ |

**Exemple 30 : B : 4'-[((3-butyl-1,4,5,6,7,8-hexahydro-4-quinoléinyl oxy] méthyl] (1,1'-byphényl)-2-carboxylate de méthyle ainsi que :**

**A : 4'[(3-butyl-1,4,5,6,7,8-hexahydro-4-oxo-1-quinoléinyl) méthyl]-(1,1'-biphényl)-2-carboxylate de méthyle.**

On introduit 0,4 g du produit obtenu pour la préparation de l'exemple 30 dans 10 ml d'acétone anhydre.

On ajoute 0,52 g de carbonate de potassium et 0,6 g de bromométhyl (1,1'-biphényl)-2-carboxylate de méthyle.

On chauffe une nuit à reflux puis verse le milieu réactionnel dans 100 ml d'eau et extrait la phase aqueuse avec 3 x 50 ml d'acétate d'éthyle.

On sèche et évapore à sec. Après chromatographie (éluant chlorure de méthylène : 98/méthanol : 2). On obtient 0,15 g du produit attendu B ainsi que 0,45 g du produit A
Spectre IR du produit A dans chloroforme

| | |
|---|---|
| =O | 1726 $cm^{-1}$ |
| C=C | 1637 $cm^{-1}$ |
| aromatique | 1595 $cm^{-1}$ |
| C=O | 1547 $cm^{-1}$ |
| | 1495 $cm^{-1}$ |

Spectre IR du produit B dans chloroforme

| | |
|---|---|
| =O | 1720 $cm^{-1}$ |
| aromatique | 1617 $cm^{-1}$, 1600 $cm^{-1}$, 1589 $cm^{-1}$, 1519 $cm^{-1}$, 1482 $cm^{-1}$ |

**Exemple 31 : Acide 4'-[((3-butyl-1,4,5,6,7,8-hexahydro-4-quinoléinyl) oxy) méthyl]-(1,1'-biphényl)-2-carboxylique ainsi que :**

**Acide 4'-[(3-butyl-1,4,5,6,7,8-hexahydro-4-oxo-1-quinoléinyl méthyl)-(1,1'-biphényl)-2-carboxylique. a) l'acide 4'-[(3-butyl-1,4,5,6,7,8-hexahydro-4-oxo-1-quinoléinyl méthyl)-(1,1'-biphényl)-2-carboxylique.**

On introduit 0,43 g du produit A obtenu à l'exemple 30 dans 10 ml d'une solution normale de soude.
On chauffe 1 heure à 60°C, agite environ 1 heure et ajoute 1 ml d'éthanol.
On agite une nuit à environ 40°C. On refroidit à la température ambiante et ajoute lentement de l'acide acétique glacial jusqu'à cristallisation de l'acide.
On agite environ 1 heure, essore, lave à l'eau puis à l'éther.
On recristallise dans 80 ml d'éthanol et obtient 0,310 g de produit attendu. F=260°C.
Spectre IR dans NUJOL

| | |
|---|---|
| =O | 1672 $cm^{-1}$ |
| Système conjugué | C=O 1630 $cm^{-1}$ |
| | C=O 1600 $cm^{-1}$ |
| aromatique | 1535 $cm^{-1}$ |
| | 1520 $cm^{-1}$ |

**b) Acide 4'[((3-butyl-1,4,5,6,7,8-hexahydro-4-quinoléinyl) oxy) méthyl]-(1,1'-biphényl)-2-carboxylique**

A partir du produit B obtenu avec l'exemple 30, et en procédant dans les mêmes conditions que ci-dessus en a) à partir du produit A de l'exemple 30, on obtient le produit attendu.

**Préparation de l'exemple 32.**

Stade A : 3-butyl 4-hydroxy 2-6 quinoléine dicarboxylate de 2-éthyl 6-méthyle.

**a) 2-butyl 3-[(4-(méthoxycarbonyl) phényl) amino] 2-butènedioate de diéthyle.**

On introduit 27,8 g de 4-aminobenzoate de méthyle et 47 g de 2-butyl 3-oxo butènedioate de diéthyle et ajoute 2 g de siliporite activée.
On agite environ 30 heures à 60°C. Après chromatographie (éluant hexane 95/Acétate d'éthyle : 5). On obtient 70 g de produit attendu.

**b) 3-butyl 4-hydroxy 2,6-quinoléine dicarboxylate de 2-éthyle 6-méthyle.**

Les 70 g de produit obtenu en a) ci-dessus sont mélangés à 70 ml de DOWTHERM.
On chauffe à environ 250°C environ 30 minutes, refroidit, empate dans l'éther et essore.
On obtient 45 g de produit attendu. F=160°C.
Spectre IR dans chloroforme

| | |
|---|---|
| =C-NH- | : 3422 $cm^{-1}$, 3380 $cm^{-1}$ |
| =O | : 1742 $cm^{-1}$, 1715 $cm^{-1}$, 1631 $cm^{-1}$ |
| aromatique | 1603 $cm^{-1}$ |
| C=C | 1577 $cm^{-1}$ |
| | 1525 $cm^{-1}$ |

Stade B : Acide 3-butyl 4-hydroxy 6-quinoléine carboxylique.

**a) Acide 3-butyl 1,4-dihydro 4-hydroxy 2-6 quinoléine dicarboxylique.**

On introduit 40 g du produit obtenu au stade F ci-dessus dans 150 ml de soude concentrée et ajoute 15 ml d'éthanol.
On chauffe environ 4 heures à environ 80°C, ajoute 100 ml d'un mélange de glace et eau, acidifie avec de l'acide chlorhydrique concentré, essore, lave à l'eau et sèche. On obtient 24 g de produit attendu. F 260°C.

**b) Acide 3-butyl 4-hydroxy 6-quinoléine carboxylique.**

On introduit 24 g du produit obtenu en a) ci-dessus dans 350 ml de DOWTHERM, chauffe à environ 250°C pendant 5 heures, refroidit à la température ambiante, empate dans l'éther essore, lave à l'eau et sèche.
On obtient 17,8 g de produit attendu. F 260°C.
Spectre IR dans NUJOL

| | |
|---|---|
| =O | 1702 $cm^{-1}$, 1682 $cm^{-1}$, 1640 $cm^{-1}$ |
| C=C | 1616 $cm^{-1}$ |
| aromatique | 1597 $cm^{-1}$, 1568 $cm^{-1}$, 1492 $cm^{-1}$ |

<u>Stade C :</u> 3-butyl 6-hydroxyméthyl 4-(1H)-quinoléinone.

On introduit 3 g du produit obtenu au stade B ci-dessus dans 800 ml de tétrahydrofuranne.
On ajoute 1,8 g de tétrahydrure de lithium et d'aluminium et agite environ 5 heures à la température ambiante.
On ajoute environ 5 ml d'une solution THF/eau 80/20, ajoute lentement une solution saturée de sel de tartrate double, filtre, lave le précipité avec du tétrahydrofuranne et sèche.
On obtient 2,5 g du produit attendu. F 260°C.
Spectre IR dans NUJOL

| | |
|---|---|
| C=O | 1638 $cm^{-1}$ |
| système conjugué | 1620 $cm^{-1}$ |
| aromatique | 1568 $cm^{-1}$ |
| | 1550 $cm^{-1}$ |
| | 1508 $cm^{-1}$ |
| | 1490 $cm^{-1}$ |

<u>Stade D :</u> 3-butyl-1,4,5,6,7,8 hexahydro-6-méthyl 4(1H) quinoléinone 1 (ainsi que 3-butyl-1,4,5,6,7,8 hexahydro 6-hydroxyméthyl 4(1H) quinoléinone 2 ).

On introduit 0,5 g du produit obtenu au stade C ci-dessus dans 100 ml de méthanol, ajoute 10 mg d'oxyde de platine et met à hydrogéner sous environ 300 mBar environ 24 heures.
On filtre, lave au méthanol et sèche. Après chromatographie (éluant chlorure de méthylène 95/méthanol 5).
On obtient 0,47 g de produit attendu 1 . F≈260°C.
Spectre IR dans NUJOL de 1

| | |
|---|---|
| =O système conjugué | 1632 $cm^{-1}$ |
| C=C | 1603 $cm^{-1}$ |
| C-N | 1500 $cm^{-1}$ |

**<u>Exemple 32 :</u> B : 4'- [((3-butyl-1,4,5,6,7,8-hexahydro-6-méthyl-4-quinoléinyl) oxy) méthyl] (1,1'-biphényl)-2-carboxylate de méthyle ainsi que :**

**A : 4'-[(3-butyl-1,4,5,6,7,8-hexahydro-6-méthyl-4-oxo-1-quinoléinyl) méthyl ]-(1,1'-biphényl)-2-carboxylate de méthyle.**

On opère comme à l'exemple 30 à partir de 0,45 g du produit 1 obtenu au stade D ci-dessus de la préparation de l'exemple 32 dans 20 ml d'acétone anhydre, ajoute 0,55 g de carbonate de potassium et 0,61 g de bromométhyl (1,1'-biphényl)-2-carboxylate de méthyle.
On chauffe environ 3 heures à reflux, verse le milieu réactionnel dans 100 ml d'eau, extrait la phase aqueuse par 3 x 50 ml d'acétate d'éthyle, sèche la phase organique et évapore.
Après chromatographie (éluant chlorure de méthylène 95/méthanol : 5), on obtient 0,1 g de produit B ainsi que 0,58 g de produit A
Spectre IR dans le chloroforme du produit A

| | |
|---|---|
| -C-OMe | 1725 $cm^{-1}$ |
| O | 1434 $cm^{-1}$ |
| C=O | 1638 $cm^{-1}$ |

| | |
|---|---|
| C=O | 1600 $cm^{-1}$ |
| aromatiques | 1545 $cm^{-1}$, 1594 $cm^{-1}$ |

**<u>Exemple 33 :</u> Acide 4'[((3-butyl-1,4,5,6,7,8-hexahydro-6-méthyl-4-quinoléinyl) oxy) méthyl] (1,1'-biphényl)-2-carboxylique ainsi que :**

l'**acide 4'-((3-butyl-1,4,5,6,7,8-hexahydro-6-méthyl-4-oxo-1-quinoléinyl) méthyl)-(1,1'-biphényl)-2-carboxylique.**

**a) Acide 4'-[(3-butyl-1,4,5,6,7,8-hexahydro-6-méthyl-4-oxo-1-quinoléinyl) méthyl) - (1,1'-biphényl) -2-carboxylique.**

On opère comme à l'exemple 31 à partir 0,4 g du produit A obtenu à l'exemple 32 dans 10 ml de soude 2N.
On agite une nuit à environ 60°C, refroidit à la température ambiante, acidifie avec de l'acide acétique glacial, décante, empate dans une solution d'acide chlorhydrique 2N, essore, sèche, recristallise dans 50 ml d'un mélange éthanol 49/eau 1 et obtient 0,220 g de produit attendu. F=255°C.
Spectre IR dans NUJOL

| | |
|---|---|
| =O | 1675 $cm^{-1}$, 1628 $cm^{-1}$ |
| C=C | 1600 $mc^{-1}$ |
| aromatique | 1533 $cm^{-1}$ |
| | 1517 $cm^{-1}$ |

**b) Acide 4'[((3-butyl-1,4,5,6,7,8-hexahydro-6-méthyl-4-quinoléinyl) oxy) méthyl] (1,1'-biphényl) -2-carboxylique.**

A partir du produit B obtenu avec l'exemple 32, et en procédant dans les <u>mêmes conditions</u> que ci-dessus en a) à partir du produit A de l'exemple 32 on peut obtenir le produit attendu.
A partir du produit 2 également obtenu au stade D de la préparation de l'exemple 32 et en procédant de la même manière que pour obtenir l'exemple 32 à partir du produit 1 obtenu à ce stade D sus-dit, on peut obtenir les produits :

> **$B_1$ : 4' [((3-butyl-1,4,5,6,7,8-hexahydro-6-hydroxyméthyl-4-quinoléinyl) oxy) méthyl] (1,1'-biphényl)-2-carboxylate de méthyle ainsi que :**
> **$A_1$ : 4'-[(3-butyl-1,4,5,6,7,8-hexahydro-6-hydroxyméthyl-4-oxo-1-quinoléinyl) méthyl)-(1,1'-biphényl)-2-carboxylate de méthyle.**

A partir des produits $A_1$ et $B_1$ définis ci-dessus, et en procédant de la même manière que pour obtenir l'exemple 33.
On peut obtenir respectivement à partir de $A_1$ l'acide 4'-((3-butyl-1,4,5,6,7,8-hexahydro-6-hydroxyméthyl-4-oxo-1-quinoléinyl) méthyl)-1,1'-biphényl) -2-carboxylique et à partir de $B_1$ l'acide 4'[((4-butyl-1,4,5,6,7,8-hexahydro-6-hydroxyméthyl-4-quinoléinyl) oxy) méthyl] (1,1'-biphényl)-2-carboxylique.

**<u>Exemple 34 :</u> carboxylate de méthyle de l'acide carboxylique de l'exemple 35.**

**<u>Exemple 35 :</u> Acide 4' - ( ( (2- (2-méthylpropyl)-8-(trifluorométhyl)-4-quinoléinyl) oxy) méthyl) (1,1'-biphényl)-2-carboxylique.**

Caractérisé par son point de fusion de 164°C le produit de l'exemple 34 a été préparé ainsi qu'il est indiqué ci-dessus pour les exemples précédents à partir de son sel de carboxylate de méthyle (position 2' du radical biphényl) qui constitue l'exemple 34 lui-même préparé ainsi qu'il est indiqué ci-dessus pour les exemples précédents.
Les produits ci-dessous peuvent également être obtenus dans des conditions décrites ci-dessus et représente les exemples 36 et 37 :

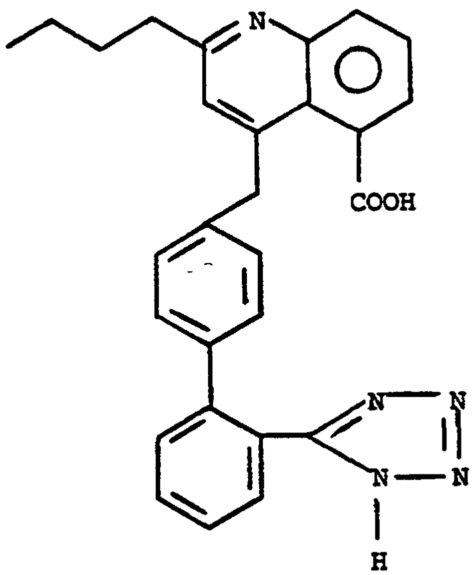

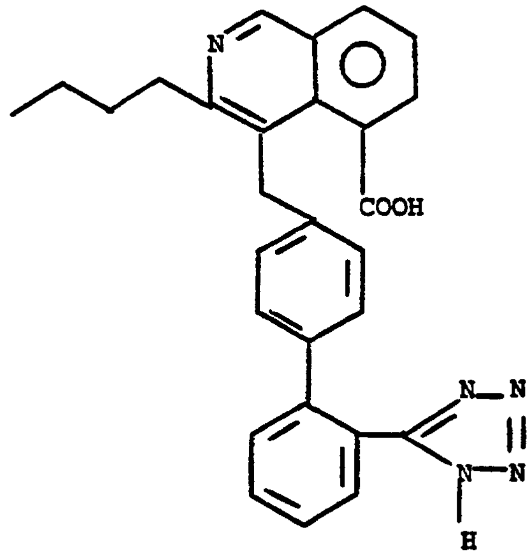

**EXEMPLE 38 : composition pharmaceutique**

On a préparé des comprimés répondant à la formule suivante :

- Produit de l'exemple 4 10 mg
- Excipient pour un comprimé terminé à 100 mg

(détail de l'excipient : lactose, talc, amidon, stéarate de magnésium)

**RESULTATS PHARMACOLOGIOUES**

1) Test sur le récepteur de l'angiotensine II

On utilise une préparation membranaire fraîche obtenue à partir de foie de rat. Le tissu est broyé au polytron dans un tampon Tris 50 mM pH 7,4 le broyage est suivi de 3 centrifugations à 30 000 g 15 minutes avec reprises intermédiaires des culots dans le tampon Tris pH 7,4.

Les derniers culots sont remis en suspension dans un tampon d'incubation pH 7,4 (Tris 20 mM, NaCl 135 mM, KCl 10 mM, glucose 5 mM, $MgCl_2$ 10 mM fluorure de phénylméthyl sulfonyle 0,3 mM, bacitracine 0,1 mM, (sérum albumine bovine 0,2 %).

On répartit des fractions aliquotes de 2 ml dans des tubes à hémolyse et ajoute de la $^{125}I$ angiotensine II (25 000 DPM/tube) et le produit à étudier. (Le produit est d'abord testé à 3 x $10^{-5}$M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition du produit de l'exemple 94 du brevet européen 0253310 à $10^{-5}$M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % ($CI_{50}$), c'est-à-dire en concentration de produit étudié, exprimée en nM, nécessaire pour déplacer 50 % de la radioactivité spécifique fixée sur le récepteur étudié.

Résultat :

| Produit de l'exemple | $CI_{50}$ en nanomoles |
|---|---|
| 4 | 540 |

2 - Mise en évidence de l'activité antagoniste de l'angioten sine II sur la veine porte isolée

La veine porte est prélevée, sur des rats mâles Wistar (350 g environ) (IFFA Crédo France) après dislocation cervicale. Elle est mise rapidement dans une solution physiologique (voir ci-dessous) à température ambiante. Un anneau de 1 mm environ est monté dans un bain à organe isolé contenant 20 ml de la solution physiologique suivante (composition en mM : NaCl 118,3 - KCl 4,7 - $MgSO_4$ 1,2 - $KH_2PO_4$ 1,2 - $NaHCO_3$ 25 - glucose 11,1 - CaCl2 2,5) le milieu est maintenu à 37°C et oxygéné par un mélange $O_2$ 95 %, $CO_2$ 5 %. La tension initiale imposée est de 1 g, les anneaux sont laissés au repos pendant 60 à 90 minutes. Afin d'éviter les contractions spontanées, du vérapamil est ajouté au bain d'incubation ($1.10^{-6}$M).

A la fin de la période de repos l'angiotensine II (hypertensine Ciba) $3.10^{-8}$M est ajoutée dans le bain d'incubation et laissée au contact de la préparation pendant 1 minute. Cette opération est répétée chaque 30 minutes, le tissu étant lavé 3 ou 4 fois entre deux stimulations à l'angiotensine. Le composé à étudier est introduit dans le bain 15 minutes avant une nouvelle stimulation par l'angiotensine. Des concentrations croissantes de la molécule étant appliquées, une $CI_{50}$ (concentration qui produit une inhibition de 50 % de la réponse à l'angiotensine) peut être calculée, celle-ci est exprimée en nanomoles.

Résultats :

| Produit de l'exemple | $CI_{50}$ en nanomoles |
|---|---|
| 4 | 131 |

3 - Etude de l'activité sur récepteur de l'endothéline

On effectue une préparation membranaire à partir de cortex postérieur plus cervelet de rat. Le tissu est broyé au POLYTRON dans un tampon Tris 50 mM pH = 7,4. Aprés 30 minutes à 25 °C (B.M.) l'homogénat est centrifugé à 30000 g pendant 15 minutes (2 centrifugations avec reprise intermediaire dans le tampon Tris pH. 7,4).

Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4). On répartit des aliquotes de 2 ml dans des tubes à hémolyse et ajoute de la 1251 Endothéline (environ 50000 dpm/tube) et le produit à étudier. (Le produit est d'abord testé à $3*10^{-5}$ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %. La liaison non spécifique est déterminée par addition d'endothéline à $10^{-6}$ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton. Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

Résultat :

La CI50 trouvée pour le produit de l'exemple 35 est de 14.000 nanomoles.

4 - Recherche d'une activité antagoniste de l'effet vasoconstricteur de l'endothéline chez le rat démédullé

La vasoconstriction provoquée par des injections (IV) en doses cumulées d'endothéline (1 - 3 - 10 - 30 ug/kg) est mesurée par l'augmentation de pression artérielle moyenne chez un groupe de rats témoins (Sprague Dawley), anesthésiés puis démédullés.

Les produits à tester sont injectés 10 minutes avant la gamme de concentrations d'endothéline. La diminution de réponse à l'endothéline indique un effet antagoniste.

Le produit de l'exemple 35 montre un effet antagoniste à partir de 1 mg/kg (IV).

## Revendications

**Revendications pour les Etats contractants suivants : AT, BE, CH/LI, DE, DK, FR, GB, IT, LU, MC, NL, SE**

**1.** Produits de formule (I) :

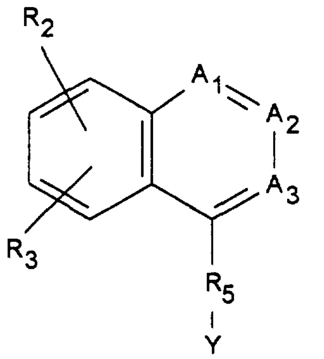

(I)

dans laquelle :

$R_2$ et $R_3$ représentent un atome d'hydrogène ou un radical alkylthio renfermant au plus 4 atomes de carbone, $A_1$, $A_2$ et $A_3$ sont tels que l'un ou deux d'entre eux représentent un atome d'azote, et les autres, identiques ou différents, représente le radical

$$=\overset{|}{C}-R_4$$

tel que $R_4$ est choisi parmi l'atome d'hydrogène, le radical n-butyle et alkylthio renfermant au plus 4 atomes de carbone,
$R_5$ représente le radical $-CH_2-$, -NH-, -O- et $-OCH_2-$
et Y représente phényle substitué par un radical tétrazole ou biphényle éventuellement substitué par un ou plusieurs radicaux choisis parmi les radicaux cyano, carboxy libre, salifié et estérifié et tétrazolyle, à l'exception des produits dans lesquels :

a) $A_1$ représente un atome d'azote, $A_2$ et $A_3$, identiques ou différents, représentent le radical

$$=\overset{|}{C}-R_4$$

dans lequel $R_4$ représente l'atome d'hydrogène ou le radical n-butyle,$R_5$ représente $-O-CH_2$,
b) $R_2$ et $R_3$ représentent hydrogène,

$A_1$ représente un atome d'azote,
$A_2$ et $A_3$ sont tels que ou bien l'un représente un atome d'azote et l'autre représente

|
=C-CH

ou bien les deux représentent

|
=C-CH,

$R_5$ représente 0, NH ou $CH_2$
et Y représente biphényle éventuellement substitué par CN, étant entendu que les 5 produits suivants :

- Chlorhydrate d'acide 4-[[(2-butyl 4-quinoléinyl) oxy] méthyl] benzoïque ;
- 4'-[[(3-butyl 5-méthylthio 4-quinoléinyl) oxy] méthyl] (1,1'-biphényl) -2-carboxylate de méthyle ;
- Acide 4'-[[(3-butyl 4-quinoléinyl) oxy] méthyl] (1,1'-biphényl) -2-carboxylique;
- Sel de diéthylamine de l'acide 4'-[[(3-butyl 1,4-dihydro 4-(méthylthio) 4-quinoléinyl) oxy] méthyl] (1,1'-biphényl) 2-carboxylique ;
- Acide 4'-[(2-phényl 4-quinazolinyl) méthyl] (1,1'-biphényl)-2-carboxylique ;

appartiennent à la présente invention,
lesdits produits de formule (I) et ces 5 produits étant sous toutes les formes isomères possibles racémiques, énantiomères et diastéréoisomères, ainsi que les sels d'addition avec les acides minéraux et organiques ou avec les bases minérales et organiques desdits produits de formule (I) et de ces 5 produits.

**2.** les produits suivants

- Sel de diéthylamine de l'acide 4'-[[(3-butyl 1,4-dihydro 4-(méthylthio) 4-quinoléinyl) oxy] méthyl] (1,1'-biphényl) 2-carboxylique ;
- Acide 4'-[[(3-butyl 4-quinoléinyl) oxy] méthyl] (1,1'-biphényl) -2-carboxylique

ces produits étant sous toutes les formes isomères possibles racémiques, énantiomères et diastéréoisomères, ainsi que les sels d'addition avec les acides minéraux et organiques ou avec les bases minérales et organiques de ces produits.

**3.** L'acide 4'-[(2-butyl 4-quinoleinyl) méthyl] (1,1'-biphényl)-2-carboxylique.

**4.** Procédé de préparation de produits de formule (I) et des produits tels que définis aux revendications 1 à 3, **caractérisé en ce que** :

a) soit l'on soumet le composé de formule (III):

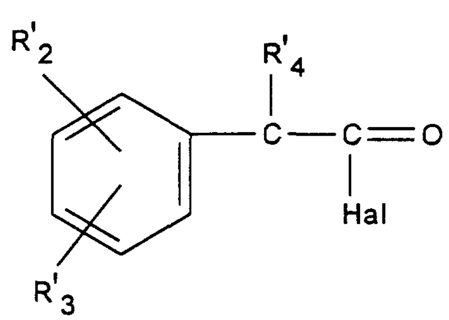

(III)

dans laquelle $R'_2$, $R'_3$ et $R'_4$ ont les significations indiquées à la revendication 1 respectivement pour $R_2$, $R_3$ et

$R_4$ dans lesquelles les éventuelles fonctions réactives sont éventuellement protégées par des groupements protecteurs, $R'_4$ pouvant également représenter un radical phényl,
et Hal représente un atome d'halogène, à une réaction de substitution pour obtenir le produit de formule (IV) :

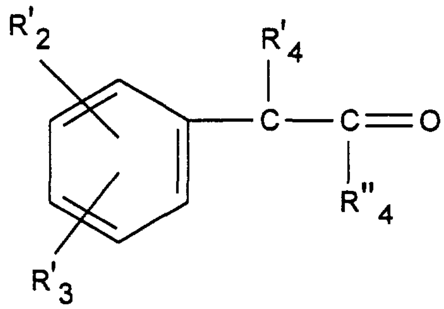

(IV)

dans laquelle $R'_2$, $R'_3$ et $R'_4$ ont les significations indiquées ci-dessus et $R''_4$, identique ou différent de $R'_4$, a la signification indiquée à la revendication 1 pour $R_4$ dans laquelle les éventuelles fonctions réactives sont éventuellement protégées par des groupements protecteurs, que l'on fait réagir :

avec un composé de formule (V) :

$$R'''_4\text{-}C{\equiv}N \qquad (V)$$

dans laquelle $R'''_4$, identique ou différent de $R'_4$ ou $R''_4$, a la signification indiquée à la revendication 1 pour $R_4$ dans laquelle les éventuelles fonctions réactives sont éventuellement protégées par des groupements protecteurs, pour obtenir après cyclisation un produit de formule (Ii) :

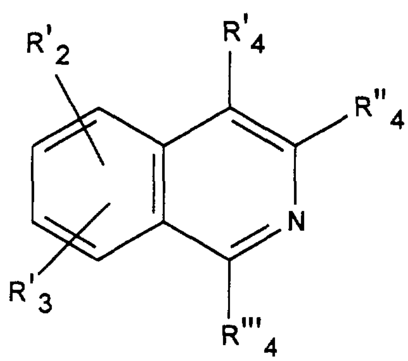

(Ii)

dans laquelle $R'_2$, $R'_3$, $R'_4$, $R'_4$ et $R'''_4$ ont les significations indiquées ci-dessus,

b) soit l'on fait réagir un produit de formule (VI):

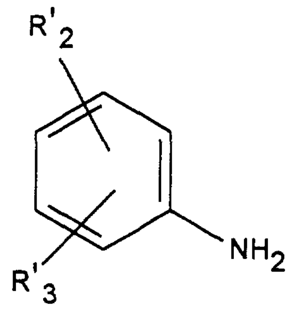

(VI)

dans laquelle $R'_2$ et $R'_3$ ont les significations indiquées ci-dessus avec un produit de formule (VII) :

$$R'_4\text{-CH}(CO_2alk)\text{-CO-}R''_4 \qquad (VII)$$

dans laquelle $R'_4$ et $R''_4$, identiques ou différents, ont les significations indiquées ci-dessus et alk représente un radical alkyle renfermant au plus 6 atomes de carbone, pour obtenir des produits de formule (VIII) :

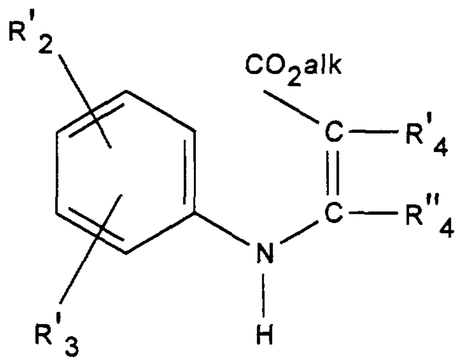

(VIII)

dans laquelle $R'_2$, $R'_3$, $R'_4$, $R''_4$ et alk ont les significations indiquées ci-dessus, que l'on cyclise pour obtenir des produits de formule (IIb) :

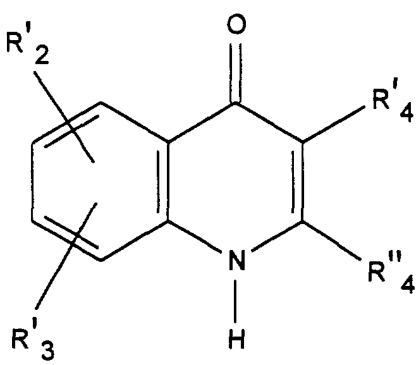

(IIb)

dans laquelle $R'_2$, $R'_3$, $R'_4$ et $R''_4$ ont les significations indiquées ci-dessus,
c) soit l'on fait réagir un produit de formule (IX) :

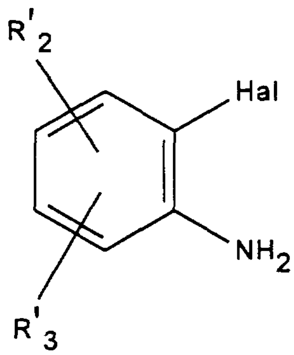

(IX)

dans laquelle $R'_2$ et $R'_3$ ont les significations indiquées ci-dessus avec un produit de formule (X) :

$$R''\,'_4\text{-C}\equiv\text{CH} \quad (X)$$

dans laquelle $R'''_4$ a la signification indiquée ci-dessus pour obtenir des produits de formule (XI) :

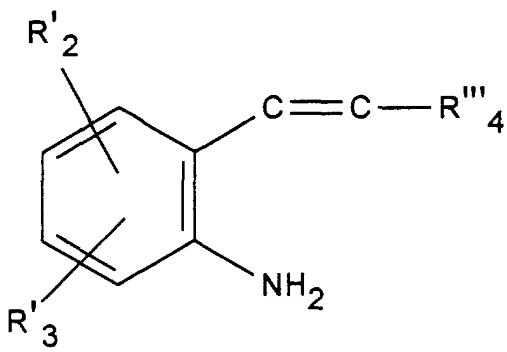

(XI)

dans laquelle $R'_2$, $R'_3$ et $R''\,'_4$ ont les significations indiquées ci-dessus, que l'on soumet à une réaction de cyclisation en présence d'un donneur d'azote tel que le nitrite de sodium, pour obtenir un produit de formule (IIC) :

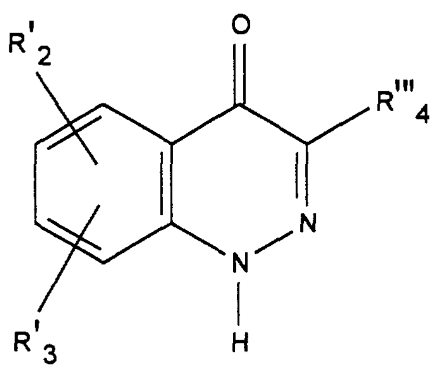

(IIc)

dans laquelle $R'_2$, $R'_3$ et $R''\,'_4$ ont les significations indiquées ci-dessus,
d) soit l'on soumet un produit de formule (XIV) :

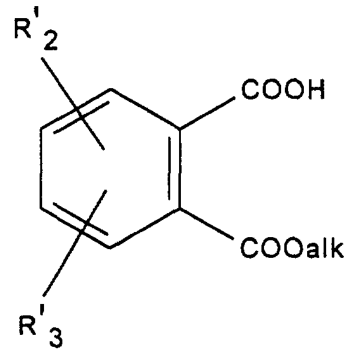

(XIV)

dans laquelle R'$_2$ et R'$_3$ et alk ont les significations indiquées ci-dessus, aprés, si désiré, une réaction d'halogénation de la fonction carboxy libre, à une réaction d'addition sur cette fonction carboxy d'un composé de formule R'$_4$-H, R'$_4$ ayant la signification indiquée ci-dessus, pour obtenir après cyclisation en présence d'hydrazine ou d'un dérivé des produits de formule (IIe) :

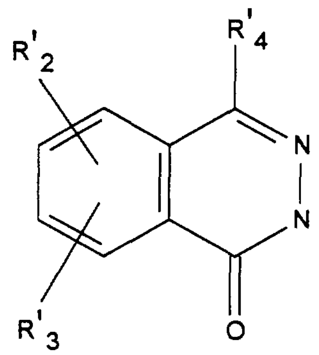

(IIe)

dans laquelle R'$_2$, R'$_3$ et R'$_4$ ont les significations indiquées ci-dessus,
e) soit l'on fait réagir un produit de formule (XV) :

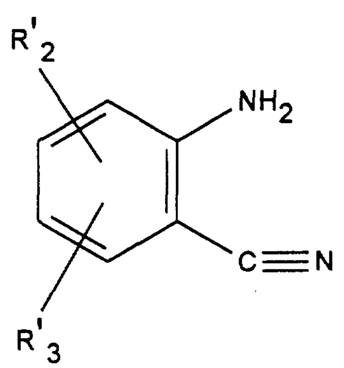

(XV)

dans laquelle R'$_2$ et R'$_3$ ont les significations indiquées ci-dessus avec un produit de formule (XVI) :

$$R'_4\text{-CO-Cl} \qquad (XVI)$$

dans laquelle R'$_4$ a la signification indiquée ci-dessus, R'$_4$ pouvant également représenter un radical phényl pour donner le produit de formule (XVII) :

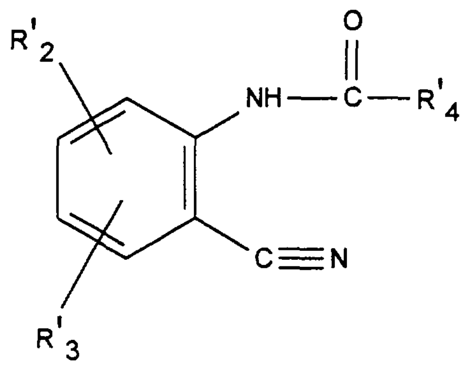

(XVII)

dans laquelle $R'_2$, $R'_3$ et $R'_4$ ont les significations indiquées précédemment, pour obtenir, aprés cyclisation, des produits de formule (IIf) :

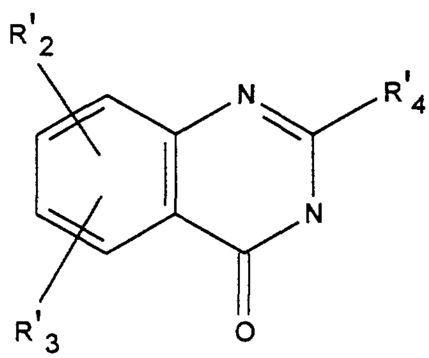

(IIf)

dans laquelle $R'_2$, $R'_3$ et $R'_4$ ont les significations indiquées ci-dessus, produits de formule (Ii) qui peuvent représenter des produits de formule (I) et produits de formules (IIb), (IIC), (IIe) et (IIf) telles que définies ci-dessus qui peuvent représenter des produits de formule (I) dans laquelle l'un au moins de $A_1$, $A_2$ et $A_3$ représente un radical méthine portant un radical hydroxyle, que l'on soumet, si désiré et si nécessaire, à l'une ou plusieurs des réactions suivantes, dans un ordre quelconque :

- une réaction de réduction complète du radical hydroxyle ou oxo en radical méthine suivie d'une aromatisation,
- sur les produits de formules (IIb), (IIc), (IIe) et (IIf) que l'on soumet

soit d'abord à une réaction de substitution du radical hydroxyle par un atome d'halogène suivie de l'action d'un produit de formule $R_5$-Y-M-Hal dans lequel $R_5$-Y- a la signification indiquée à la revendication 1 pour $R_4$ dans lesquelles les éventuelles fonctions réactives sont éventuellement protégées par des groupements protecteurs, M représente un atome de métal choisi parmi le magnésium, le cuivre et le zinc et Hal représente un atome d'halogène, soit à l'action d'un produit de formule $R_5$Y - Hal dans lequel Hal représente un atome d'halogène, pour obtenir les produits de formule (I) correspondants,

- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification d'une fonction acide,
- une réaction de saponification d'une fonction ester en fonction acide,
- une réaction de transformation de la fonction cyano en fonction acide,
- une réaction de dédoublement des formes racémiques, lesdits produits ainsi obtenus étant sous toutes les formes isomères possibles racémiques, énantiomères et diastéréoisomères.

5. A titre de médicaments, les produits de formule (I) et les produits tels que définis aux revendications 1 à 3, ces produits étant sous toutes les formes isomères possibles racémiques, énantiomères et diastéréoisomères, ainsi que les sels d'addition avec les acides minéraux et organiques ou avec les bases minérales et organiques pharmaceutiquement acceptables de ces produits.

6. Les compositions pharmaceutiques contenant à titre de principe actif, l'un au moins des médicaments tels que définis à la revendication 4.

7. Utilisation des produits de formule ($I_a$) :

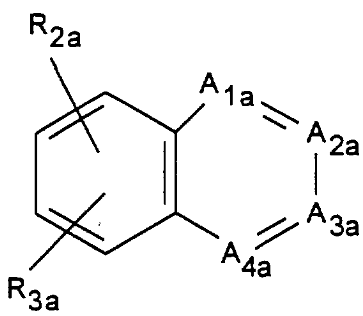

(Ia)

dans laquelle :

$R_{2a}$ et $R_{3a}$, identiques ou différents, sont choisis dans le groupe formé par :

- l'atome d'hydrogène,
- les atomes d'halogène,
- le radical hydroxyle,
- le radical mercapto, cyano, nitro, formyle, benzoyle, acyle ayant au plus 6 atomes de carbone,
- les radicaux carboxy libre, salifié ou estérifié par un radical alkyle linéaire ou ramifié renfermant au plus 4 atomes de carbone,
- les radicaux alkyle, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole et isoxazole,
- les radicaux amino, mono- ou dialkylamino, carbamoyle, pyrrolyle, morpholino, pipérazinyle, pyrrolylméthyle, morpholinométhyle, pipérazinyleméthyle, pyrrolylcarbonyle, morpholinocarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,

$A_{1a}$, $A_{2a}$, $A_{3a}$ et $A_{4a}$, identiques ou différents, représentent un atome d'azote et le radical

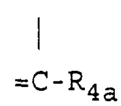

tel que :
soit $R_{4a}$ représente le radical $R_{1a}$ tel que $R_{1a}$ représente :

- l'atome d'hydrogène, le radical hydroxyle, cyano, carboxy libre, salifié ou estérifié par un radical alkyle

renfermant au plus 4 atomes de carbone,

- le radical alkyle, alkényle, alkyloxy, acyle ou alkylthio, ces radicaux étant linéaires ou ramifiés et renfermant au plus 7 atomes de carbone, le radical phényle, benzyle, phénoxy, phénylthio, tous ces radicaux aliphatiques ou cycliques étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, tétrazole et isoxazole,

soit $R_{4a}$ représente le radical - $R_{5a}$ - Ya dans lequel :

- $R_{5a}$ représente un radical $-CH_2-$, -NH-, -O-, $-OCH_2-$ ou $-SCH_2-$

et - Ya représente un radical phényle substitué par un radical tétrazole ou isoxazole ou biphényle éventuellement substitué par un ou plusieurs radicaux choisis parmi les radicaux hydroxyle, halogène, alkyle et alkyloxy renfermant au plus 4 atomes de carbone, trifluorométhyle, cyano, nitro, carboxy libre, salifié ou estérifié, tétrazole et isoxazole, étant entendu que les produits de formule (Ia) sont tels que l'un au moins et deux au plus de $A_{1a}$, $A_{2a}$, $A_{3a}$ et $A_{4a}$ représentent un atome d'azote et l'un au moins représente le radical méthine substitué par le radical - $R_{5a}$ - $Y_a$, à l'exception des produits dans lesquels :

$A_{1a}$ représente un atome d'azote,

$A_{2a}$ représente le radical

$$=\overset{|}{C}-R_{4a}$$

dans lequel $R_{4a}$ représente un atome d'hydrogène, un radical alkyle éventuellement substitué par un ou plusieurs atomes de fluor, les radicaux hydroxyle, phényle ou alcoxy (1-4C) ou un radical phényle,

$A_{3a}$ représente le radical

$$=\overset{|}{C}-R_{4a}$$

dans lequel $R_{4a}$ représente un atome d'hydrogène, un radical alkyle, carboxy, libre estérifié ou salifié, cyano, nitro, phényle ou phénylalkyle,

$A_{4a}$ représente le radical

$$=\overset{|}{C}-R_{4a}$$

dans lequel $R_{4a}$ représente le radical $-R_{5a}-Y_a$ dans lequel $R_{5a}$ représente le radical $-O-CH_2-$, $Y_a$ représente le radical phényle éventuellement substitué par tétrazole ou phényle, lui-même éventuellement substitué par un substituant choisi parmi les radicaux tétrazolyle, carboxy éventuellement salifié ou estérifié, alkyle, alkoxy, halogène, trifluorométhyle, cyano et nitro,

$R_{2a}$ et $R_{3a}$ sont choisis parmi l'atome d'hydrogène ; les atomes d'halogène ; le radical hydroxyle ; trifluorométhyl ; cyano ; nitro ; alkyle renfermant au plus 4 atomes de carbone éventuellement substitué par

hydroxyle ou alcoxy renfermant au plus 4 atomes de carbone ; alcoxy renfermant au plus 4 atomes de carbone éventuellement substitué par un atome de fluor ; alkylthio renfermant au plus 6 atomes de carbone ; carbamoyle ; amino éventuellement substitué par un ou deux radicaux alkyle pour renfermer au plus 6 atomes de carbone ; carboxy ; alcoxycarbonyl renfermant au plus 4 atomes de carbone ; acyl renfermant au plus 4 atomes de carbone ; et utilisation des 4 produits suivants :

- Chlorhydrate d'acide 4-[[(2-butyl 4-quinoléinyl) oxy] méthyl] benzoïque ;
- 4'-[[(3-butyl 5-méthylthio 4-quinoléinyl) oxy] méthyl] (1,1'-biphényl) -2-carboxylate de méthyle ;
- Sel de diéthylamine de l'acide 4'-[[(3-butyl 1,4-dihydro 4-(méthylthio) 4-quinoléinyl) oxy] méthyl] (1,1'-biphényl) 2-carboxylique ;
- Acide 4'-[[(3-butyl 4-quinoléinyl) oxy] méthyl] (1,1'-biphényl)-2-carboxylique,

lesdits produits de formule ($I_a$) et les 4 produits indiqués ci-dessus étant sous toutes les formes isomères possibles racémiques, énantiomères et diastéréoisomères, ainsi que les sels d'addition avec les acides minéraux et organiques ou avec les bases minérales et organiques desdits produits de formule ($I_a$) et des 4 produits indiqués ci-dessus pour la préparation de médicaments destinés,
soit au traitement de l'hypertension artérielle, des insuffisances cardiaques, des insuffisances rénales et dans la prévention des resténoses post-angioplastie,
soit au traitement de certains désordres gastro-intestinaux ou gynécologiques.

**Revendications pour les Etats contractants suivants : ES, GR, PT**

**1.** Procédé de préparation des produits de formule (I) :

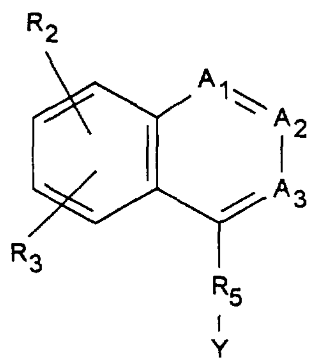

(I)

dans laquelle :

$R_2$ et $R_3$ représentent un atome d'hydrogène ou un radical alkylthio renfermant au plus 4 atomes de carbone, $A_1$, $A_2$ et $A_3$ sont tels que l'un ou deux d'entre eux représentent un atome d'azote, et les autres, identiques ou différents, représente le radical

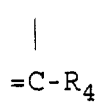

tel que $R_4$ est choisi parmi l'atome d'hydrogène, le radical n-butyle et alkylthio renfermant au plus 4 atomes de carbone,
$R_5$ représente le radical $-CH_2-$, -NH-, -O- et $-OCH_2-$et Y représente phényle substitué par un radical tétrazole ou biphényle éventuellement substitué par un ou plusieurs radicaux choisis parmi les radicaux cyano, carboxy libre, salifié et estérifié et tétrazolyle, à l'exception des produits dans lesquels :

a) $A_1$ représente un atome d'azote, $A_2$ et $A_3$, identiques ou différents, représentent le radical

|
$=C-R_4$

dans lequel $R_4$ représente l'atome d'hydrogène ou le radical n-butyle,$R_5$ représente -O-$CH_2$
b) $R_2$ et $R_3$ représentent hydrogène,

$A_1$ représente un atome d'azote,
$A_2$ et $A_3$ sont tels que ou bien l'un représente un atome d'azote et l'autre représente

|
$=C-CH$

ou bien les deux représentent

|
$=C-CH,$

$R_5$ représente O, NH ou $CH_2$
et Y représente biphényle éventuellement substitué par CN, et des 5 produits suivants :

- Chlorhydrate d'acide 4-[[(2-butyl 4-quinoléinyl) oxy] méthyl] benzoïque ;
- 4'-[[(3-butyl 5-méthylthio 4-quinoléinyl) oxy] méthyl] (1,1'-biphényl) -2-carboxylate de méthyle ;
- Sel de diéthylamine de l'acide 4'-[[(3-butyl 1,4-dihydro 4-(méthylthio) 4-quinoléinyl) oxy] méthyl] (1,1'-biphényl) 2-carboxylique ;
- Acide 4'-[[(3-butyl 4-quinoléinyl) oxy] méthyl] (1,1'-biphényl) -2-carboxylique ;
- Acide 4'-[(2-phényl 4-quinazolinyl) méthyl] (1,1'-biphényl)-2-carboxylique,

lesdits produits de formule (I) et ces 5 produits étant sous toutes les formes isomères possibles racémiques, énantiomères et diastéréoisomères, ainsi que les sels d'addition avec les acides minéraux et organiques ou avec les bases minérales et organiques desdits produits de formule (I)et de ces 5 produits,
**caractérisé en ce que** :

a) soit l'on soumet le composé de formule (III) :

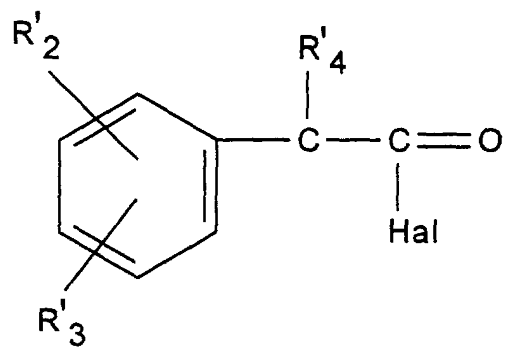

(III)

dans laquelle $R'_2$, $R'_3$ et $R'_4$ ont les significations indiquées à la revendication 1 respectivement pour $R_2$, $R_3$ et $R_4$ dans lesquelles les éventuelles fonctions réactives sont éventuellement protégées par des groupements protecteurs, $R'_4$ pouvant également représenter un radical phényl,
et Hal représente un atome d'halogène, à une réaction de substitution pour obtenir le produit de formule (IV) :

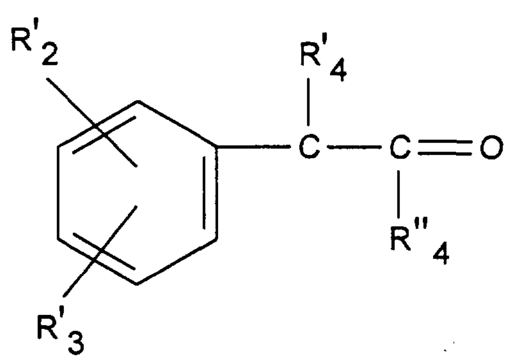

(IV)

dans laquelle $R'_2$, $R'_3$ et $R'_4$ ont les significations indiquées ci-dessus et $R''_4$, identique ou différent de $R'_4$, a la signification indiquée à la revendication 1 pour $R_4$ dans laquelle les éventuelles fonctions réactives sont éventuellement protégées par des groupements protecteurs, que l'on fait réagir :
avec un composé de formule (V) :

$$R'''_4\text{-}C{\equiv}N \qquad (V)$$

dans laquelle $R'''_4$, identique ou différent de $R'_4$ ou $R''_4$, a la signification indiquée à la revendication 1 pour $R_4$ dans laquelle les éventuelles fonctions réactives sont éventuellement protégées par des groupements protecteurs, pour obtenir après cyclisation un produit de formule (Ii) :

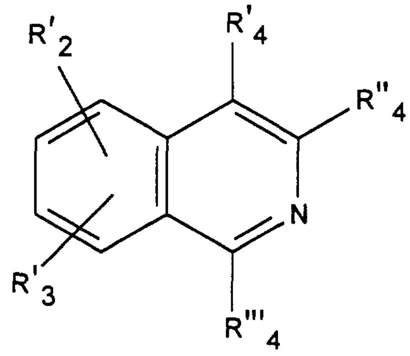

(Ii)

dans laquelle $R'_2$, $R'_3$, $R'_4$, $R'_4$ et $R''{}'_4$ ont les significations indiquées ci-dessus,

b) soit l'on fait réagir un produit de formule (VI) :

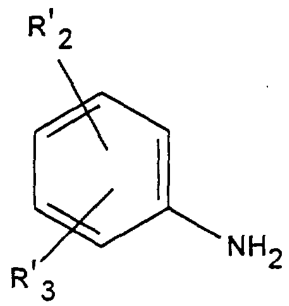

(VI)

dans laquelle $R'_2$ et $R'_3$ ont les significations indiquées ci-dessus avec un produit de formule (VII) :

$$R'_4\text{-CH}(CO_2\text{alk})\text{-CO-}R''_4 \quad \text{(VII)}$$

dans laquelle $R'_4$ et $R''_4$, identiques ou différents, ont les significations indiquées ci-dessus et alk représente un radical alkyle renfermant au plus 6 atomes de carbone, pour obtenir des produits de formule (VIII) :

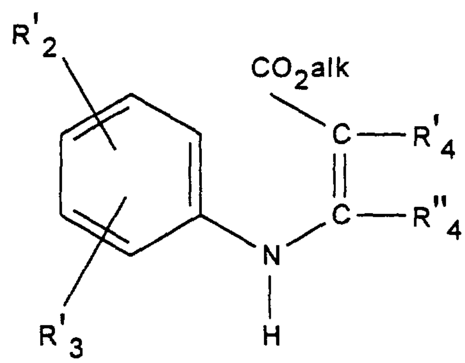

(VIII)

dans laquelle $R'_2$, $R'_3$, $R'_4$, $R''_4$ et alk ont les significations indiquées ci-dessus, que l'on cyclise pour obtenir des produits de formule (IIb) :

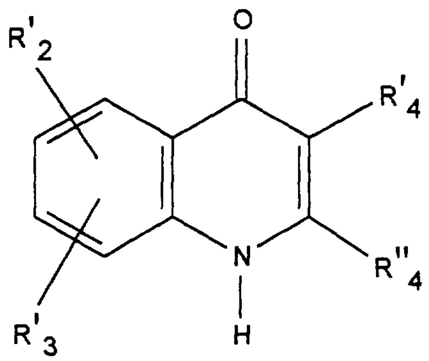

(IIb)

dans laquelle $R'_2$, $R'_3$, $R'_4$ et $R''_4$ ont les significations indiquées ci-dessus,
c) soit l'on fait réagir un produit de formule (IX) :

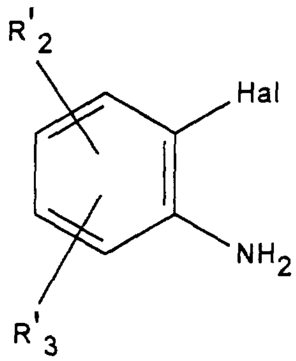

(IX)

dans laquelle $R'_2$ et $R'_3$ ont les significations indiquées ci-dessus avec un produit de formule (X) :

$$R''\,'_4\text{-}C\equiv CH \qquad (X)$$

dans laquelle $R''\,'_4$ a la signification indiquée ci-dessus pour obtenir des produits de formule (XI) :

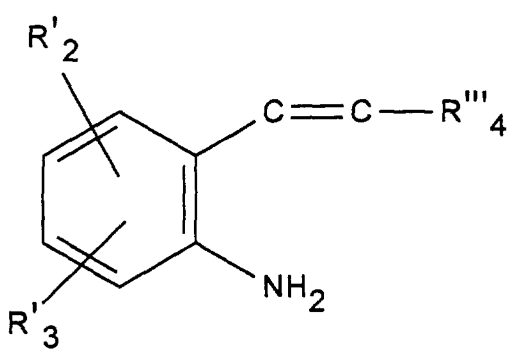

(XI)

dans laquelle $R'_2$, $R'_3$ et $R''\,'_4$ ont les significations indiquées ci-dessus, que l'on soumet à une réaction de cyclisation en présence d'un donneur d'azote tel que le nitrite de sodium, pour obtenir un produit de formule (IIC) :

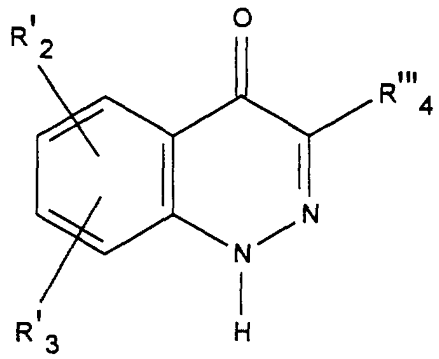

(IIc)

dans laquelle R'$_2$, R'$_3$ et R'' '$_4$ ont les significations indiquées ci-dessus,
d) soit l'on soumet un produit de formule (XIV) :

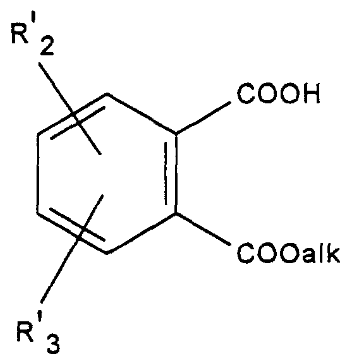

(XIV)

dans laquelle R'$_2$ et R'$_3$ et alk ont les significations indiquées ci-dessus, aprés, si désiré, une réaction d'halogénation de la fonction carboxy libre, à une réaction d'addition sur cette fonction carboxy d'un composé de formule R'$_4$-H, R'$_4$ ayant la signification indiquée ci-dessus, pour obtenir après cyclisation en présence d'hydrazine ou d'un dérivé des produits de formule (IIe) :

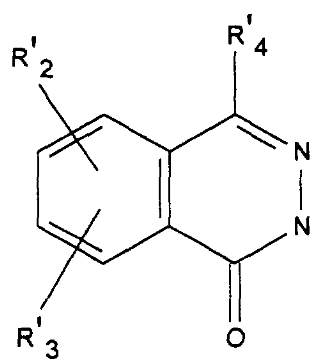

(IIe)

dans laquelle R'$_2$, R'$_3$ et R'$_4$ ont les significations indi quées ci-dessus,
e) soit l'on fait réagir un produit de formule (XV) :

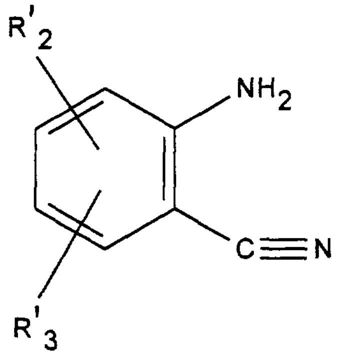

(XV)

dans laquelle $R'_2$ et $R'_3$ ont les significations indiquées ci-dessus avec un produit de formule (XVI) :

$R'_4$-CO-Cl (XVI)

dans laquelle $R'_4$ a la signification indiquée ci-dessus pour donner le produit de formule (XVII) :

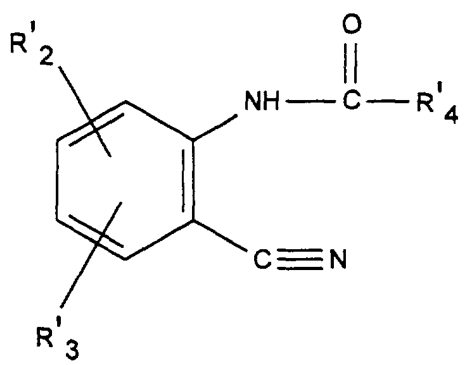

(XVII)

dans laquelle $R'_2$, $R'_3$ et $R'_4$ ont les significations indiquées précédemment, pour obtenir, aprés cyclisation, des produits de formule (IIf) :

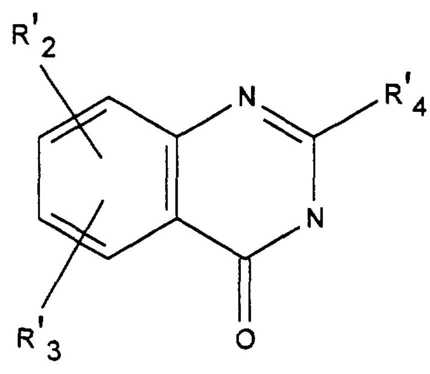

(IIf)

dans laquelle $R'_2$, $R'_3$ et $R'_4$ ont les significations indiquées ci-dessus, produits de formule (Ii) qui peuvent représenter des produits de formule (I) et produits de formules (IIb), (IIc), (IIe) et (IIf) telles que définies ci-dessus qui peuvent représenter des produits de formule (I) dans laquelle l'un au moins de $A_1$, $A_2$ et $A_3$ représente un radical méthine portant un radical hydroxyle, que l'on soumet, si désiré et si nécessaire, à l'une ou plusieurs des réactions suivantes, dans un ordre quelconque :

- une réaction de réduction complète du radical hydroxyle ou oxo en radical méthine suivie d'une aromatisation,
- sur les produits de formule (Ii) dans lesquels l'un de $R'_4$, $R''_4$ ou $R'''_4$ représente un radical hydroxyle ou sur les produits de formules (IIb), (IIc),(IIe) et (IIf) que l'on soumet

soit d'abord à une réaction de substitution du radical hydroxyle par un atome d'halogène suivie de l'action d'un produit de formule $R_{p4}$-M-Hal dans lequel $R_{p4}$ a la signification indiquée à la revendication 1 pour $R_4$ dans lesquelles les éventuelles fonctions réactives sont éventuellement protégées par des groupements protecteurs, M représente un atome de métal choisi parmi le magnésium, le cuivre et le zinc et Hal représente un atome d'halogène,
soit à l'action d'un produit de formule $R_{p4}$ - Hal dans lequel Hal représente un atome d'halogène, pour obtenir les produits de formule (I) correspondants,

- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification d'une fonction acide,
- une réaction de saponification d'une fonction ester en fonction acide,
- une réaction de transformation de la fonction cyano en fonction acide,
- une réaction de dédoublement des formes racémiques, lesdits 0 produits ainsi obtenus étant sous toutes les formes isomères possibles racémiques, énantiomères et diastéréoisomères. 2. Procédé selon la Revendication 1, pour la préparation de l'acide 4'-[(2-butyl-4-quinoleinyl)méthyl] (1,1'-biphényl)-2-carboxylique.

**Patentansprüche**

**Patentansprüche für folgende Vertragsstaaten : AT, BE, CH/LI, DE, DK, FR, GB, IT, LU, MC, NL, SE**

**1.** Produkte der Formel (1):

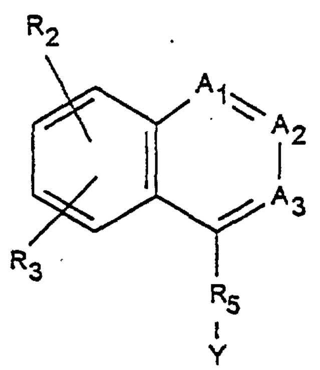

(I)

in der:

$R_2$ und $R_3$ ein Wasserstoffatom oder einen Alkylthiorest mit höchstens 4 Kohlenstoffatomen darstellen,
$A_1$, $A_2$ und $A_3$ derart sind, dass eins oder zwei von ihnen ein Stickstoffatom darstellen und die anderen, die gleich oder verschieden sind, den Rest

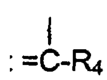

darstellen, derart, dass $R_4$ unter dem Wasserstoffatom, dem n-Butyl- und Alkylthiorest mit höchstens 4 Kohlenstoffatomen ausgewählt ist,
$R_5$ den Rest $-CH_2-$, -NH-, -O- und $-OCH_2-$ darstellt
und Y Phenyl, substituiert mit einem Tetrazolrest, oder Biphenyl, gegebenenfalls substituiert mit einem oder

mehreren Resten, die unter den Resten Cyano, freies, versalztes und verestertes Carboxy und Tetrazolyl ausgewählt sind, darstellt, mit Ausnahme der Produkte, in denen:

a) $A_1$ ein Stickstoffatom darstellt, $A_2$ und $A_3$, die gleich oder verschieden sind, den Rest

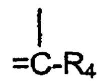

darstellen, in dem $R_4$ das Wasserstoffatom oder den n-Butylrest darstellt, $R_5$ -O-$CH_2$ darstellt,
b) $R_2$ und $R_3$ Wasserstoff darstellen,

$A_1$ ein Stickstoffatom darstellt,
$A_2$ und $A_3$ so sind, dass entweder das eine ein Stickstoffatom darstellt und das andere

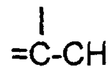

darstellt oder aber beide

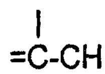

darstellen,
$R_5$ O, NH oder $CH_2$ darstellt
und Y Biphenyl, gegebenenfalls substituiert mit CN, darstellt,

wobei es sich versteht, dass die 5 folgenden Produkte:

- 4-[[(2-Butyl-4-chinolinyl)oxy]methyl]benzoesäure Chlorhydrat;
- 4'-[[(3-Butyl-5-methylthio-4-chinolinyl)oxy]methyl]-(1,1'-biphenyl)-2-carbonsäuremethylester;
- 4'-[[(3-Butyl-4-chinolinyl)oxy]methyl]-(1,1'-biphenyl)-2-carbonsäure;
- Diethylamin-Salz der 4'-[[(3-Butyl-1,4-dihydro-4-(methylthio)-4-chinolinyl)oxy]methyl]-(1,1'-biphenyl)-2-carbonsäure;
- 4'-[(2-Phenyl-4-chinazolinyl)methyl]-(1,1'-biphenyl)-2-carbonsäure der vorliegenden Erfindung angehören,

wobei besagte Produkte der Formel (1) und diese 5 Produkte in allen möglichen racemischen, enantiomeren und diastereoisomeren Isomerformen vorliegen, sowie die Additionssalze besagter Produkte der Formel (1) und dieser 5 Produkte mit den mineralischen und organischen Säuren oder mit den mineralischen und organischen Basen.

**2.** Die folgenden Produkte:

- Diethylamin-Salz der 4'-[[(3-Butyl-1,4-dihydro-4-(methylthio)-4-chinolinyl)oxy]-methyl]-(1,1'-biphenyl)-2-carbonsäure;
- 4'-[[(3-Butyl-4-chinolinyl)oxy]methyl]-(1,1'-biphenyl)-2-carbonsäure, wobei diese Produkte in allen möglichen racemischen, enantiomeren und diastereoisomeren Isomerformen vorliegen, sowie die Additionssalze dieser Produkte mit den mineralischen und organischen Säuren oder mit den mineralischen und organischen Basen.

**3.** 4'-[(2-Butyl-4-chinolinyl)methyl]-(1,1'-biphenyl)-2-carbonsäure.

**4.** Verfahren zur Herstellung von Produkten der Formel (I) und der Produkte, wie in den Ansprüchen 1 bis 3 definiert, **dadurch gekennzeichnet, dass**:

a) man entweder die Verbindung der Formel (III):

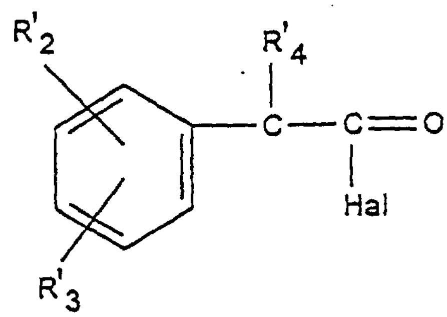

(III)

in der $R'_2$, $R'_3$ und $R'_4$ die in Anspruch 1 für $R_2$, $R_3$ beziehungsweise $R_4$ angegebenen Bedeutungen haben, in denen die etwaigen reaktiven Funktionen gegebenenfalls durch Schutzgruppen geschützt sind, wobei $R'_4$ auch einen Phenylrest darstellen kann,
und Hal ein Halogenatom darstellt, einer Substitutionsreaktion unterzieht, um das Produkt der Formel (IV):

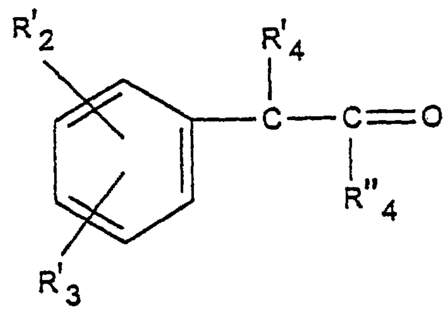

(IV)

in der $R'_2$, $R'_3$ und $R'_4$ die oben angegebenen Bedeutungen haben und $R''_4$, gleich oder verschieden von $R'_4$, die in Anspruch 1 für $R_4$ angegebene Bedeutung hat, in der die etwaigen reaktiven Funktionen gegebenenfalls durch Schutzgruppen geschützt sind, zu erhalten, das man:
mit einer Verbindung der Formel (V):

$$R'''_4\text{-}C{\equiv}N \qquad (V)$$

in der $R'''_4$, gleich oder verschieden von $R'_4$ oder $R''_4$, die in Anspruch 1 für $R_4$ angegebene Bedeutung hat, in der die etwaigen reaktiven Funktionen gegebenenfalls durch Schutzgruppen geschützt sind, umsetzt, um nach Cyklisierung ejn Produkt der Formel (I i):

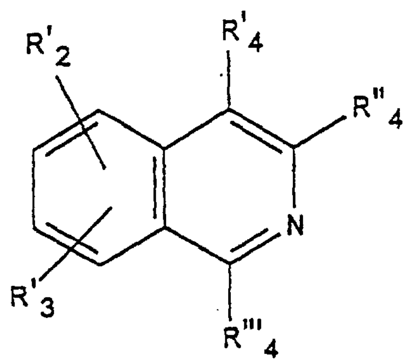

(I i)

in der $R'_2$, $R'_3$, $R'_4$, $R''_4$ und $R'''_4$ die oben angegebenen Bedeutungen haben, zu erhalten,
b) oder man ein Produkt der Formel (VI):

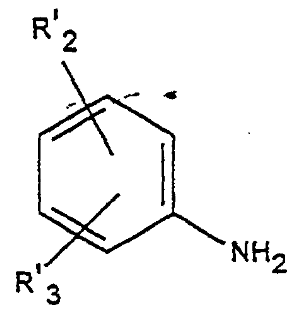

(VI)

in der $R'_2$ und $R'_3$ die oben angegebenen Bedeutungen haben, mit einem Produkt der Formel (VII):

$$R'_4\text{-CH}(CO_2\text{alk})\text{-CO-}R''_4 \qquad (VII)$$

in der $R'_4$ und $R''_4$, die gleich oder verschieden sind, die oben angegebenen Bedeutungen haben und alk einen Alkylrest mit höchstens 6 Kohlenstoffatomen darstellt, umsetzt, um Produkte der Formel (VIII):

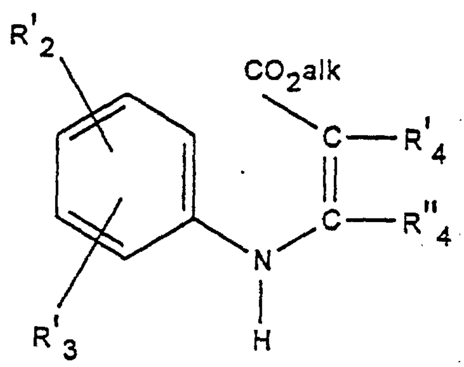

(VIII)

in der $R'_2$, $R'_3$, $R'_4$, $R''_4$ und alk die oben angegebenen Bedeutungen haben, zu erhalten, die man cyklisiert, um Produkte der Formel (II b):

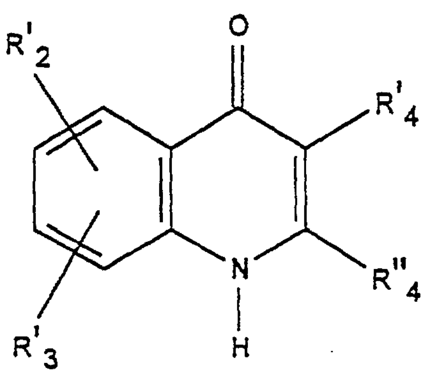

(II b)

in der $R'_2$, $R'_3$, $R'_4$ und $R''_4$ die oben angegebenen Bedeutungen haben, zu erhalten,
c) oder man ein Produkt der Formel (IX):

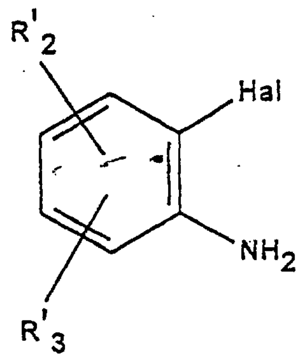

(IX)

in der $R'_2$ und $R'_3$ die oben angegebenen Bedeutungen haben, mit einem Produkt der Formel (X):

$$R'''_4\text{-}C{\equiv}CH \qquad (X)$$

in der $R'''_4$ die oben angegebene Bedeutung hat, umsetzt, um Produkte der Formel (XI):

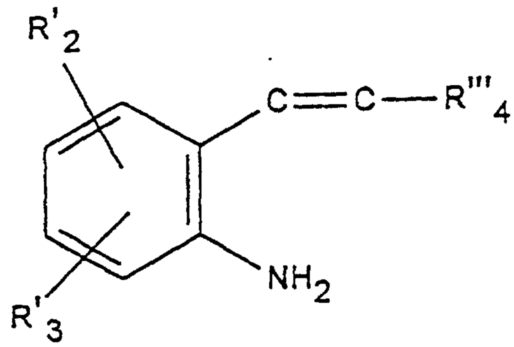

(XI)

in der $R'_2$, $R'_3$ und $R'''_4$ die oben angegebenen Bedeutungen haben, zu erhalten, die man in Gegenwart eines Stickstoffdonors, wie Natriumnitrit, einer Cyklisierungsreaktion unterzieht, um ein Produkt der Formel (II c):

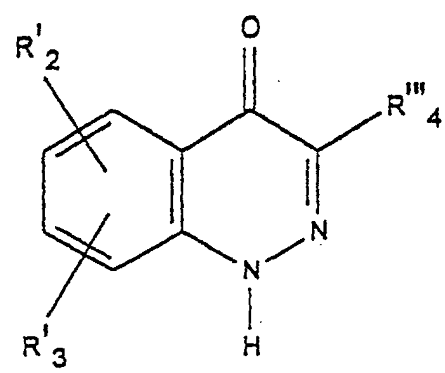

(II c)

in der $R'_2$, $R'_3$ und $R'''_4$ die oben angegebenen Bedeutungen haben, zu erhalten,

d) oder man ein Produkt der Formel (XIV):

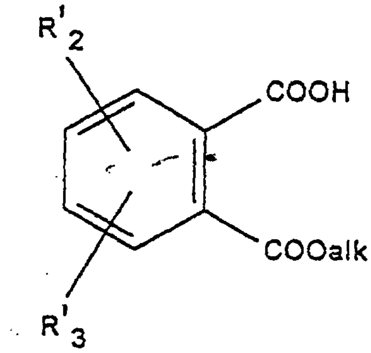

(XIV)

in der $R'_2$ und $R'_3$ und alk die oben angegebenen Bedeutungen haben, nach, wenn gewünscht, einer Reaktion zur Halogenierung der freien Carboxyfunktion, einer Reaktion zur Addition an diese Carboxyfunktion einer Verbindung der Formel $R'_4$-H, wobei $R'_4$ die oben angegebene Bedeutung hat, unterzieht, um nach Cyklisierung in Gegenwart von Hydrazin oder eines Derivats Produkte der Formel (II e):

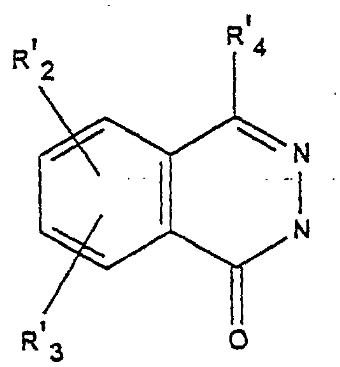

(II e)

in der $R'_2$, $R'_3$ und $R'_4$ die oben angegebenen Bedeutungen haben, zu erhalten,
e) oder man ein Produkt der Formel (XV):

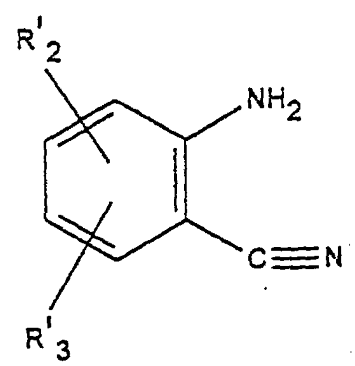

(XV)

in der $R'_2$ und $R'_3$ die oben angegebenen Bedeutungen haben, mit einem Produkt der Formel (XVI):

$$R'_4\text{-CO-Cl} \quad (XVI)$$

in der $R'_4$ die oben angegebene Bedeutung hat, wobei $R'_4$ auch einen Phenylrest darstellen kann, umsetzt, um das Produkt der Formel (XVII):

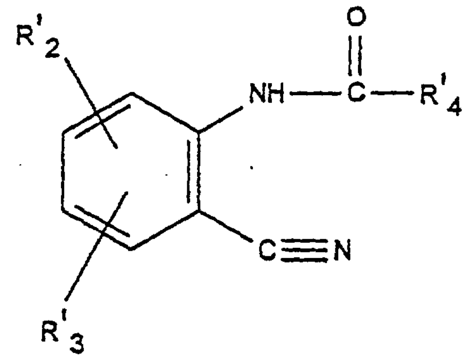

(XVII)

in der $R'_2$, $R'_3$ und $R'_4$ die zuvor angegebenen Bedeutungen haben, zu ergeben, um nach Cyklisierung Produkte der Formel (II f):

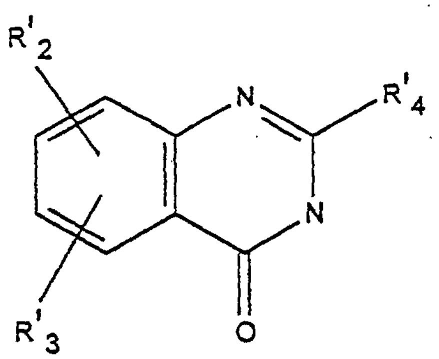

(II f)

in der $R'_2$, $R'_3$ und $R'_4$ die oben angegebenen Bedeutungen haben, zu erhalten, Produkte der Formel (I i), die Produkte der Formel (I) darstellen können, und Produkte der Formeln (II b), (II c), (II e) und (II f), wie oben definiert, die Produkte der Formel (I) darstellen können, in der wenigstens eins von $A_1$, $A_2$ und $A_3$ einen Methinrest, der einen Hydroxyrest trägt, darstellt, die man, wenn gewünscht und wenn notwendig, einer oder mehreren der folgenden Reaktionen in einer beliebigen Reihenfolge unterzieht:

- einer Reaktion zur vollständigen Reduktion des Hydroxy- oder Oxorests zum Methinrest und anschließender Aromatisierung,
- die Produkte der Formeln (II b), (II c), (II e) und (II f) unterzieht :

entweder zuerst einer Reaktion zur Substitution des Hydroxyrests durch ein Halogenatom und anschließend der Einwirkung eines Produkts der Formel $R_5$-Y-M-Hal, in dem $R_5$-Y- die in Anspruch 1 für $R_4$ angegebene Bedeutung hat, in denen die etwaigen reaktiven Funktionen gegebenenfalls durch Schutzgruppen geschützt sind, M ein Metallatom, das unter Magnesium, Kupfer und Zink ausgewählt ist, darstellt und Hal ein Halogenatom darstellt, oder der Einwirkung eines Produkts der Formel $R_5$Y-Hal, in dem Hal ein Halogenatom darstellt, um die entsprechenden Produkte der Formel (1) zu erhalten,

- einer Reaktion zur Entfernung der Schutzgruppen, die die geschützten reaktiven Funktionen tragen können,
- einer Reaktion zur Salzbildung durch eine mineralische oder organische Säure oder durch eine mineralische oder organische Base, um das entsprechende Salz zu erhalten,
- einer Reaktion zur Veresterung einer Säurefunktion,
- einer Reaktion zur Verseifung einer Esterfunktion zu einer Säurefunktion,
- einer Reaktion zur Umwandlung der Cyanofunktion in die Säurefunktion,
- einer Reaktion zur Spaltung der racemischen Formen, wobei besagte so erhaltene Produkte in allen möglichen racemischen, enantiomeren und diastereoisomeren Isomerformen vorliegen.

**5.** Als Medikamente die Produkte der Formel (I) und die Produkte, wie in den Ansprüchen 1 bis 3 definiert, wobei diese Produkte in allen möglichen racemischen, enantiomeren und diastereoisomeren Isomerformen vorliegen, sowie die Additionssalze dieser Produkte mit den mineralischen und organischen Säuren oder mit den mineralischen und organischen Basen, die pharmazeutisch annehmbar sind.

**6.** Die pharmazeutischen Zusammensetzungen, die als Wirkstoff wenigstens eins der Medikamente, wie in Anspruch 4 definiert, enthalten.

**7.** Verwendung der Produkte der Formel ($I_a$):

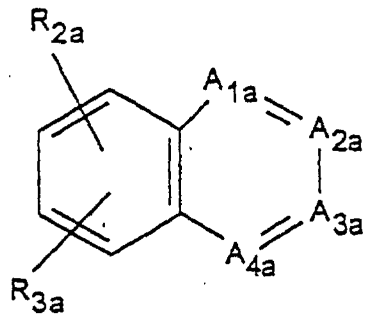

(I a)

in der:

$R_{2a}$ und $R_{3a}$, die gleich oder verschieden sind, aus der Gruppe ausgewählt sind, die gebildet wird von:

- dem Wasserstoffatom,
- den Halogenatomen,
- dem Hydroxyrest,
- dem Mercapto-, Cyano-, Nitro-, Formyl-, Benzoyl-, Acylrest mit höchstens 6 Kohlenstoffatomen,
- den freien, versalzten oder mit einem linearen oder verzweigten Alkylrest mit höchstens 4 Kohlenstoffatomen veresterten Carboxyresten,
- den Resten Alkyl, Alkyloxy und Alkylthio, linear oder verzweigt, mit höchstens 6 Kohlenstoffatomen, Phenyl, Naphthyl, Benzyl und Phenylthio, wobei alle diese Reste gegebenenfalls mit einem oder mehreren gleichen oder verschiedenen Resten substituiert sind, die ausgewählt sind unter den Halogenatomen, dem Hydroxyrest, den Resten Alkyloxy mit höchstens 4 Kohlenstoffatomen, Trifluormethyl, Cyano, Acyl, freies, versalztes oder verestertes Carboxy, Tetrazol und Isoxazol,
- den Resten Amino, Mono- oder Dialkylamino, Carbamoyl, Pyrrolyl, Morpholino, Piperazinyl, Pyrrolylmethyl, Morpholinomethyl, Piperazinylmethyl, Pyrrolylcarbonyl, Morpholinocarbonyl, Piperazinylcarbonyl, wobei alle Piperazinylreste dieser Reste gegebenenfalls am zweiten Stickstoffatom mit einem Alkyl- oder Phenylrest substituiert sind, wobei diese Alkyl- und Phenylreste selbst gegebenenfalls mit einem oder mehreren Resten substituiert sind, die ausgewählt sind unter den Halogenatomen, den Resten Hydroxy, Nitro, Alkyl, Alkyloxy oder Acyl mit höchstens 4 Kohlenstoffatomen, Trifluormethyl, Cyano, freies, versalztes oder verestertes Carboxy, Tetrazol und Isoxazol,

$A_{1a}$, $A_{2a}$, $A_{3a}$ und $A_{4a}$, die gleich oder verschieden sind, ein Stickstoffatom und den Rest

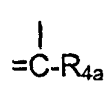

darstellen, derart, dass entweder $R_{4a}$ den Rest $R_{1a}$ darstellt, derart, dass $R_{1a}$ darstellt:

- das Wasserstoffatom, den Rest Hydroxy, Cyano, freies, versalztes oder mit einem Alkylrest mit höchstens 4 Kohlenstoffatomen verestertes Carboxy,
- den Alkyl-, Alkenyl-, Alkyloxy-, Acyl- oder Alkylthiorest, wobei diese Reste linear oder verzweigt sind und höchstens 7 Kohlenstoffatome enthalten, den Phenyl-, Benzyl-, Phenoxy-, Phenylthiorest, wobei alle diese aliphatischen oder cyclischen Reste gegebenenfalls mit einem oder mehreren Resten substituiert sind, die ausgewählt sind unter den Halogenatomen, den Resten Hydroxy, Nitro, Alkyl, Alkyloxy oder Acyl mit

höchstens 4 Kohlenstoffatomen, Trifluormethyl, Cyano, freies, versalztes oder mit einem Alkylrest mit höchstens 4 Kohlenstoffatomen verestertes Carboxy, Tetrazol und Isoxazol,
oder $R_{4a}$ den Rest -$R_{5a}$-Ya darstellt, in dem:

- $R_{5a}$ einen Rest -$CH_2$-, -NH-, -O-, -$OCH_2$- oder -$SCH_2$- darstellt und
- Ya einen Phenylrest, der mit einem Tetrazol- oder Isoxazolrest substituiert ist, oder Biphenylrest darstellt, der gegebenenfalls mit einem oder mehreren Resten substituiert ist, die ausgewählt sind unter den Resten Hydroxy, Halogen, Alkyl und Alkyoxy mit höchstens 4 Kohlenstoffatomen, Trifluormethyl, Cyano, Nitro, freies, versalztes oder verestertes Carboxy, Tetrazol und Isoxazol,
wobei es sich versteht, dass die Produkte der Formel (I a) derart sind, dass wenigstens eins und höchstens zwei von $A_{1a}$, $A_{2a}$, $A_{3a}$ und $A_{4a}$ ein Stickstoffatom darstellen und wenigstens eins den Methinrest darstellt, der mit dem Rest -$R_{5a}$-$Y_a$ substituiert ist,

mit Ausnahme der Produkte, in denen:

$A_{1a}$ ein Stickstoffatom darstellt,

$A_{2a}$ den Rest

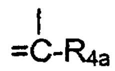

darstellt, in dem $R_{4a}$ ein Wasserstoffatom, einen Alkylrest, der gegebenenfalls substituiert ist mit einem oder mehreren Fluoratomen, den Resten Hydroxy, Phenyl oder Alkoxy (1 - 4 C), oder einen Phenylrest darstellt,

$A_{3a}$ den Rest

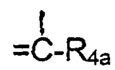

darstellt, in dem $R_{4a}$ ein Wasserstoffatom, einen Alkylrest, einen freien, veresterten oder versalzten Carboxyrest, Cyano-, Nitro-, Phenyl- oder Phenylalkylrest darstellt,

$A_{4a}$ den Rest

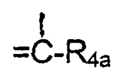

darstellt, in dem $R_{4a}$ den Rest -$R_{5a}$-$Y_a$ darstellt, in dem $R_{5a}$ den Rest -O-$CH_2$- darstellt, $Y_a$ den Phenylrest darstellt, der gegebenenfalls mit Tetrazol oder Phenyl substituiert ist, das selbst gegebenenfalls mit einem Substituenten substituiert ist, der ausgewählt ist unter den Resten Tetrazolyl, gegebenenfalls versalztes oder verestertes Carboxy, Alkyl, Alkoxy, Halogen, Trifluormethyl, Cyano und Nitro,

$R_{2a}$ und $R_{3a}$ ausgewählt sind unter dem Wasserstoffatom, den Halogenatomen, dem Rest Hydroxy, Trifluormethyl, Cyano, Nitro, Alkyl mit höchstens 4 Kohlenstoffatomen, gegebenenfalls substituiert mit Hydroxy oder Alkoxy mit höchstens 4 Kohlenstoffatomen, Alkoxy mit höchstens 4 Kohlenstoffatomen, gegebenenfalls substituiert mit einem Fluoratom, Alkylthio mit höchstens 6 Kohlenstoffatomen, Carbamoyl, Amino, gegebenenfalls substituiert mit einem oder zwei Alkylresten, um höchstens 6 Kohlenstoffatome zu enthalten, Carboxy, Alkoxycarbonyl mit höchstens 4 Kohlenstoffatomen, Acyl mit höchstens 4 Kohlenstoffatomen;

und Verwendung der folgenden 4 Produkte:

- 4-[[(2-Butyl-4-chinolinyl)oxy]methyl]benzoesäure Chlorhydrat;
- 4'-[[(3-Butyl-5-methylthio-4-chinolinyl)oxy]methyl]-(1,1'-biphenyl)-2-carbonsäuremethylester;

- Diethylamin-Salz der 4'-[[(3-Butyl-1,4-dihydro-4-(methylthio)-4-chinolinyl)oxy]-methyl]-(1,1'-biphenyl)-2-carbonsäure;
- 4'-[[(3-Butyl-4-chinolinyl)oxy]methyl]-(1,1'-biphenyl)-2-carbonsäure;

wobei besagte Produkte der Formel ($I_a$) und die 4 oben angegebenen Produkte in allen möglichen racemischen, enantiomeren und diastereoisomeren Isomerformen vorliegen, sowie der Additionssalze besagter Produkte der Formel ($I_a$) und der 4 oben angegebenen Produkte mit den mineralischen und organischen Säuren oder mit den mineralischen und organischen Basen
für die Herstellung von Medikamenten, die bestimmt sind
entweder für die Behandlung des arteriellen Bluthochdrucks, von Herzinsuffizienzen, Niereninsuffizienzen und bei der Vorbeugung von wiederkehrenden Verengungen nach Angioplastie,
oder für die Behandlung von bestimmten gastrointestinalen oder gynäkologischen Störungen.

**Patentansprüche für folgende Vertragsstaaten : ES, GR, PT**

**1.** Verfahren zur Herstellung der Produkte der Formel (I):

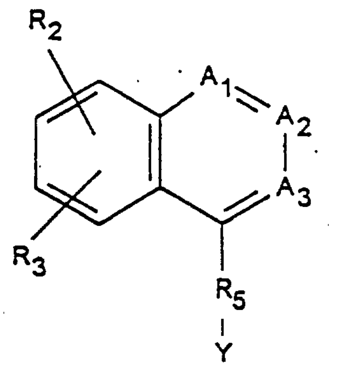

(I)

in der:

$R_2$ und $R_3$ ein Wasserstoffatom oder einen Alkylthiorest mit höchstens 4 Kohlenstoffatomen darstellen,
$A_1$, $A_2$ und $A_3$ derart sind, dass eins oder zwei von ihnen ein Stickstoffatom darstellen und die anderen, die gleich oder verschieden sind, den Rest

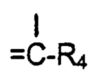

darstellen, derart, dass $R_4$ unter dem Wasserstoffatom, dem n-Butyl- und Alkylthiorest mit höchstens 4 Kohlenstoffatomen ausgewählt ist,
$R_5$ den Rest-$CH_2$-, -NH-, -O- und -$OCH_2$- darstellt
und Y Phenyl, substituiert mit einem Tetrazolrest, oder Biphenyl, gegebenenfalls substituiert mit einem oder mehreren Resten, die unter den Resten Cyano, freies, versalztes und verestertes Carboxy und Tetrazolyl ausgewählt sind, darstellt, mit Ausnahme der Produkte, in denen:

a) $A_1$ ein Stickstoffatom darstellt,
$A_2$ und $A_3$, die gleich oder verschieden sind, den Rest

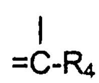

darstellen, in dem $R_4$ das Wasserstoffatom oder den n-Butylrest darstellt, $R_5$ -O-$CH_2$ darstellt,
b) $R_2$ und $R_3$ Wasserstoff darstellen,

$A_1$ ein Stickstoffatom darstellt,
$A_2$ und $A_3$ so sind, dass entweder das eine ein Stickstoffatom darstellt und das andere

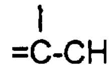

darstellt oder aber beide

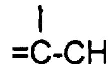

darstellen,
$R_5O$, NH oder $CH_2$ darstellt
und Y Biphenyl, gegebenenfalls substituiert mit CN, darstellt,

und der 5 folgenden Produkte:

- 4-[[(2-Butyl-4-chinolinyl)oxy]methyl]benzoesäure Chlorhydrat;
- 4'-[[(3-Butyl-5-methylthio-4-chinolinyl)oxy]methyl]-(1,1'-biphenyl)-2-carbonsäuremethylester;
- Diethylamin-Salz der 4'-[[(3-Butyl-1,4-dihydro-4-(methylthio)-4-chinolinyl)oxy]-methyl]-(1,1'-biphenyl)-2-carbonsäure;
- 4'-[[(3-Butyl-4-chinolinyl)oxy]methyl]-(1,1'-biphenyl)-2-carbonsäure;
- 4'-[(2-Phenyl-4-chinazolinyl)methyl]-(1,1'-biphenyl)-2-carbonsäure,

wobei besagte Produkte der Formel (I) und diese 5 Produkte in allen möglichen racemischen, enantiomeren und diastereoisomeren Isomerformen vorliegen, sowie der Additionssalze besagter Produkte der Formel (I) und dieser 5 Produkte mit den mineralischen und organischen Säuren oder mit den mineralischen und organischen Basen, **dadurch gekennzeichnet, dass**:

a) man entweder die Verbindung der Formel (III):

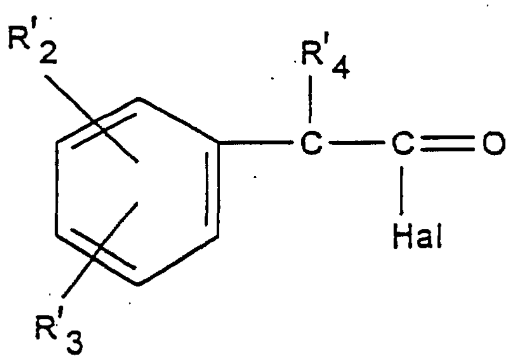

(III)

in der $R'_2$, $R'_3$ und $R'_4$ die in Anspruch 1 für $R_2$, $R_3$ beziehungsweise $R_4$ angegebenen Bedeutungen haben, in denen die etwaigen reaktiven Funktionen gegebenenfalls durch Schutzgruppen geschützt sind, wobei $R'_4$ auch einen Phenylrest darstellen kann,
und Hal ein Halogenatom darstellt, einer Substitutionsreaktion unterzieht, um das Produkt der Formel (IV):

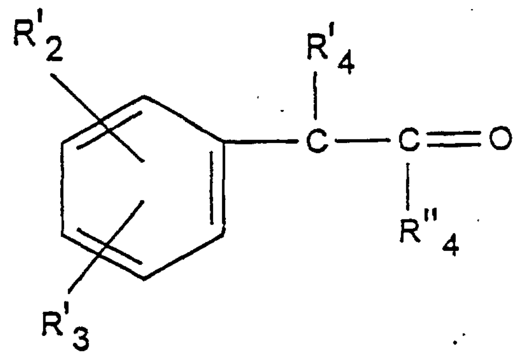

(IV)

in der $R'_2$, $R'_3$ und $R'_4$ die oben angegebenen Bedeutungen haben und $R''_4$, gleich oder verschieden von $R'_4$, die in Anspruch 1 für $R_4$ angegebene Bedeutung hat, in der die etwaigen reaktiven Funktionen gegebenenfalls durch Schutzgruppen geschützt sind, zu erhalten, das man:
mit einer Verbindung der Formel (V):

$$R'''_4\text{-}C{\equiv}N \qquad (V)$$

in der $R'''_4$, gleich oder verschieden von $R'_4$ oder $R''_4$, die in Anspruch 1 für $R_4$ angegebene Bedeutung hat, in der die etwaigen reaktiven Funktionen gegebenenfalls durch Schutzgruppen geschützt sind, umsetzt, um nach Cyclisierung ein Produkt der Formel (I i):

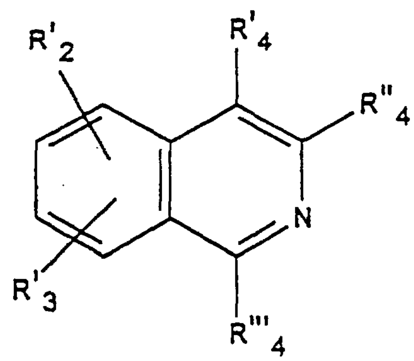

(I i)

in der $R'_2$, $R'_3$, $R'_4$, $R''_4$ und $R'''_4$ die oben angegebenen Bedeutungen haben, zu erhalten,
b) oder man ein Produkt der Formel (VI):

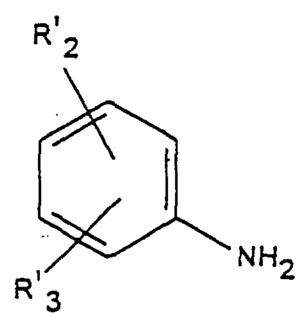

(VI)

in der $R'_2$ und $R'_3$ die oben angegebenen Bedeutungen haben, mit einem Produkt der Formel (VII):

$$R'_4\text{-}CH(CO_2alk)\text{-}CO\text{-}R''_4 \qquad \text{(VII)}$$

in der $R'_4$ und $R''_4$, die gleich oder verschieden sind, die oben angegebenen Bedeutungen haben und alk einen Alkylrest mit höchstens 6 Kohlenstoffatomen darstellt, umsetzt, um Produkte der Formel (VIII):

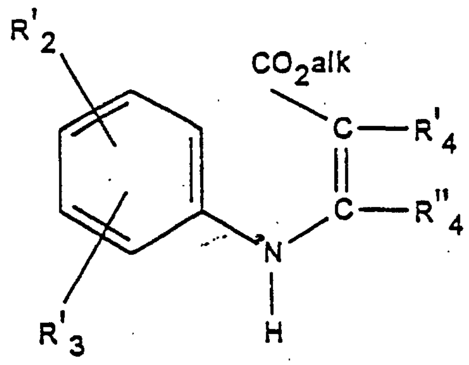

(VIII)

in der $R'_2$, $R'_3$, $R'_4$, $R''_4$ und alk die oben angegebenen Bedeutungen haben, zu erhalten, die man cyclisiert, um Produkte der Formel (II b):

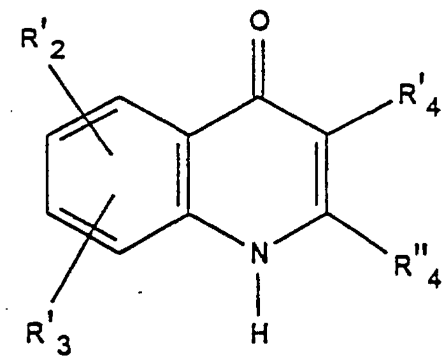

(II b)

in der $R'_2$, $R'_3$, $R'_4$ und $R''_4$ die oben angegebenen Bedeutungen haben, zu erhalten,
c) oder man ein Produkt der Formel (IX):

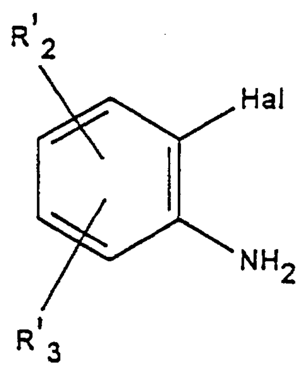

(IX)

in der $R'_2$ und $R'_3$ die oben angegebenen Bedeutungen haben, mit einem Produkt der Formel (X):

$R'''_4-C\equiv CH$ (X)

in der $R'''_4$ die oben angegebene Bedeutung hat, umsetzt, um Produkte der Formel (XI):

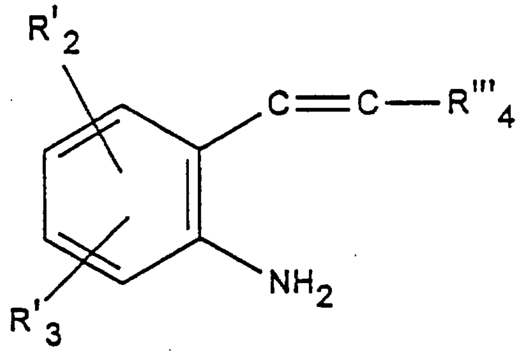

(XI)

in der $R'_2$, $R'_3$ und $R'''_4$ die oben angegebenen Bedeutungen haben, zu erhalten, die man in Gegenwart eines Stickstoffdonors, wie Natriumnitrit, einer Cyclisierungsreaktion unterzieht, um ein Produkt der Formel (II c):

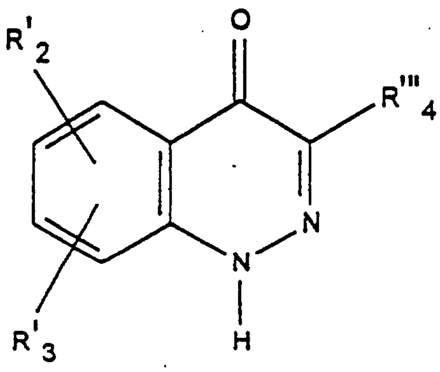

(II c)

in der $R'_2$, $R'_3$ und $R'''_4$ die oben angegebenen Bedeutungen haben, zu erhalten,
d) oder man ein Produkt der Formel (XIV):

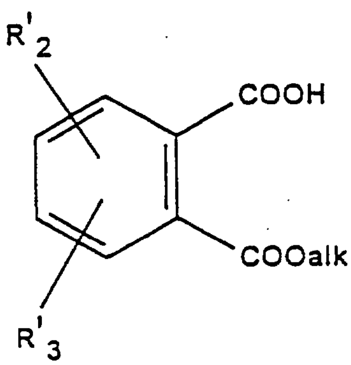

(XIV)

in der $R'_2$ und $R'_3$ und alk die oben angegebenen Bedeutungen haben, nach, wenn gewünscht, einer Reaktion zur Halogenierung der freien Carboxyfunktion, einer Reaktion zur Addition an diese Carboxyfunktion einer Verbindung der Formel $R'_4$-H, wobei $R'_4$ die oben angegebene Bedeutung hat, unterzieht, um nach Cyclisierung in Gegenwart von Hydrazin oder eines Derivats Produkte der Formel (11 e):

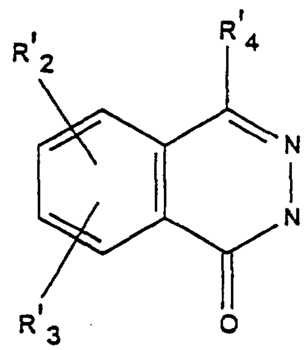

(II e)

in der $R'_2$, $R'_3$ und $R'_4$ die oben angegebenen Bedeutungen haben, zu erhalten,
e) oder man ein Produkt der Formel (XV):

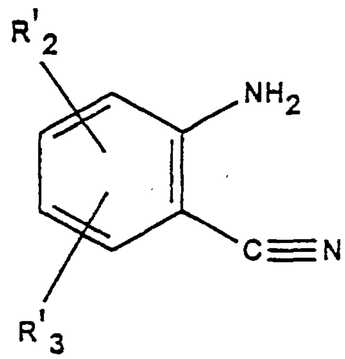

(XV)

in der $R'_2$ und $R'_3$ die oben angegebenen Bedeutungen haben, mit einem Produkt der Formel (XVI):

$R'_4$-CO-Cl (XVI)

in der $R'_4$ die oben angegebene Bedeutung hat, umsetzt, um das Produkt der Formel (XVII):

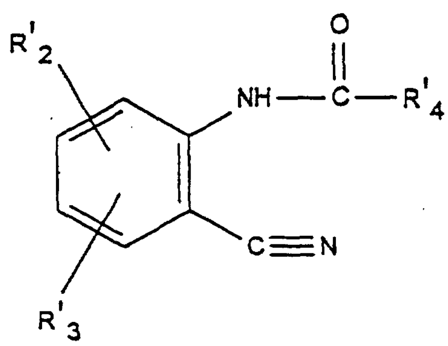

(XVII)

in der $R'_2$, $R'_3$ und $R'_4$ die zuvor angegebenen Bedeutungen haben, zu ergeben, um nach Cyclisierung Produkte der Formel (II f):

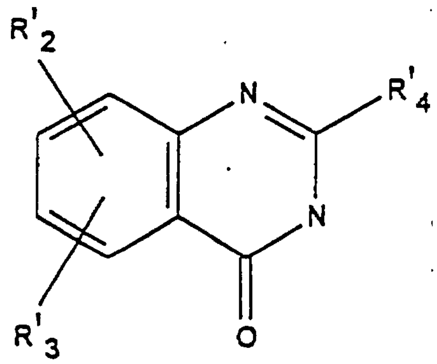

(II f)

in der $R'_2$, $R'_3$ und $R'_4$ die oben angegebenen Bedeutungen haben, zu erhalten, Produkte der Formel (I i), die Produkte der Formel (I) darstellen können, und Produkte der Formeln (II b), (II c), (II e) und (II f), wie oben definiert, die Produkte der Formel (I) darstellen können, in der wenigstens eins von $A_1$, $A_2$ und $A_3$ einen Methinrest, der einen Hydroxyrest trägt, darstellt, die man, wenn gewünscht und wenn notwendig, einer oder mehreren der folgenden Reaktionen in einer beliebigen Reihenfolge unterzieht:

- einer Reaktion zur vollständigen Reduktion des Hydroxy- oder Oxorests zum Methinrest und anschließender Aromatisierung,
- die Produkte der Formel (I i), in denen eins von $R'_4$, $R''_4$ oder $R'''_4$ einen Hydroxyrest darstellt, oder die Produkte der Formeln (II b), (II c), (II e) und (II f) unterzieht:

entweder zuerst einer Reaktion zur Substitution des Hydroxyrests durch ein Halogenatom und anschließend der Einwirkung eines Produkts der Formel $R_{p4}$-M-Hal, in dem $R_{p4}$ die in Anspruch 1 für $R_4$ angegebene Bedeutung hat, in denen die etwaigen reaktiven Funktionen gegebenenfalls durch Schutzgruppen geschützt sind, M ein Metallatom, das unter Magnesium, Kupfer und Zink ausgewählt ist, darstellt und Hal ein Halogenatom darstellt,
oder der Einwirkung eines Produkts der Formel $R_{p4}$-Hal, in dem Hal ein Halogenatom darstellt, um die entsprechenden Produkte der Formel (I) zu erhalten,

- einer Reaktion zur Entfernung der Schutzgruppen, die die geschützten reaktiven Funktionen tragen können,
- einer Reaktion zur Salzbildung durch eine mineralische oder organische Säure oder durch eine mineralische oder organische Base, um das entsprechende Salz zu erhalten,
- einer Reaktion zur Veresterung einer Säurefunktion,
- einer Reaktion zur Verseifung einer Esterfunktion zu einer Säurefunktion,
- einer Reaktion zur Umwandlung der Cyanofunktion in die Säurefunktion,
- einer Reaktion zur Spaltung der racemischen Formen, wobei besagte so erhaltene Produkte in allen möglichen racemischen, enantiomeren und diastereoisomeren Isomerformen vorliegen.

**2.** Verfahren gemäß Anspruch 1 für die Herstellung der 4'-[(2-Butyl-4-chinolinyl)methyl]-(1 ,1'-biphenyl)-2-carbonsäure.

## Claims

**Claims for the following Contracting States : AT, BE, CH/LI, DE, DK, FR, GB, IT, LU, MC, NL, SE**

**1.** Products of formula (I):

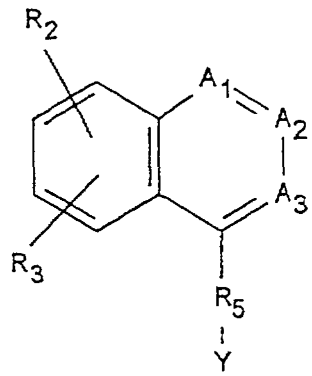

(I)

in which:

$R_2$ and $R_3$ represent a hydrogen atom or an alkylthio radical containing at most 4 carbon atoms,
$A_1$, $A_2$ and $A_3$ are such that one or two of them represent a nitrogen atom, and the others, identical or different, represent the

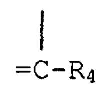

radical such that $R_4$ is chosen from the hydrogen atom, the n-butyl and alkylthio radical containing at most 4 carbon atoms,
$R_5$ represents the $-CH_2-$, -NH-, -O- and $-OCH_2-$ radical and Y represents phenyl substituted by a tetrazole or biphenyl radical optionally substituted by one or more radicals chosen from the cyano, free, salified and esterified carboxy and tetrazolyl radicals, with the exception of the products in which:

a) $A_1$ represents a nitrogen atom,
$A_2$ and $A_3$, identical or different, represent the

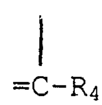

radical in which $R_4$ represents the hydrogen atom or the n-butyl radical, $R_5$ represents $-O-CH_2$,
b) $R_2$ and $R_3$ represent hydrogen,

$A_1$ represents a nitrogen atom,
$A_2$ and $A_3$ are such that one represents a nitrogen atom and the other represents

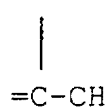

or both represent

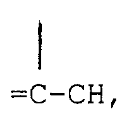

$R_5$ represents 0, NH or $CH_2$ and Y represents biphenyl optionally substituted by CN, it being understood that the following 5 products:

- hydrochloride of 4-[[(2-butyl 4-quinolinyl) oxy] methyl] benzoic acid;
- methyl 4'-[[(3-butyl 5-methylthio 4-quinolinyl) oxy] methyl] (1,1'-biphenyl)-2-carboxylate;
- 4'-[[(3-butyl 4-quinolinyl) oxy] methyl] (1,1'-biphenyl)-2-carboxylic acid;
- diethylamine salt of 4'-[[(3-butyl 1,4-dihydro 4-(methylthio) 4-quinolinyl) oxy] methyl] (1,1'-biphenyl) 2-carboxylic acid;
- 4'-[(2-phenyl 4-quinazolinyl) methyl] (1,1'-biphenyl)-2-carboxylic acid;

belong to the present invention,
said products of formula (I) and these 5 products being in all possible racemic, enantiomeric and diastereoisomeric isomer forms, as well as the addition salts with mineral and organic acids or with mineral and organic bases of said products of formula (I)and of these 5 products.

**2.** the following products

- diethylamine salt of 4'-[[(3-butyl 1,4-dihydro 4-(methylthio) 4-quinolinyl) oxy] methyl] (1,1'-biphenyl) 2-carboxylic acid;
- 4'-[[(3-butyl 4-quinolinyl) oxy] methyl] (1,1'-biphenyl)-2-carboxylic acid these products being in all possible racemic, enantiomeric and diastereoisomeric isomer forms, as well as the addition salts with mineral and organic acids or with mineral and organic bases of these products.

**3.** 4'-[(2-butyl 4-quinolinyl) methyl] (1,1'-biphenyl)-2-carboxylic acid.

**4.** Process for the preparation of products of formula (I) and of the products as defined in claims 1 to 3, **characterized in that**:

a) either the compound of formula (III):

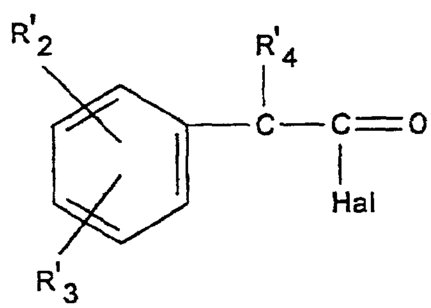

(III)

in which $R'_2$, $R'_3$ and $R'_4$ have the meanings indicated in claim 1 for $R_2$, $R_3$ and $R_4$ respectively in which the optional reactive functions are optionally protected by the protective groups, $R_4$ can also represent a phenyl radical,
and Hal represents a halogen atom, is subjected to a substitution reaction in order to obtain the product of formula (IV):

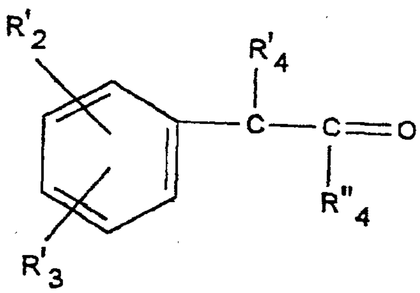

(IV)

in which $R'_2$, $R'_3$ and $R'_4$ have the meanings indicated above and $R''_4$, identical to or different from $R'_4$, has the meaning indicated in claim 1 for $R_4$ in which the optional reactive functions are optionally protected by protective groups, is reacted:

with a compound of formula (V):

$$R'''_4\text{-}C\equiv N \qquad (V)$$

in which $R'''_4$, identical to or different from $R'_4$ or $R''_4$, has the meaning indicated in claim 1 for $R_4$ in which the optional reactive functions are optionally protected by protective groups, in order to obtain after cyclization a product of formula (Ii):

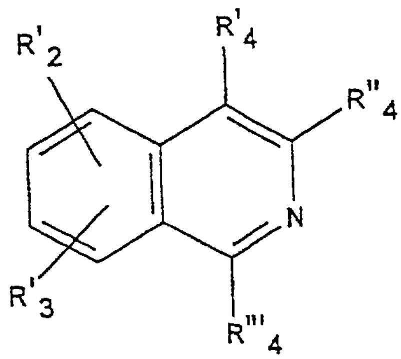

(Ii)

in which $R'_2$, $R'_3$, $R'_4$, $R'_4$ and $R'''_4$ have the meanings indicated above,

b) or a product of formula (VI):

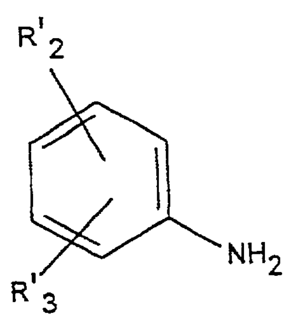

(VI)

in which $R'_2$ and $R'_3$ have the meanings indicated above is reacted with a product of formula (VII):

$$R'_4\text{-CH}(CO_2alk)\text{-CO-}R''_4 \qquad \text{(VII)}$$

in which $R'_4$ and $R''_4$, identical or different, have the meanings indicated above and alk represents an alkyl radical containing at most 6 carbon atoms, in order to obtain products of formula (VIII):

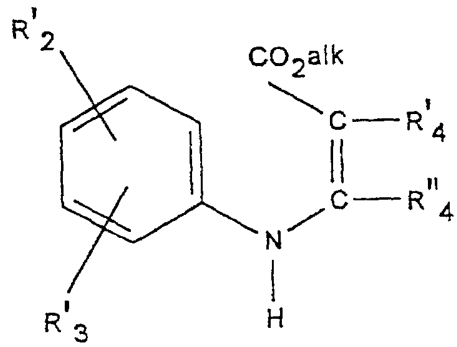

(VIII)

in which $R'_2$, $R'_3$, $R'_4$, $R''_4$ and alk have the meanings indicated above, which is cyclized in order to obtain products of formula (IIb):

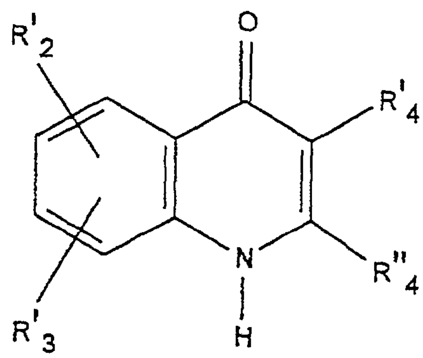

(IIb)

in which $R'_2$, $R'_3$, $R'_4$ and $R''_4$ have the meanings indicated above,
c) or a product of formula (IX):

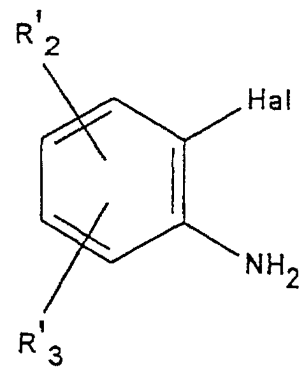

(IX)

in which $R'_2$ and $R'_3$ have the meanings indicated above is reacted with a product of formula (X):

$$R''{}'_4\text{-}C\equiv CH \quad (X)$$

in which $R''{}'_4$ has the meaning indicated above in order to obtain products of formula (XI):

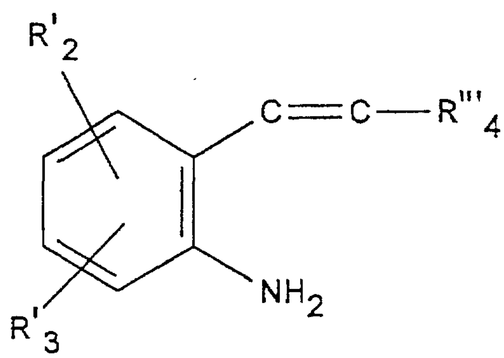

(XI)

in which $R'_2$, $R'_3$ and $R''{}'_4$ have the meanings indicated above, which is subjected to a cyclization reaction in the presence of a nitrogen donor such as sodium nitrite, in order to obtain a product of formula (IIc):

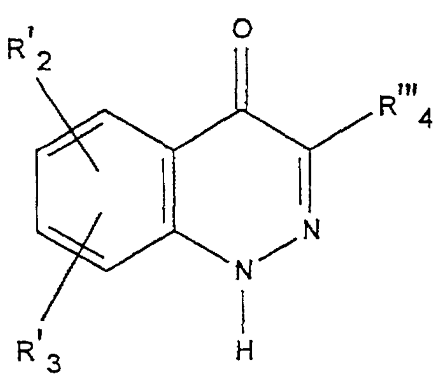

(IIc)

in which $R'_2$, $R'_3$ and $R'''_4$ have the meanings indicated above,
d) or a product of formula (XIV):

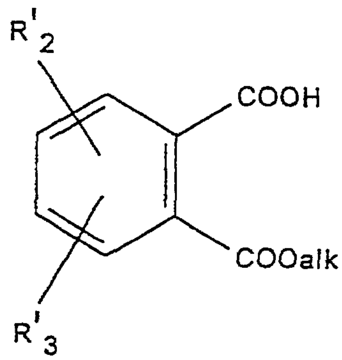

(XIV)

in which $R'_2$ and $R'_3$ and alk have the meanings indicated above, which, if desired, after a halogenation reaction of the free carboxy function, is subjected to an addition reaction on this carboxy function of a compound of formula $R'_4$-H, $R'_4$ having the meaning indicated above, in order to obtain after cyclization in the presence of hydrazine or of a derivative the products of formula (IIe) :

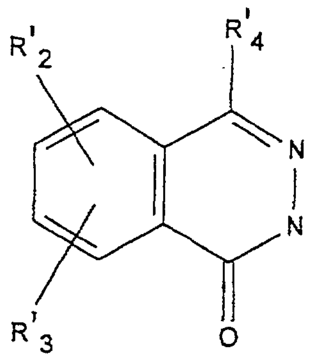

(IIe)

in which $R'_2$, $R'_3$ and $R'_4$ have the meanings indicated above,
e) or a product of formula (XV):

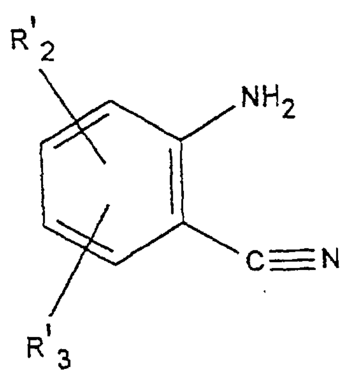

(XV)

in which $R'_2$ and $R'_3$ have the meanings indicated above is reacted with a product of formula (XVI):

$R'_4$-CO-Cl (XVI)

in which $R'_4$ has the meaning indicated above, $R'_4$ can also represent a phenyl radical in order to produce the product of formula (XVII):

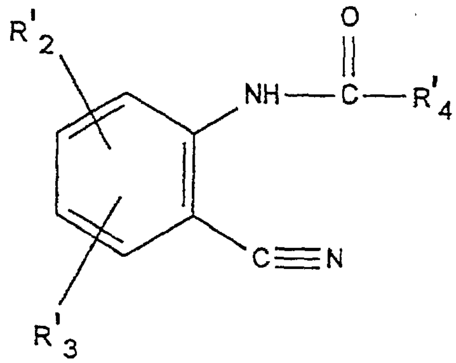

(XVII)

in which $R'_2$, $R'_3$ and $R'_4$ have the meanings indicated previously, in order to obtain, after cyclization, the products of formula (IIf):

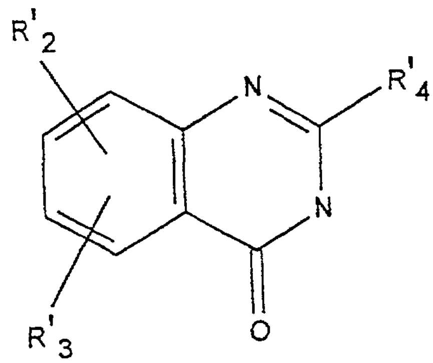

(IIf)

in which $R'_2$, $R'_3$ and $R'_4$ have the meanings indicated above, which products of formula (Ii) can represent the products of formula (I) and products of formulae (IIb), (IIc), (IIe) and (IIf) as defined above which can represent the products of formula (I) in which at least one of $A_1$, $A_2$ and $A_3$ represents a methine radical carrying a hydroxyl radical, which, if desired and if necessary, is subjected to one or more of the following reactions, in any order:

- a complete reduction reaction of the hydroxyl or oxo radical to a methine radical followed by an aromatization,
- on the products of formulae (IIb), (IIc), (IIe) and (IIf) which are subjected either firstly to a substitution reaction of the hydroxyl radical by a halogen atom followed by the action of a product of formula $R_5$-Y-M-Hal in which $R_5$-Y- has the meaning indicated in claim 1 for $R_4$ in which the optional reactive functions are optionally protected by protective groups, M represents a metal atom chosen from magnesium, copper and zinc and Hal represents a halogen atom, or to the action of a product of formula $R_5$Y - Hal in which Hal represents a halogen atom, in order to obtain the corresponding products of formula (I),
- an elimination reaction of the protective groups which can be carried by the protected reactive functions,
- a salification reaction by a mineral or organic acid or by a mineral or organic base in order to obtain the corresponding salt,
- an esterification reaction of an acid function,
- a saponification reaction of an ester function to an acid function,
- a conversion reaction of the cyano function to an acid function,
- a resolution reaction of the racemic forms, said products thus obtained being in all possible racemic, enantiomeric and diastereoisomeric isomer forms.

**5.** As medicaments, the products of formula (I) and the products as defined in claims 1 to 3, these products being in all possible racemic, enantiomeric and diastereoisomeric isomer forms, as well as the addition salts with pharmaceutically acceptable mineral and organic acids or mineral and organic bases of these products.

**6.** The pharmaceutical compositions containing as active ingredient, at least one of the medicaments as defined in claim 4.

**7.** Use of the products of formula ($I_a$):

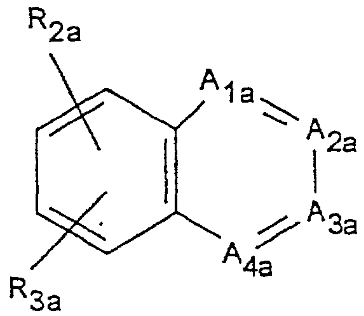

(Ia)

in which:

$R_{2a}$ and $R_{3a}$, identical or different, are chosen from the group form by:

- the hydrogen atom,
- the halogen atoms,
- the hydroxyl radical,
- the mercapto, cyano, nitro, formyl, benzoyl, acyl radical having at most 6 carbon atoms,
- the carboxy radicals free, salified or esterified by a linear or branched alkyl radical containing at most 4 carbon atoms,
- the linear or branched alkyl, alkyloxy and alkylthio radicals containing at most 6 carbon atoms, phenyl, naphthyl, benzyl and phenylthio, all these radicals being optionally substituted by one or more identical or different radicals chosen from the halogen atoms, the hydroxyl radical, alkyloxy radicals containing at most 4 carbon atoms, trifluoromethyl, cyano, acyl, free, salified or esterified carboxy, tetrazole and isoxazole radicals,
- the amino, mono- or dialkylamino, carbamoyl, pyrrolyl, morpholino, piperazinyl, pyrrolylmethyl, morpholinomethyl, piperazinylemethyl, pyrrolylcarbonyl, morpholinocarbonyl, piperazinylcarbonyl radicals, all the piperazinyl radicals of these radicals being optionally substituted on the second nitrogen atom by an alkyl or phenyl radical, these alkyl and phenyl radicals being themselves optionally substituted by one or more radicals chosen from halogen atoms, the hydroxyl, nitro, alkyl, alkyloxy or acyl radicals containing at most 4 carbon atoms, trifluoromethyl, cyano, free, salified or esterified carboxy, tetrazole and isoxazole,

$A_{1a}$, $A_{2a}$, $A_{3a}$ and $A_{4a}$, identical or different, represent a nitrogen atom and the

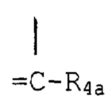

radical such that:

either $R_{4a}$ represents the $R_{1a}$ radical such that $R_{1a}$ represents:

- the hydrogen atom, the hydroxyl, cyano, carboxy radical free, salified or esterified by an alkyl radical containing at most 4 carbon atoms,
- the alkyl, alkenyl, alkyloxy, acyl or alkylthio radical, these radicals being linear or branched and containing at most 7 carbon atoms, the phenyl, benzyl, phenoxy, phenylthio radical, all these aliphatic or cyclic radicals

being optionally substituted by one or more radicals chosen from halogen atoms, the hydroxyl, nitro, alkyl, alkyloxy or acyl radicals containing at most 4 carbon atoms, trifluoromethyl, cyano, carboxy free, salified or esterified by an alkyl radical containing at most 4 carbon atoms, tetrazole and isoxazole radicals,

or $R_{4a}$ represents the - $R_{5a}$ - Ya radical in which:

- $R_{5a}$ represents a $-CH_{2-}$, -NH-, -O-, $-OCH_2-$ or $-SCH_2$-radical

and - Ya represents a phenyl radical substituted by a tetrazole or isoxazole or biphenyl radical optionally substituted by one or more radicals chosen from the hydroxyl, halogen, alkyl and alkyloxy radicals containing at most 4 carbon atoms, trifluoromethyl, cyano, nitro, free, salified or esterified carboxy, tetrazole and isoxazole radicals, it being understood that the products of formula (Ia) are such that one at least and two at most of $A_{1a}$, $A_{2a}$, $A_{3a}$ and $A_{4a}$ represent a nitrogen atom and one at least represents the methine radical substituted by the - $R_{5a}$- Ya, radical with the exception of the products in which:

$A_{1a}$ represents a nitrogen atom,

$A_{2a}$ represents the

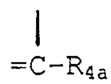

radical in which $R_{4a}$ represents a hydrogen atom, an alkyl radical optionally substituted by one or more fluorine atoms, the hydroxyl, phenyl or (1-4C) alkoxy radicals or a phenyl radical,

$A_{3a}$ represents the

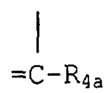

radical in which $R_{4a}$ represents a hydrogen atom, an alkyl radical, free, esterified or salified carboxy, cyano, nitro, phenyl or phenylalkyl,

$A_{4a}$ represents the

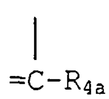

radical in which $R_{4a}$ represents the $-R_{5a}$-Ya radical in which $R_{5a}$ represents the $-O-CH_2$-radical, $Y_a$ represents the phenyl radical optionally substituted by tetrazole or phenyl, itself optionally substituted by a substituent chosen from tetrazolyl, carboxy optionally salified or esterified, alkyl, alkoxy, halogen, trifluoromethyl, cyano and nitro radicals,

$R_{2a}$ and $R_{3a}$ are chosen from the hydrogen atom; the halogen atoms; the hydroxyl; trifluoromethyl; cyano; nitro; alkyl radical containing at most 4 carbon atoms optionally substituted by hydroxyl or alkoxy containing at most 4 carbon atoms; alkoxy containing at most 4 carbon atoms optionally substituted by a fluorine atom; alkylthio containing at most 6 carbon atoms; carbamoyl; amino optionally substituted by one or two alkyl radicals containing at most 6 carbon atoms; carboxy; alcoxycarbonyl containing at most 4 carbon atoms; acyl containing at most 4 carbon atoms; and use of the following 4 products:

- hydrochloride of 4-[[(2-butyl 4-quinolinyl) oxy] methyl] benzoic acid;
- methyl 4'-[[(3-butyl 5-methylthio 4-quinolinyl) oxy] methyl] (1,1'-biphenyl)-2-carboxylate;

- diethylamine salt of 4'-[[(3-butyl 1,4-dihydro 4-(methylthio) 4-quinolinyl) oxy] methyl] (1,1'-biphenyl) 2-carboxylic acid;
- 4'-[[(3-butyl 4-quinolinyl) oxy] methyl] (1,1'-biphenyl)-2-carboxylic acid,

said products of formula ($I_a$) and the 4 products indicated above being in all possible racemic, enantiomeric and diastereoisomeric isomer forms, as well as the addition salts with mineral and organic acids or with mineral and organic bases of said products of formula ($I_a$) and of the 4 products indicated above for the preparation of medicaments intended,
either for the treatment of arterial hypertension, cardiac insufficiencies, renal insufficiencies and in the prevention of the post-angioplasty recurrence of stenosis,
or for the treatment of certain gastro-intestinal or gynaecological disorders.

**Claims for the following Contracting States : ES, GR, PT**

**1.** Process for the preparation of the products of formula (I):

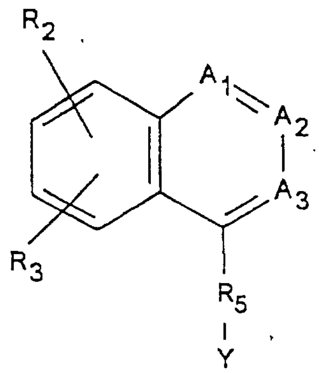

(I)

in which:

$R_2$ and $R_3$ represent a hydrogen atom or an alkylthio radical containing at most 4 carbon atoms,
$A_1$, $A_2$ and $A_3$ are such that one or two of them represent a nitrogen atom, and the others, identical or different, represent the

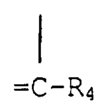

radical such that $R_4$ is chosen from the hydrogen atom, the n-butyl and alkylthio radical containing at most 4 carbon atoms,
$R_5$ represents the $-CH_2-$, -NH-, -O- and $-OCH_2-$ radical and Y represents phenyl substituted by a tetrazole or biphenyl radical optionally substituted by one or more radicals chosen from the cyano, free, salified and esterified carboxy and tetrazolyl radicals, with the exception of the products in which:

a) $A_1$ represents a nitrogen atom,
$A_2$ and $A_3$, identical or different, represent the

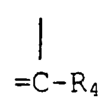

radical in which $R_4$ represents the hydrogen atom or the n-butyl radical, $R_5$ represents $-O-CH_2$

b) $R_2$ and $R_3$ represent hydrogen,

$A_1$ represents a nitrogen atom,
$A_2$ and $A_3$ are such that one represents a nitrogen atom and the other represents

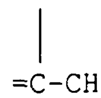

or both represent

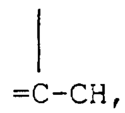

$R_5$ represents 0, NH or $CH_2$
and Y represents biphenyl optionally substituted by CN,
and of the following 5 products:

- hydrochloride of 4-[[(2-butyl 4-quinolinyl) oxy] methyl] benzoic acid;
- methyl 4'-[[(3-butyl 5-methylthio 4-quinolinyl) oxy] methyl] (1,1'-biphenyl) -2-carboxylate;
- diethylamine salt of 4'-[[(3-butyl 1,4-dihydro 4-(methylthio) 4-quinolinyl) oxy] methyl] (1,1'-biphenyl) 2-carboxylic acid;
- 4'-[[(3-butyl 4-quinolinyl) oxy] methyl] (1,1'-biphenyl)-2-carboxylic acid;
- 4'-[(2-phenyl 4-quinazolinyl) methyl] (1,1'-biphenyl)-2-carboxylic acid,

said products of formula (I) and these 5 products being in all the possible racemic, enantiomeric and diastereoisomeric isomer forms, as well as the addition salts with mineral and organic acids or with mineral and organic bases of said products of formula (I) and of these 5 products,
**characterized in that**:

a) either the compound of formula (III):

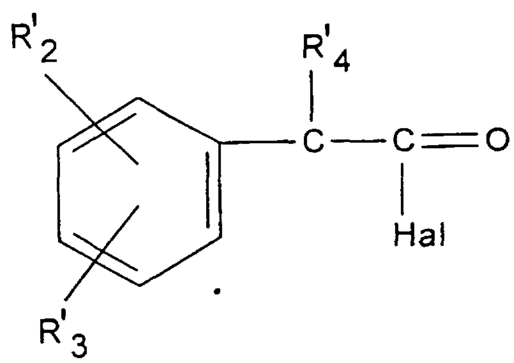

(III)

in which $R'_2$, $R'_3$ and $R'_4$ have the meanings indicated in claim 1 for $R_2$, $R_3$ and $R_4$ respectively in which the optional reactive functions are optionally protected by the protective groups, $R'_4$ can also represent a phenyl radical,
and Hal represents a halogen atom, is subjected to a substitution reaction in order to obtain the product of formula (IV):

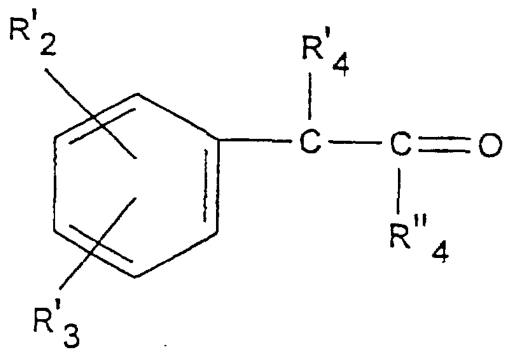

(IV)

in which $R'_2$, $R'_3$ and $R'_4$ have the meanings indicated above and $R''_4$, identical to or different from $R'_4$, has the meaning indicated in claim 1 for $R_4$ in which the optional reactive functions are optionally protected by protective groups, which is reacted:
with a compound of formula (V):

$$R'''_4\text{-}C\equiv N \qquad (V)$$

in which $R'''_4$, identical to or different from $R'_4$ or $R''_4$, has the meaning indicated in claim 1 for $R_4$ in which the optional reactive functions are optionally protected by protective groups, in order to obtain after cyclization a product of formula (Ii):

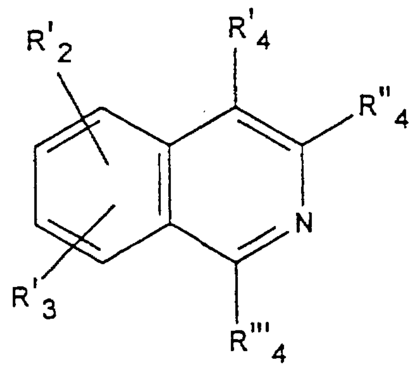

(Ii)

in which $R'_2$, $R'_3$, $R'_4$, $R'_4$ and $R'''_4$ have the meanings indicated above,
b) or a product of formula (VI):

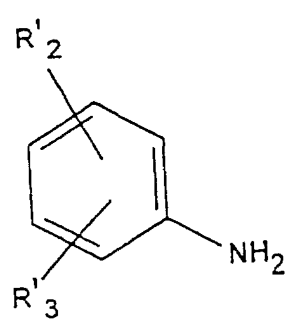

(VI)

in which $R'_2$ and $R'_3$ have the meanings indicated above is reacted with a product of formula (VII):

$$R'_4\text{-CH}(CO_2alk)\text{-CO-}R''_4 \quad (VII)$$

in which $R'_4$ and $R''_4$, identical or different, have the meanings indicated above and alk represents an alkyl radical containing at most 6 carbon atoms, in order to obtain products of formula (VIII):

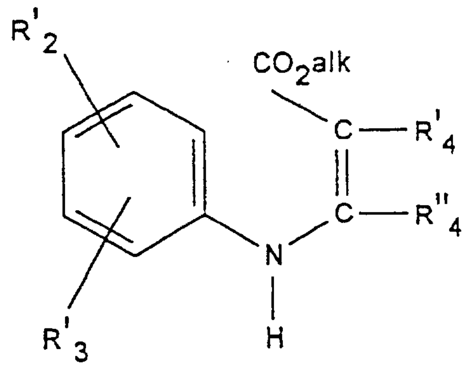

(VIII)

in which $R'_2$, $R'_3$, $R'_4$, $R''_4$ and alk have the meanings indicated above, which is cyclized in order to obtain products of formula (IIb):

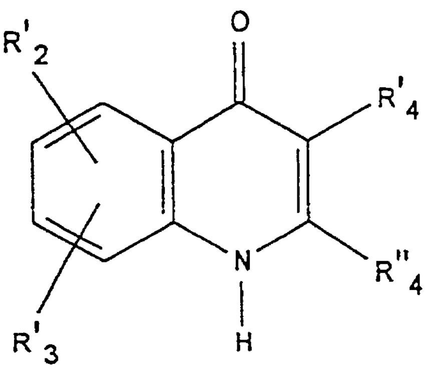

(IIb)

in which $R'_2$, $R'_3$, $R'_4$ and $R''_4$ have the meanings indicated above,
c) or a product of formula (IX):

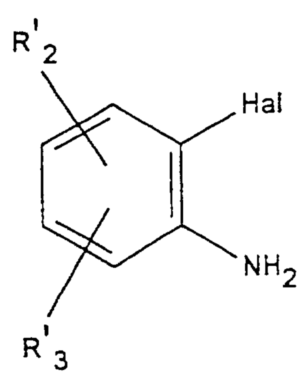

(IX)

in which $R'_2$ and $R'_3$ have the meanings indicated above is reacted with a product of formula (X):

$$R''\,'_4\text{-}C{\equiv}CH \qquad (X)$$

in which $R''\,'_4$ a the meaning indicated above in order to obtain products of formula (XI) :

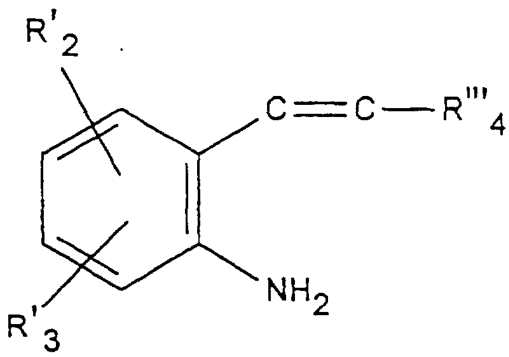

(XI)

in which $R'_2$, $R'_3$ and $R''\,'_4$ have the meanings indicated above, which is subjected to a cyclization reaction in the presence of a nitrogen donor such as sodium nitrite, in order to obtain a product of formula (IIc):

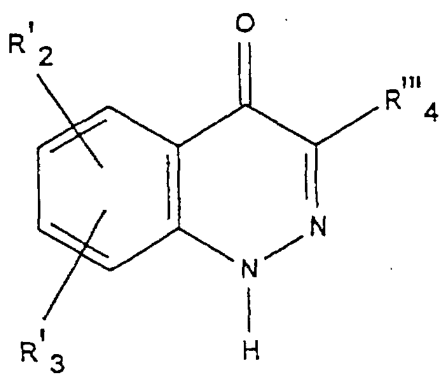

(IIc)

in which $R'_2$, $R'_3$ and $R'''_4$ have the meanings indicated above,
d) or a product of formula (XIV):

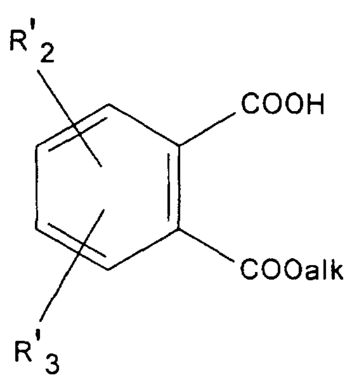

(XIV)

in which $R'_2$ and $R'_3$ and alk have the meanings indicated above, which, if desired, after a halogenation reaction of the free carboxy function, is subjected to an addition reaction on this carboxy function of a compound of formula $R'_4$-H, $R'_4$ having the meaning indicated above, in order to obtain after cyclization in the presence of hydrazine or a derivative the products of formula (IIe):

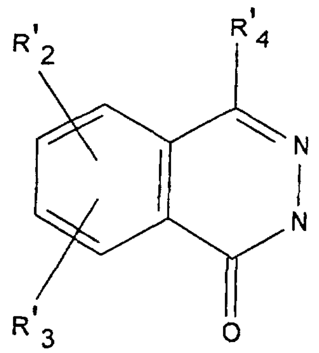

(IIe)

in which R'2, $R'_3$ and $R'_4$ have the meanings indicated above,
e) or a product of formula (XV):

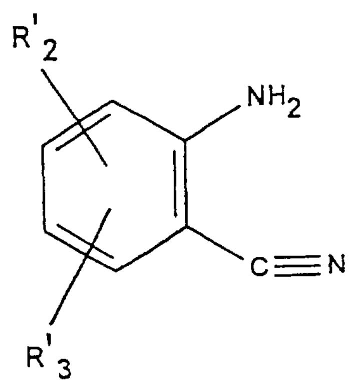

(XV)

in which $R'_2$ and $R'_3$ have the meanings indicated above is reacted with a product of formula (XVI) :

$$R'_4\text{-CO-Cl} \quad \text{(XVI)}$$

in which $R'_4$ a the meaning indicated above in order to produce the product of formula (XVII) :

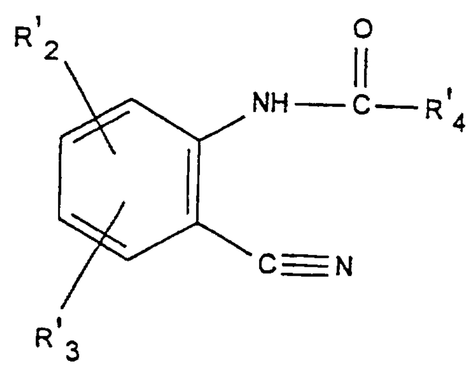

(XVII)

in which R'$_2$, R'$_3$ and R'$_4$ have the meanings indicated previously, in order to obtain, after cyclization, the products of formula (IIf) :

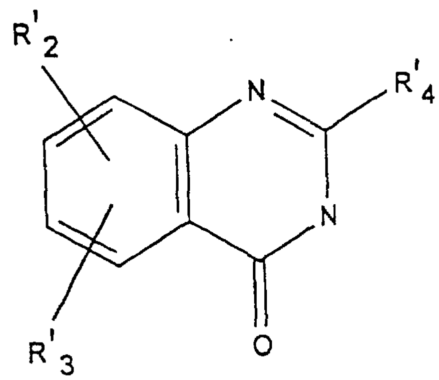

(IIf)

in which R'$_2$, R'$_3$ and R'$_4$ have the meanings indicated above, which products of formula (Ii) can represent the products of formula (I) and products of formulae (IIb), (IIc), (IIe) and (IIf) as defined above which can represent the products of formula (I) in which at least one of $A_1$, $A_2$ and $A_3$ represents a methine radical carrying a hydroxyl radical, which, if desired and if necessary, is subjected to one or more of the following reactions, in any order:

- a complete reduction reaction of the hydroxyl or oxo radical to a methine radical followed by an aromatization,
- on the products of formula (Ii) in which one of R'$_4$, R"$_4$ or R" '$_4$ represents a hydroxyl radical or on the products of formulae (IIb), (IIc), (IIe) and (IIf) which are subjected

either firstly to a substitution reaction of the hydroxyl radical by a halogen atom followed by the action of a product of formula $R_{p4}$-M-Hal in which $R_{p4}$ has the meaning indicated in claim 1 for $R_4$ in which the optional reactive functions are optionally protected by protective groups, M represents a metal atom chosen from magnesium, copper and zinc and Hal represents a halogen atom, or to the action of a product of formula $R_{p4}$ - Hal in which Hal represents a halogen atom, in order to obtain the corresponding products of formula (I),

- an elimination reaction of the protective groups which can be carried by the protected reactive functions,
- a salification reaction by a mineral or organic acid or by a mineral or organic base in order to obtain the corresponding salt,
- an esterification reaction of an acid function,
- a saponification reaction of an ester function to an acid function,
- a conversion reaction of the cyano function in acid function,
- a resolution reaction of the racemic forms, said products thus obtained being in all possible racemic, enantiomeric and diastereoisomeric isomer forms.

**2.** Process according to claim 1, for the preparation of 4'-[(2-butyl-4-quinolinyl)methyl] (1,1'-biphenyl)-2-carboxylic acid.